# EUROPEAN PATENT APPLICATION

(11) **EP 2 333 089 A1**
(43) Date of publication of application: **15.06.2011**
(21) Application number: 10180374.0
(22) Date of filing: 07.11.2006
(51) Int. Cl.: C12N 15/82, A01H 5/00, A01H 5/10

(54) **Class I homeodomain leucine zipper (HDZip) hox5 polypetide-expressing plants having improved growth characteristics and a method for making the same**

(30) Priority: 07.11.2005 EP 05110413; 07.11.2005 EP 05110429; 14.11.2005 US 736194 P; 17.11.2005 EP 05110900; 23.11.2005 US 739686 P; 24.11.2005 EP 05111260; 05.12.2005 US 742287 P
(62) Divisional of application: 06819308.5
(71) Applicant: CropDesign N.V., 9052 Zwijnaarde (BE)
(72) Inventor: Sanz Molinero, Ana Isabel, 9050, Gentbrugge (BE); Reuzeau, Christophe, F-24350, La Chapelle Gonaguet (FR); Hatzfeld, Yves, 59000, Lille (FR); Mironov, Valdimir, 7052, Trondheim (NO); Inzé, Dirk, Moorsel-Aalst, 9310 (BE); De Veylder, Lieven, 9031, Drongen (BE); Boudolf, Veronique, 9860, Oosterzele (BE)
(74) Representative: Mistry, Meeta

(57) **Abstract**

The present invention relates generally to the field of molecular biology and concerns a method for improving plant growth characteristics relative to corresponding wild type plants. More specifically, the present invention concerns a method for improving plant growth characteristics comprising modulating expression in a plant of a nucleic acid encoding a class I homeodomain leucine zipper (HDZip) hox5 polypeptide or a homologue thereof. The present invention also concerns plants having modulated expression of a nucleic acid encoding a class I homeodomain leucine zipper (HDZip) hox5 polypeptide or a homologue thereof, which plants have improved growth characteristics relative to corresponding wild type plants. The invention also provides constructs useful in the methods of the invention.

SEQUENCE LISTING

<110>  CropDesign N.V.

<120>  Plants having improved growth characteristics and a method for
       making the same

<130>  PF57967

<150>  EP 05110413.1
<151>  2005-11-07

<150>  EP 05110429.7
<151>  2005-11-07

<150>  US 60/736,194
<151>  2005-11-14

<150>  EP 05110900.7
<151>  2005-11-17

<150>  US 60/739,686
<151>  2005-11-23

<150>  EP 05111260.5
<151>  2005-11-24

<150>  US 60/742,287
<151>  2005-12-05

<160>  51

<170>  PatentIn version 3.3

<210>  1
<211>  1050
<212>  DNA
<213>  Oryza sativa

<400>  1
atggatcccg gccgcgtcgt gttcgactcc ggcgtggcgc ggcgggcgtg ccccggcggc      60
gcgcagatgc ttctcttcgg cggcggcggc agcgccaaca gcggcggctt cttccgaggc     120
gtgccggcgg cggtgctggg gatggatgaa tcgcggtcgt cgtcgtcggc ggcgggggcg     180
ggggcgaagc ggccgttctt cacgacgcac gaggagctcc tggaggagga gtactacgac     240
gagcaggcgc cggagaagaa gcggcggctg acggcggagc aggtgcagat gctggagcgg     300
agcttcgagg aggagaacaa gctggagccg gagcggaaga cggagctcgc ccgccgcctc     360
ggcatggccc cccggcaggt cgccgtctgg ttccagaacc gccgcgcccg ctggaagacc     420
aagcagctcg agcacgactt cgaccgcctc aaggccgcct acgacgccct cgccgccgac     480
caccatgccc tcctctccga caacgaccgc ctccgcgcgc aggtaatctc attaaccgag     540
aagctgcaag acaaggagac gtcgccgtcg tcggcgacca tcaccaccgc ggcgcaggag     600
gtcgaccagc cggacgaaca cacggaggcc gcgtcaacca ccggcttcgc caccgtcgac     660
ggcgcattgg cggcgccacc gcccggccac cagcagccgc cgcataaaga tgatcttgtg     720
agcagcggcg gcaccaacga cgacggcgat ggcggcgcgg ccgtggtggt cttcgacgtc     780
accgagggcg ccaacgaccg cctcagctgc gagtcggcgt acttcgccga cgccgcggag     840
gcgtacgagc gcgactgcgc cgggcactac gccctctcgt cggaggagga ggacggcggc     900
gcggtcagcg acgagggctg cagcttcgac ctccccgacg ccgccgccgc cgccgccgcc     960
atgttcggcg ccgccggagt tgtgcaccac gacgccgcgg acgacgagga ggcgcagctc    1020
ggcagctgga ccgcctggtt ctggagctga                                     1050

<210>  2
<211>  349
<212>  PRT
<213>  Oryza sativa

<400>  2
Met Asp Pro Gly Arg Val Val Phe Asp Ser Gly Val Ala Arg Arg Ala
1               5                   10                  15
Cys Pro Gly Gly Ala Gln Met Leu Leu Phe Gly Gly Gly Gly Ser Ala
            20                  25                  30
Asn Ser Gly Gly Phe Phe Arg Gly Val Pro Ala Ala Val Leu Gly Met
        35                  40                  45
Asp Glu Ser Arg Ser Ser Ser Ser Ala Ala Gly Ala Gly Ala Lys Arg
    50                  55                  60
Pro Phe Phe Thr Thr His Glu Glu Leu Leu Glu Glu Glu Tyr Tyr Asp
65                  70                  75                  80
Glu Gln Ala Pro Glu Lys Lys Arg Arg Leu Thr Ala Glu Gln Val Gln
                85                  90                  95
Met Leu Glu Arg Ser Phe Glu Glu Glu Asn Lys Leu Glu Pro Glu Arg
            100                 105                 110
Lys Thr Glu Leu Ala Arg Arg Leu Gly Met Ala Pro Arg Gln Val Ala
        115                 120                 125
Val Trp Phe Gln Asn Arg Arg Ala Arg Trp Lys Thr Lys Gln Leu Glu
    130                 135                 140
His Asp Phe Asp Arg Leu Lys Ala Ala Tyr Asp Ala Leu Ala Ala Asp
145                 150                 155                 160
His His Ala Leu Leu Ser Asp Asn Asp Arg Leu Arg Ala Gln Val Ile
                165                 170                 175
Ser Leu Thr Glu Lys Leu Gln Asp Lys Glu Thr Ser Pro Ser Ser Ala
            180                 185                 190
Thr Ile Thr Thr Ala Ala Gln Glu Val Asp Gln Pro Asp Glu His Thr
        195                 200                 205
Glu Ala Ala Ser Thr Thr Gly Phe Ala Thr Val Asp Gly Ala Leu Ala
    210                 215                 220
Ala Pro Pro Pro Gly His Gln Gln Pro Pro His Lys Asp Asp Leu Val
225                 230                 235                 240
Ser Ser Gly Gly Thr Asn Asp Asp Gly Asp Gly Gly Ala Ala Val Val
                245                 250                 255
Val Phe Asp Val Thr Glu Gly Ala Asn Asp Arg Leu Ser Cys Glu Ser
            260                 265                 270
Ala Tyr Phe Ala Asp Ala Ala Glu Ala Tyr Glu Arg Asp Cys Ala Gly
        275                 280                 285
His Tyr Ala Leu Ser Ser Glu Glu Glu Asp Gly Gly Ala Val Ser Asp
    290                 295                 300
Glu Gly Cys Ser Phe Asp Leu Pro Asp Ala Ala Ala Ala Ala Ala Ala
305                 310                 315                 320
Met Phe Gly Ala Ala Gly Val Val His His Asp Ala Ala Asp Asp Glu
                325                 330                 335
Glu Ala Gln Leu Gly Ser Trp Thr Ala Trp Phe Trp Ser
            340                 345

<210>  3
<211>  1197
<212>  DNA
<213>  Oryza sativa

<400>  3
atggagtccg gccggctcat cttcagcacg gcgggctccg gcgccgggca gatgctcttc      60
ttggactgcg gcgctggcgg cggcggcgtc ggcggcgggg ccatgttcca tcgaggggcg     120

agaccggtgc tcggcatgga ggaaggaggg cgcggcgtca agcggccctt cttcaccacc 180
cccgacgagc tcctcgaaga ggagtactac gacgagcagc tcccggagaa gaagcggcgc 240
ctcacgccgg agcaggtgca tctgctggag aggagcttcg aggaggagaa caagctggag 300
ccggagcgga agacggagct ggcgcggaag ctaggcctgc agccgcggca ggtcgccgtg 360
tggttccaga accgccgcgc gcgctggaag accaagcagc tcgagcgcga cttcgaccgc 420
ctcaaggcgt cgttcgacgc cctccgcgcc gaccacgacg ccctcctcca ggacaaccac 480
cgcctccact ctcaggtcat gtcgttgacc gagaagctgc aagagaagga gacgacgacc 540
gagggcagcg ccggcgcggc cgttgacgtc ccgggcttgc ctgcggcggc cgacgtgaag 600
gtcgccgtcc cggacgccga ggaaccggcg ctggaggagg cggcggcggc gttcgaggag 660
cagcaggagc agcaggtgaa ggccgaggac aggctgagca cgggcagcgg cgggagcgcg 720
gtggtggaca cggacgcgca actggtggtc gggtgcggcc ggcaagcatc tcgccgccgt 780
ggacagcagc gtggagtcgt acttcccggg cggcgacgag taccacgact gcgtgatggg 840
ccccatggac cacgccgcgg ggggcatcca gtcggaggag gacgacggcg ccggcagcga 900
cgagggctgc agctactacg ccgacgacgc cggcgtcctc ttcgccgacc acggccacca 960
ccaccaccac caacacgcgg acgacgacga ggaggacggc cagcagatca gctgctggtg 1020
gatgtggaac tagatttctc gcgcgcgcgc gtcgtcgtgc attcaattct cgtgttaaaa 1080
aaatcgttct ctttttcatt tttccgcttc tttgtctgta atgttgagtt tcgatcggct 1140
atgagaagga aggaggtgta tgcatgtgca tggtatggta gggtaacaca tcggtga 1197

<210> 4
<211> 343
<212> PRT
<213> Oryza sativa

<400> 4
Met Glu Ser Gly Arg Leu Ile Phe Ser Thr Ala Gly Ser Gly Ala Gly
1               5                   10                  15
Gln Met Leu Phe Leu Asp Cys Gly Ala Gly Gly Gly Gly Val Gly Gly
            20                  25                  30
Gly Ala Met Phe His Arg Gly Ala Arg Pro Val Leu Gly Met Glu Glu
        35                  40                  45
Gly Gly Arg Gly Val Lys Arg Pro Phe Phe Thr Thr Pro Asp Glu Leu
    50                  55                  60
Leu Glu Glu Glu Tyr Tyr Asp Glu Gln Leu Pro Glu Lys Lys Arg Arg
65                  70                  75                  80
Leu Thr Pro Glu Gln Val His Leu Leu Glu Arg Ser Phe Glu Glu Glu
                85                  90                  95
Asn Lys Leu Glu Pro Glu Arg Lys Thr Glu Leu Ala Arg Lys Leu Gly
            100                 105                 110
Leu Gln Pro Arg Gln Val Ala Val Trp Phe Gln Asn Arg Arg Ala Arg
        115                 120                 125
Trp Lys Thr Lys Gln Leu Glu Arg Asp Phe Asp Arg Leu Lys Ala Ser
    130                 135                 140
Phe Asp Ala Leu Arg Ala Asp His Asp Ala Leu Leu Gln Asp Asn His
145                 150                 155                 160
Arg Leu His Ser Gln Val Met Ser Leu Thr Glu Lys Leu Gln Glu Lys
                165                 170                 175
Glu Thr Thr Thr Glu Gly Ser Ala Gly Ala Ala Val Asp Val Pro Gly
            180                 185                 190
Leu Pro Ala Ala Ala Asp Val Lys Val Ala Val Pro Asp Ala Glu Glu
        195                 200                 205
Pro Ala Leu Glu Glu Ala Ala Ala Ala Phe Glu Glu Gln Gln Glu Gln
    210                 215                 220
Gln Val Lys Ala Glu Asp Arg Leu Ser Thr Gly Ser Gly Gly Ser Ala
225                 230                 235                 240
Val Val Asp Thr Asp Ala Gln Leu Val Val Gly Cys Gly Arg Gln His
                245                 250                 255
Leu Ala Ala Val Asp Ser Ser Val Glu Ser Tyr Phe Pro Gly Gly Asp
            260                 265                 270

Glu Tyr His Asp Cys Val Met Gly Pro Met Asp His Ala Ala Gly Gly
        275                 280                 285
Ile Gln Ser Glu Glu Asp Asp Gly Ala Gly Ser Asp Glu Gly Cys Ser
    290                 295                 300
Tyr Tyr Ala Asp Asp Ala Gly Val Leu Phe Ala Asp His Gly His His
305                 310                 315                 320
His His His Gln His Ala Asp Asp Asp Glu Glu Asp Gly Gln Gln Ile
                325                 330                 335
Ser Cys Trp Trp Met Trp Asn
            340

<210>  5
<211>  819
<212>  DNA
<213>  Zea mays

<400>  5
atggatccga gcgcggtcag tttcgactct ggcggcgcgc ggcggggcgg cggcgcgcag      60
atgctgctct tcggcggcgg aggcagcgcc aacagcaacg gcttcttccg aggtgttccg     120
atggcggtcc tgggcatgga cgacgcgacg cgcgtgggca agcggccctt cttcacgaca     180
cacgaggagc tcctagagga ggagtactac gacgagcagg cgccggagaa gaagcgccga     240
ctgacggcgg agcaggtgca gctgctggag cggagcttcg aagaagagaa caagctggag     300
ccggagcgca agaccgagct ggctcgccgc ctggggatgg cgccccgcca ggtagctgtt     360
tggttccaga accgccgcgc gcgctggaag accaagcaac tcgagaccga ctatgaccgc     420
ctcaaggctg cttacgacgc actcgccgcc gaccaccagg gcctcctggc cgacaacgat     480
aacctccggg cacaggtgat ctccctgacg gagaagctgc aaggcaagga gacatccccg     540
tcggcaacca ctgctgccca agaggtcgac cagccagacg aacacaccgc tgtgtcaggc     600
acggaagaac tgctggcgca gcagctcaag gacaacctcc acagcagcgg cgactgcact     660
ggccatggca ccctctcttc ggaagaagac gacggtggcg tggtcagtga cgagggctgc     720
agcttcgctc tcccggatgc catgttcgct gccgggttca cccaccatgg cgccgaggag     780
gtgcagctgg ccaactggac atccatgttc tggaactga                            819

<210>  6
<211>  272
<212>  PRT
<213>  Zea mays

<400>  6
Met Asp Pro Ser Ala Val Ser Phe Asp Ser Gly Gly Ala Arg Arg Gly
1               5                   10                  15
Gly Gly Ala Gln Met Leu Leu Phe Gly Gly Gly Gly Ser Ala Asn Ser
            20                  25                  30
Asn Gly Phe Phe Arg Gly Val Pro Met Ala Val Leu Gly Met Asp Asp
        35                  40                  45
Ala Thr Arg Val Gly Lys Arg Pro Phe Phe Thr Thr His Glu Glu Leu
    50                  55                  60
Leu Glu Glu Glu Tyr Tyr Asp Glu Gln Ala Pro Glu Lys Lys Arg Arg
65                  70                  75                  80
Leu Thr Ala Glu Gln Val Gln Leu Leu Glu Arg Ser Phe Glu Glu Glu
                85                  90                  95
Asn Lys Leu Glu Pro Glu Arg Lys Thr Glu Leu Ala Arg Arg Leu Gly
            100                 105                 110
Met Ala Pro Arg Gln Val Ala Val Trp Phe Gln Asn Arg Arg Ala Arg
        115                 120                 125
Trp Lys Thr Lys Gln Leu Glu Thr Asp Tyr Asp Arg Leu Lys Ala Ala
    130                 135                 140
Tyr Asp Ala Leu Ala Ala Asp His Gln Gly Leu Leu Ala Asp Asn Asp
145                 150                 155                 160
Asn Leu Arg Ala Gln Val Ile Ser Leu Thr Glu Lys Leu Gln Gly Lys

                165                 170                 175
Glu Thr Ser Pro Ser Ala Thr Thr Ala Ala Gln Glu Val Asp Gln Pro
            180                 185                 190
Asp Glu His Thr Ala Val Ser Gly Thr Glu Glu Leu Leu Ala Gln Gln
        195                 200                 205
Leu Lys Asp Asn Leu His Ser Ser Gly Asp Cys Thr Gly His Gly Thr
    210                 215                 220
Leu Ser Ser Glu Glu Asp Asp Gly Gly Val Val Ser Asp Glu Gly Cys
225                 230                 235                 240
Ser Phe Ala Leu Pro Asp Ala Met Phe Ala Ala Gly Phe Thr His His
                245                 250                 255
Gly Ala Glu Glu Val Gln Leu Ala Asn Trp Thr Ser Met Phe Trp Asn
            260                 265                 270

<210>  7
<211>  993
<212>  DNA
<213>  Zea mays

<400>  7
atggagtctg gacggctcat cttcaacgcg ccgggctctg gcgccgggca gatgctcttc      60
ctcgactgcg gcgcaggcgg cggtcccggc ggcggcttgt tccatcgagg cgggagaccg     120
atgcttggcc ttgaagaagg gcgcggcgta aaacggccct tcttcacctc gcccgacgag     180
ctcctcgagg aagagtacta cgacgagcag ctgccggaga agaagcgccg cctcacccca     240
gagcaggtgc ttctgctgga gaggagcttc gaggaggaga acaagctgga gccggagcgc     300
aagacggagc tggcgcgcaa gctgggcctg cagcctcgcc aggtggccgt ctggttccag     360
aaccgccgcg cccggtggaa gaccaagcag ctcgagcgcg acttcgaccg cctcaaggcc     420
tccttcgacg ctctccgagc ggaccacgac gccctcctcc aggacaacaa ccgcctccgc     480
tcacaggttg tgtcgttgac cgagaagctg caagagaagg aggatgcgac ggagggcggc      540
gccaccgctg acaccgccgc gccggcggtg gacgtcgagg cttccctggc cgacgacgtc     600
gaggagccag cagagcctgc ggcgacgttc gaggtgctgc aggaggtgaa gtccgaggac     660
aggctgagca ccggcagcgg cgggagcgcg gtggtggacg cggacgcgct gctgtacggc     720
aggttcgccg cggcagttga tagcagcgtg gagtcgtact tccccggcgg cgaggaccac     780
taccacgact gcgggacgat gggccccgtg aatcatggcg ccggaggagg catccagtcg     840
gacgacgacg gcgccggcag cgacgagggg tgcagctact acgccgacga agccgccgcc     900
gccgccgccg cgttcttcgc cggacacgcc acccaccacc acgcggacga ggacgaggac     960
gccggccaga tcagctggtg gatgtggaac tag                                  993

<210>  8
<211>  330
<212>  PRT
<213>  Zea mays

<400>  8
Met Glu Ser Gly Arg Leu Ile Phe Asn Ala Pro Gly Ser Gly Ala Gly
1               5                   10                  15
Gln Met Leu Phe Leu Asp Cys Gly Ala Gly Gly Gly Pro Gly Gly Gly
            20                  25                  30
Leu Phe His Arg Gly Gly Arg Pro Met Leu Gly Leu Glu Glu Gly Arg
        35                  40                  45
Gly Val Lys Arg Pro Phe Phe Thr Ser Pro Asp Glu Leu Leu Glu Glu
    50                  55                  60
Glu Tyr Tyr Asp Glu Gln Leu Pro Glu Lys Lys Arg Arg Leu Thr Pro
65                  70                  75                  80
Glu Gln Val Leu Leu Leu Glu Arg Ser Phe Glu Glu Glu Asn Lys Leu
                85                  90                  95
Glu Pro Glu Arg Lys Thr Glu Leu Ala Arg Lys Leu Gly Leu Gln Pro
            100                 105                 110
Arg Gln Val Ala Val Trp Phe Gln Asn Arg Arg Ala Arg Trp Lys Thr

115                 120                 125
Lys Gln Leu Glu Arg Asp Phe Asp Arg Leu Lys Ala Ser Phe Asp Ala
    130                 135                 140
Leu Arg Ala Asp His Asp Ala Leu Leu Gln Asp Asn Asn Arg Leu Arg
145                 150                 155                 160
Ser Gln Val Val Ser Leu Thr Glu Lys Leu Gln Glu Lys Glu Asp Ala
                165                 170                 175
Thr Glu Gly Gly Ala Thr Ala Asp Thr Ala Ala Pro Ala Val Asp Val
            180                 185                 190
Glu Ala Ser Leu Ala Asp Asp Val Glu Glu Pro Ala Glu Pro Ala Ala
        195                 200                 205
Thr Phe Glu Val Leu Gln Glu Val Lys Ser Glu Asp Arg Leu Ser Thr
    210                 215                 220
Gly Ser Gly Gly Ser Ala Val Val Asp Ala Asp Ala Leu Leu Tyr Gly
225                 230                 235                 240
Arg Phe Ala Ala Ala Val Asp Ser Ser Val Glu Ser Tyr Phe Pro Gly
                245                 250                 255
Gly Glu Asp His Tyr His Asp Cys Gly Thr Met Gly Pro Val Asn His
            260                 265                 270
Gly Ala Gly Gly Gly Ile Gln Ser Asp Asp Asp Gly Ala Gly Ser Asp
        275                 280                 285
Glu Gly Cys Ser Tyr Tyr Ala Asp Glu Ala Ala Ala Ala Ala Ala Ala
    290                 295                 300
Phe Phe Ala Gly His Ala Thr His His His Ala Asp Glu Asp Glu Asp
305                 310                 315                 320
Ala Gly Gln Ile Ser Trp Trp Met Trp Asn
                325                 330

<210>  9
<211>  825
<212>  DNA
<213>  Saccharum officinarum

<400>  9
atggatccga gcgcggtcag tttcaactcc ggcggcgcgc ggcggggcgg cggcggcacg      60
cagatgctgc tcttcggcgg cggaggcagc gccaacagca acggcttctt ccgaggtgtt     120
ccgatggcgg tcctgggcat ggacgacgcg acgcgcgtgg gcaagcggcc cttcttcacc     180
acacacgagg agctcctgga ggaggagtac tacgacgagc aggcgcccga gaagaagcgc     240
cgtctgacgg cggagcaggt gcagctgctg gagcggagct tcgaggaaga gaacaagctg     300
gagcccgagc gcaagaccga gctggctcgc cgcctcggga tggcgccccg ccaggtggcc     360
gtctggttcc agaaccgccg cgcgcgctgg aagaccaagc agctcgagac cgactatgac     420
cacctcaagg ctgcctacga cgcgctcgcc gccgaccacc agggcctcct ggccgacaac     480
gatagcctcc gggcacaggt ggtctcccta acagagaagc tgcaaggcaa ggagacatcc     540
ccgtcggcca ccactgctgc ccaagaggtc gaccagccag acgaacacac cgcggcgtca     600
ggcactgaga aactgctggc gcagcagctc aaggacgacc tccacagcag cggcgactgc     660
actggccatg gtgccctctc ctcagaggaa gaagatggtg gtgtggtcag tgacgagggc     720
agctttgatc tcccggatgc catgtttgct gccggggtca cccaccatgg cgccgacgcc     780
gaggaggcac agctggccaa ctggacatcc tggttctgga actga                     825

<210>  10
<211>  274
<212>  PRT
<213>  Saccharum officinarum

<400>  10
Met Asp Pro Ser Ala Val Ser Phe Asn Ser Gly Gly Ala Arg Arg Gly
1               5                   10                  15
Gly Gly Gly Thr Gln Met Leu Leu Phe Gly Gly Gly Gly Ser Ala Asn
            20                  25                  30

Ser Asn Gly Phe Phe Arg Gly Val Pro Met Ala Val Leu Gly Met Asp
        35                  40                  45
Asp Ala Thr Arg Val Gly Lys Arg Pro Phe Phe Thr Thr His Glu Glu
    50                  55                  60
Leu Leu Glu Glu Glu Tyr Tyr Asp Glu Gln Ala Pro Glu Lys Lys Arg
65                  70                  75                  80
Arg Leu Thr Ala Glu Gln Val Gln Leu Leu Glu Arg Ser Phe Glu Glu
                85                  90                  95
Glu Asn Lys Leu Glu Pro Glu Arg Lys Thr Glu Leu Ala Arg Arg Leu
            100                 105                 110
Gly Met Ala Pro Arg Gln Val Ala Val Trp Phe Gln Asn Arg Arg Ala
        115                 120                 125
Arg Trp Lys Thr Lys Gln Leu Glu Thr Asp Tyr Asp His Leu Lys Ala
    130                 135                 140
Ala Tyr Asp Ala Leu Ala Ala Asp His Gln Gly Leu Leu Ala Asp Asn
145                 150                 155                 160
Asp Ser Leu Arg Ala Gln Val Val Ser Leu Thr Glu Lys Leu Gln Gly
                165                 170                 175
Lys Glu Thr Ser Pro Ser Ala Thr Thr Ala Ala Gln Glu Val Asp Gln
            180                 185                 190
Pro Asp Glu His Thr Ala Ala Ser Gly Thr Glu Lys Leu Leu Ala Gln
        195                 200                 205
Gln Leu Lys Asp Asp Leu His Ser Ser Gly Asp Cys Thr Gly His Gly
    210                 215                 220
Ala Leu Ser Ser Glu Glu Glu Asp Gly Gly Val Val Ser Asp Glu Gly
225                 230                 235                 240
Ser Phe Asp Leu Pro Asp Ala Met Phe Ala Ala Gly Val Thr His His
                245                 250                 255
Gly Ala Asp Ala Glu Glu Ala Gln Leu Ala Asn Trp Thr Ser Trp Phe
            260                 265                 270
Trp Asn

<210>  11
<211>  828
<212>  DNA
<213>  Sorghum bicolor

<400>  11
atggatccga gcgcggtcag tttcgactcc ggcggcgcgc ggcggggcgg cggcggcggc      60
ggcgcgcaga tgctgctctt cggcggcgga ggcagcgcca acagcaacgg cttcttccga     120
ggtgttccga tggcggtcct gggcatggac gacgcgacgc gcgtgggcaa gcggcctttc     180
ttcaccacgc acgaggagct cctggaggag gagtactacg acgagcaggc gcccgagaag     240
aagcgccgtc tgacggcgga gcaggtgcag ctgctggagc ggagcttcga ggaagagaac     300
aagctggagc cggagcgcaa gaccgagctg gctcgccgcc tcgggatggc gcctcgccag     360
gtggccgtct ggttccagaa ccgccgcgcg cgctggaaga ctaagcagct cgagaccgac     420
tatgaccgcc tcaaggctgc ctacgacgcg ctcgccgccg accaccaggg cctcctggcc     480
gacaacgata gcctccgggc acaggtgatc tccctaacgg ataagctgca acgcaaggag     540
acatccccgt cggcgaccac tgctgcccaa gaggtcgacc agccagacga acacaccgct     600
gcgtcaggca ctgagaaact gctggtgcag cagctcaagg acgacctcca cagcagcggc     660
gacttcactg gccatggtgc cctctcttca gaggaagagg atggtggtgt ggtcagcgac     720
gagggctgca gctttgatct cccggatgcc atgttcgctg ccggggtcac ccaccatggc     780
gccgaggagg cgcagctggc caactggaca tcctggttct ggaactga                  828

<210>  12
<211>  275
<212>  PRT
<213>  Sorghum bicolor

<400>  12
Met Asp Pro Ser Ala Val Ser Phe Asp Ser Gly Gly Ala Arg Arg Gly
1               5                   10                  15
Gly Gly Gly Gly Gly Ala Gln Met Leu Leu Phe Gly Gly Gly Gly Ser
            20                  25                  30
Ala Asn Ser Asn Gly Phe Phe Arg Gly Val Pro Met Ala Val Leu Gly
        35                  40                  45
Met Asp Asp Ala Thr Arg Val Gly Lys Arg Pro Phe Phe Thr Thr His
    50                  55                  60
Glu Glu Leu Leu Glu Glu Glu Tyr Tyr Asp Glu Gln Ala Pro Glu Lys
65                  70                  75                  80
Lys Arg Arg Leu Thr Ala Glu Gln Val Gln Leu Leu Glu Arg Ser Phe
                85                  90                  95
Glu Glu Glu Asn Lys Leu Glu Pro Glu Arg Lys Thr Glu Leu Ala Arg
            100                 105                 110
Arg Leu Gly Met Ala Pro Arg Gln Val Ala Val Trp Phe Gln Asn Arg
        115                 120                 125
Arg Ala Arg Trp Lys Thr Lys Gln Leu Glu Thr Asp Tyr Asp Arg Leu
    130                 135                 140
Lys Ala Ala Tyr Asp Ala Leu Ala Ala Asp His Gln Gly Leu Leu Ala
145                 150                 155                 160
Asp Asn Asp Ser Leu Arg Ala Gln Val Ile Ser Leu Thr Asp Lys Leu
                165                 170                 175
Gln Arg Lys Glu Thr Ser Pro Ser Ala Thr Thr Ala Ala Gln Glu Val
            180                 185                 190
Asp Gln Pro Asp Glu His Thr Ala Ala Ser Gly Thr Glu Lys Leu Leu
        195                 200                 205
Val Gln Gln Leu Lys Asp Asp Leu His Ser Ser Gly Asp Phe Thr Gly
    210                 215                 220
His Gly Ala Leu Ser Ser Glu Glu Glu Asp Gly Gly Val Val Ser Asp
225                 230                 235                 240
Glu Gly Cys Ser Phe Asp Leu Pro Asp Ala Met Phe Ala Ala Gly Val
                245                 250                 255
Thr His His Gly Ala Glu Glu Ala Gln Leu Ala Asn Trp Thr Ser Trp
            260                 265                 270
Phe Trp Asn
        275

<210>  13
<211>  1023
<212>  DNA
<213>  Triticum aestivum

<400>  13
atggagcccg gccggctcat cttcaacacg tcgggctccg gcaacggaca gatgctcttc      60
atggactgcg gcgcgggcgg catcgccggc gcggccggca tgttccatcg aggggtgaga     120
ccggtcctcg gcggcatgga agaagggcgc ggcgtgaagc ggcccttctt cacctcgccg     180
gatgacatgc tcgaggagga gtactacgac gagcagctcc cggagaagaa gcggcgcctc     240
accccggagc aggtccacct gctggagagg agcttcgagg aggagaacaa gctggagccg     300
gagaggaaga cggagctggc ccgcaagctc gggctgcagc cacgccaggt ggccgtctgg     360
ttccagaacc gccgcgcccg gtggaagaca aagacgctgg agcgcgactt cgaccgcctc     420
aaggcgtcct tcgacgccct ccgggccgac cacgacgccc tcctccagga caaccaccgg     480
ctccggtcac aggtggtaac gttgaccgag aagatgcaag ataaggaggc gccggaaggc     540
agcttcggtg cagccgccga cgcctcggag ccggagcagg cggcggcgga ggcgaaggct     600
tccttggccg acgccgagga gcaggccgcg gcagcggagg cgttcgaggt ggtgcagcag     660
cagctgcacg tgaaggacga ggagaggctg agcccgggga gcggcgggag cgcggtgctg     720
gacgcgaggg acgcgctgct cgggagcgga tgcggcctcg ccggcgtggt ggacagcagc     780
gtggactcgt actgcttccc ggggggcgcc ggcggcgacg agtaccacga gtgcgtggtg     840
ggccccgtgg cgggcggcat ccagtcggag gaggacgacg gcgcgggcag cgacgagggc     900

tgcagctact accccgacga cgccgccgtc ttcttcgccg ccgcgcaagg gcacggccac     960
catcgcacgg acgacgacga tcagcaggac gacggccaga tcagctactg gatgtggaac    1020
tag                                                                  1023

<210>  14
<211>  340
<212>  PRT
<213>  Triticum aestivum

<400>  14
Met Glu Pro Gly Arg Leu Ile Phe Asn Thr Ser Gly Ser Gly Asn Gly
1               5                   10                  15
Gln Met Leu Phe Met Asp Cys Gly Ala Gly Gly Ile Ala Gly Ala Ala
            20                  25                  30
Gly Met Phe His Arg Gly Val Arg Pro Val Leu Gly Gly Met Glu Glu
        35                  40                  45
Gly Arg Gly Val Lys Arg Pro Phe Phe Thr Ser Pro Asp Asp Met Leu
    50                  55                  60
Glu Glu Glu Tyr Tyr Asp Glu Gln Leu Pro Glu Lys Lys Arg Arg Leu
65                  70                  75                  80
Thr Pro Glu Gln Val His Leu Leu Glu Arg Ser Phe Glu Glu Glu Asn
                85                  90                  95
Lys Leu Glu Pro Glu Arg Lys Thr Glu Leu Ala Arg Lys Leu Gly Leu
            100                 105                 110
Gln Pro Arg Gln Val Ala Val Trp Phe Gln Asn Arg Arg Ala Arg Trp
        115                 120                 125
Lys Thr Lys Thr Leu Glu Arg Asp Phe Asp Arg Leu Lys Ala Ser Phe
    130                 135                 140
Asp Ala Leu Arg Ala Asp His Asp Ala Leu Leu Gln Asp Asn His Arg
145                 150                 155                 160
Leu Arg Ser Gln Val Val Thr Leu Thr Glu Lys Met Gln Asp Lys Glu
                165                 170                 175
Ala Pro Glu Gly Ser Phe Gly Ala Ala Ala Asp Ala Ser Glu Pro Glu
            180                 185                 190
Gln Ala Ala Ala Glu Ala Lys Ala Ser Leu Ala Asp Ala Glu Glu Gln
        195                 200                 205
Ala Ala Ala Ala Glu Ala Phe Glu Val Val Gln Gln Gln Leu His Val
    210                 215                 220
Lys Asp Glu Glu Arg Leu Ser Pro Gly Ser Gly Gly Ser Ala Val Leu
225                 230                 235                 240
Asp Ala Arg Asp Ala Leu Leu Gly Ser Gly Cys Gly Leu Ala Gly Val
                245                 250                 255
Val Asp Ser Ser Val Asp Ser Tyr Cys Phe Pro Gly Gly Ala Gly Gly
            260                 265                 270
Asp Glu Tyr His Glu Cys Val Val Gly Pro Val Ala Gly Gly Ile Gln
        275                 280                 285
Ser Glu Glu Asp Asp Gly Ala Gly Ser Asp Glu Gly Cys Ser Tyr Tyr
    290                 295                 300
Pro Asp Asp Ala Ala Val Phe Phe Ala Ala Ala Gln Gly His Gly His
305                 310                 315                 320
His Arg Thr Asp Asp Asp Asp Gln Gln Asp Asp Gly Gln Ile Ser Tyr
                325                 330                 335
Trp Met Trp Asn
            340

<210>  15
<211>  819
<212>  DNA
<213>  Arabidopsis thaliana

<400>  15
atggaatcca attcgttttt cttcgatcca tctgcttcac acggcaacag catgttcttc      60
cttgggaatc tcaatcccgt cgtccaagga ggaggagcaa gatcgatgat gaacatggag     120
gaaacttcga agcgaaggcc cttctttagc tcccctgagg atctctacga cgatgacttt     180
tacgacgacc agttgcctga aaagaagcgt cgcctcacta ccgaacaagt gcatctgctg     240
gagaaaagct tcgagacaga gaacaagcta gagcctgaac gcaagactca gcttgccaag     300
aagcttggtc tacagccaag gcaagtggct gtctggtttc agaatcgccg agctcgttgg     360
aaaacaaaac agcttgagag agactacgat cttctcaagt ccacttacga ccaacttctt     420
tctaactacg actccatcgt catggacaac gataagctca gatccgaggt tacttccctg     480
accgaaaagc ttcagggcaa acaagagaca gctaatgaac cacctggtca agtgcccgaa     540
ccaaaccaac ttgatccggt ttacattaat gcggcagcaa tcaaaaccga ggaccggtta     600
agttcaggga gcgttgggag cgcggtacta gacgacgacg cacctcaact actagacagc     660
tgtgactctt acttcccaag catcgtaccc atccaagaca acagcaacgc cagtgatcat     720
gacaatgacc ggagctgttt cgccgacgtc tttgtgccca ccacttcacc gtcgcacgat     780
catcacggtg aatcattggc tttctgggga tggccttag                            819

<210>  16
<211>  272
<212>  PRT
<213>  Arabidopsis thaliana

<400>  16
Met Glu Ser Asn Ser Phe Phe Phe Asp Pro Ser Ala Ser His Gly Asn
1               5                   10                  15
Ser Met Phe Phe Leu Gly Asn Leu Asn Pro Val Val Gln Gly Gly Gly
            20                  25                  30
Ala Arg Ser Met Met Asn Met Glu Glu Thr Ser Lys Arg Arg Pro Phe
        35                  40                  45
Phe Ser Ser Pro Glu Asp Leu Tyr Asp Asp Asp Phe Tyr Asp Asp Gln
    50                  55                  60
Leu Pro Glu Lys Lys Arg Arg Leu Thr Thr Glu Gln Val His Leu Leu
65                  70                  75                  80
Glu Lys Ser Phe Glu Thr Glu Asn Lys Leu Glu Pro Glu Arg Lys Thr
                85                  90                  95
Gln Leu Ala Lys Lys Leu Gly Leu Gln Pro Arg Gln Val Ala Val Trp
            100                 105                 110
Phe Gln Asn Arg Arg Ala Arg Trp Lys Thr Lys Gln Leu Glu Arg Asp
        115                 120                 125
Tyr Asp Leu Leu Lys Ser Thr Tyr Asp Gln Leu Leu Ser Asn Tyr Asp
    130                 135                 140
Ser Ile Val Met Asp Asn Asp Lys Leu Arg Ser Glu Val Thr Ser Leu
145                 150                 155                 160
Thr Glu Lys Leu Gln Gly Lys Gln Glu Thr Ala Asn Glu Pro Pro Gly
                165                 170                 175
Gln Val Pro Glu Pro Asn Gln Leu Asp Pro Val Tyr Ile Asn Ala Ala
            180                 185                 190
Ala Ile Lys Thr Glu Asp Arg Leu Ser Ser Gly Ser Val Gly Ser Ala
        195                 200                 205
Val Leu Asp Asp Asp Ala Pro Gln Leu Leu Asp Ser Cys Asp Ser Tyr
    210                 215                 220
Phe Pro Ser Ile Val Pro Ile Gln Asp Asn Ser Asn Ala Ser Asp His
225                 230                 235                 240
Asp Asn Asp Arg Ser Cys Phe Ala Asp Val Phe Val Pro Thr Thr Ser
                245                 250                 255
Pro Ser His Asp His His Gly Glu Ser Leu Ala Phe Trp Gly Trp Pro
            260                 265                 270

<210>  17

<211>  969
<212>  DNA
<213>  Daucus carota

<400>  17
atggcgggtc ggagggtgtt ctatggggag ggagccaata cgacgtcggc tagcctgttg      60
tttcatagtc aaagacctga gcctttcttt ctttctgcac cttctccttc tctaattggt     120
tcaaaatcca tggttagctt tcaagatgct aagcgaaaaa atccctacga tgggttcttt     180
atgcggtcat atgatgaaga agaaattggg gatgaagaat atgatgaata ctttcagcag     240
cctgagaaga agaggaggct caaggctgat caaatccagt ttcttgagaa aagttttgag     300
actgataaca agcttgagcc tgaaagaaaa gttcagcttg caaaagaact cggcttgcag     360
ccaagacagg ttgcgatatg gtttcagaac cgtcgagcac ggtggaagac caaaacacta     420
gaaaaagatt atgatgtatt gcaaaatagc tacaacagcc tcaaggctga ctatgacaat     480
ctacttgccg agaaagaaaa acttaaagcc gaggttctcg acctgacaga caagctactt     540
ctcaaagaag ataaggggag caagacagta gtttttgata agcaaaaggt gtctgcagca     600
ttccaacaag aacgtgttag taatgacata tctgtgggtg aagtactcag taactcagtt     660
atggactgca agcaagaaga tcataactct gtgaaaagtg atgcagttga ttctgacagt     720
ccacactaca gtgatgaagt ctactccagt tttatggagc cagtggatcg ctcttatgtt     780
tttgaacctg ctcagtcgga tatatctcaa gatgaagaag atgacatggg gaacaactta     840
tttctcccat catatcatgt tttctcaaag actgaagacg gtagttactc cgaccagcct     900
tcgaactctt cgtactttgg cttcccagtt gaagatcata cgtttggctt ttggggtact     960
gaattataa                                                             969

<210>  18
<211>  322
<212>  PRT
<213>  Daucus carota

<400>  18
Met Ala Gly Arg Arg Val Phe Tyr Gly Glu Gly Ala Asn Thr Thr Ser
1               5                   10                  15
Ala Ser Leu Leu Phe His Ser Gln Arg Pro Glu Pro Phe Phe Leu Ser
            20                  25                  30
Ala Pro Ser Pro Ser Leu Ile Gly Ser Lys Ser Met Val Ser Phe Gln
        35                  40                  45
Asp Ala Lys Arg Lys Asn Pro Tyr Asp Gly Phe Phe Met Arg Ser Tyr
    50                  55                  60
Asp Glu Glu Glu Ile Gly Asp Glu Glu Tyr Asp Glu Tyr Phe Gln Gln
65                  70                  75                  80
Pro Glu Lys Lys Arg Arg Leu Lys Ala Asp Gln Ile Gln Phe Leu Glu
                85                  90                  95
Lys Ser Phe Glu Thr Asp Asn Lys Leu Glu Pro Glu Arg Lys Val Gln
            100                 105                 110
Leu Ala Lys Glu Leu Gly Leu Gln Pro Arg Gln Val Ala Ile Trp Phe
        115                 120                 125
Gln Asn Arg Arg Ala Arg Trp Lys Thr Lys Thr Leu Glu Lys Asp Tyr
    130                 135                 140
Asp Val Leu Gln Asn Ser Tyr Asn Ser Leu Lys Ala Asp Tyr Asp Asn
145                 150                 155                 160
Leu Leu Ala Glu Lys Glu Lys Leu Lys Ala Glu Val Leu Asp Leu Thr
                165                 170                 175
Asp Lys Leu Leu Leu Lys Glu Asp Lys Gly Ser Lys Thr Val Val Phe
            180                 185                 190
Asp Lys Gln Lys Val Ser Ala Ala Phe Gln Gln Glu Arg Val Ser Asn
        195                 200                 205
Asp Ile Ser Val Gly Glu Val Leu Ser Asn Ser Val Met Asp Cys Lys
    210                 215                 220
Gln Glu Asp His Asn Ser Val Lys Ser Asp Ala Val Asp Ser Asp Ser
225                 230                 235                 240

Pro His Tyr Ser Asp Glu Val Tyr Ser Ser Phe Met Glu Pro Val Asp
                245                 250                 255
Arg Ser Tyr Val Phe Glu Pro Ala Gln Ser Asp Ile Ser Gln Asp Glu
            260                 265                 270
Glu Asp Asp Met Gly Asn Asn Leu Phe Leu Pro Ser Tyr His Val Phe
        275                 280                 285
Ser Lys Thr Glu Asp Gly Ser Tyr Ser Asp Gln Pro Ser Asn Ser Ser
    290                 295                 300
Tyr Phe Gly Phe Pro Val Glu Asp His Thr Phe Gly Phe Trp Gly Thr
305                 310                 315                 320
Glu Leu

<210>  19
<211>  1038
<212>  DNA
<213>  Glycine max

<400>  19
   atggcgagtg gcaagcttta tgcgggttca aacatgtcac ttctcctcca aaacgaaagg      60
   ctcccttgct cctctgaagt ccttgagtct ctttgggctc agacctctaa ccctgcttcc     120
   ttccaaggtt caaaacccgt ggttgatttt gagaatgtaa gtgggagcag gatgacggat     180
   aggcctttct ttcaagcgtt ggagaaggaa gagaactgtg atgaggatta cgaggggtgt     240
   ttccaccaac cggggaagaa aaggaggctc acaagcgaac aagttcagtt ccttgaaagg     300
   aactttgagg tagagaacaa gcttgaaccc gaaaggaaag tccaacttgc aaaagagctt     360
   ggcttgcagc caaggcaagt tgctatatgg ttccaaaacc gaagggcaag gttcaagacc     420
   aagcagctag aaaaagacta tggcgtgttg aaagctagtt atgacagact caaaagtgac     480
   tatgaaagtc ttgttcaaga gaatgacaag ttaaaagcag aggtgaattc tctggagagc     540
   aaattgattc ttagagataa agagaaggag gagaattcgg atgacaagtc atctcctgat     600
   gatgctgtca attcttcttc accccacaac aacaaggagc ctatggattt attaattatt     660
   tcaaaaaatg caacaacaac aacaacatct gaaaatggga ccaaagtgtt gtcaccactc     720
   ccactcccta ttatggtaac atgctgcaag caagaagatg ccaactcagc caaaagtgat     780
   gtccttgatt cggatagccc acattgcact tcattcgtgg agcctgctga ttcctctcat     840
   gcctttgaac cagaagacca ctcagaagac ttctcccaag atgaagagga taaccttagt     900
   gaaaaccttt tgatgacctt cccttcttct tgttgcttac ctaaggttga agaacactgc     960
   tatgacggcc ctcctgaaaa ctcttgtaat tttggcttcc aggttgagga tcaaaccttc    1020
   tgtttctggc cctattga                                                  1038

<210>  20
<211>  345
<212>  PRT
<213>  Glycine max

<400>  20
Met Ala Ser Gly Lys Leu Tyr Ala Gly Ser Asn Met Ser Leu Leu Leu
1               5                   10                  15
Gln Asn Glu Arg Leu Pro Cys Ser Ser Glu Val Leu Glu Ser Leu Trp
            20                  25                  30
Ala Gln Thr Ser Asn Pro Ala Ser Phe Gln Gly Ser Lys Pro Val Val
        35                  40                  45
Asp Phe Glu Asn Val Ser Gly Ser Arg Met Thr Asp Arg Pro Phe Phe
    50                  55                  60
Gln Ala Leu Glu Lys Glu Glu Asn Cys Asp Glu Asp Tyr Glu Gly Cys
65                  70                  75                  80
Phe His Gln Pro Gly Lys Lys Arg Arg Leu Thr Ser Glu Gln Val Gln
                85                  90                  95
Phe Leu Glu Arg Asn Phe Glu Val Glu Asn Lys Leu Glu Pro Glu Arg
            100                 105                 110
Lys Val Gln Leu Ala Lys Glu Leu Gly Leu Gln Pro Arg Gln Val Ala

115                 120                 125
Ile Trp Phe Gln Asn Arg Arg Ala Arg Phe Lys Thr Lys Gln Leu Glu
    130                 135                 140
Lys Asp Tyr Gly Val Leu Lys Ala Ser Tyr Asp Arg Leu Lys Ser Asp
145                 150                 155                 160
Tyr Glu Ser Leu Val Gln Glu Asn Asp Lys Leu Lys Ala Glu Val Asn
                165                 170                 175
Ser Leu Glu Ser Lys Leu Ile Leu Arg Asp Lys Glu Lys Glu Glu Asn
            180                 185                 190
Ser Asp Asp Lys Ser Ser Pro Asp Asp Ala Val Asn Ser Ser Ser Pro
        195                 200                 205
His Asn Asn Lys Glu Pro Met Asp Leu Leu Ile Ile Ser Lys Asn Ala
    210                 215                 220
Thr Thr Thr Thr Thr Ser Glu Asn Gly Thr Lys Val Leu Ser Pro Leu
225                 230                 235                 240
Pro Leu Pro Ile Met Val Thr Cys Cys Lys Gln Glu Asp Ala Asn Ser
                245                 250                 255
Ala Lys Ser Asp Val Leu Asp Ser Asp Ser Pro His Cys Thr Ser Phe
            260                 265                 270
Val Glu Pro Ala Asp Ser Ser His Ala Phe Glu Pro Glu Asp His Ser
        275                 280                 285
Glu Asp Phe Ser Gln Asp Glu Glu Asp Asn Leu Ser Glu Asn Leu Leu
    290                 295                 300
Met Thr Phe Pro Ser Ser Cys Cys Leu Pro Lys Val Glu Glu His Cys
305                 310                 315                 320
Tyr Asp Gly Pro Pro Glu Asn Ser Cys Asn Phe Gly Phe Gln Val Glu
                325                 330                 335
Asp Gln Thr Phe Cys Phe Trp Pro Tyr
            340                 345

<210>  21
<211>  858
<212>  DNA
<213>  Craterostigma plantagineum

<400>  21
atgaactctg ctcggatttt cttcgaccca tcttcccacg gcaacatgct gcagtttctt      60
gggaacgccg gcggcgattc atccgttttc cgaggaacaa gatcgtcgtc ggtgctgaac     120
atggaggaga gctcgttaaa acgacagatt ttcagcggcg gcggcggcga tgaattctac     180
gacgaggaat actacgacga gcagttgttg cctgagaaga agcgccgact caccgccgag     240
caggttcact tgcttgagaa gagcttcgag gctgagaaca agcttgagcc tgagcgaaag     300
gctgagctgg cgaagaagct cggattgcag ccgaggcaag tcgccatttg gttccaaaac     360
cgccgagcac ggtggaagac taagcagtta gagagggact acgacaagct taagtcttcc     420
tatgattctc ttctctcaac ctacgactct attcgccagg aaaacgacaa gctcaaagcc     480
gagctccttt ccctgaacga gaaattgcaa cccaaagacg acgacgaccc atcggccgaa     540
ataggtcgaa atctcagttc atcgtcgccg cctgtcgacg cggctgagcc gccgtgcctg     600
aagctgacgg tgaaggtgga ggaccgcctg agcacgggga gcaacggcag cgcagtaatg     660
gacggcgacg gacctcagca gctcctcgac gacagcggcg actcgtactt cgagaacgac     720
gaggaatacg actgcgccgc cgcaagtttg gctgctgcga aggaggacga cggcagcgat     780
gagggcgggt gttacttcac cgaggctctc gcggcggagg aggaggaggc gccgtttgct     840
tggtgtattt ggtcttaa                                                   858

<210>  22
<211>  285
<212>  PRT
<213>  Craterostigma plantagineum

<400>  22
Met Asn Ser Ala Arg Ile Phe Phe Asp Pro Ser Ser His Gly Asn Met

1               5                   10                  15
Leu Gln Phe Leu Gly Asn Ala Gly Gly Asp Ser Ser Val Phe Arg Gly
            20                  25                  30
Thr Arg Ser Ser Ser Val Leu Asn Met Glu Glu Ser Ser Leu Lys Arg
        35                  40                  45
Gln Ile Phe Ser Gly Gly Gly Gly Asp Glu Phe Tyr Asp Glu Glu Tyr
    50                  55                  60
Tyr Asp Glu Gln Leu Leu Pro Glu Lys Lys Arg Arg Leu Thr Ala Glu
65                  70                  75                  80
Gln Val His Leu Leu Glu Lys Ser Phe Glu Ala Glu Asn Lys Leu Glu
                85                  90                  95
Pro Glu Arg Lys Ala Glu Leu Ala Lys Lys Leu Gly Leu Gln Pro Arg
            100                 105                 110
Gln Val Ala Ile Trp Phe Gln Asn Arg Arg Ala Arg Trp Lys Thr Lys
        115                 120                 125
Gln Leu Glu Arg Asp Tyr Asp Lys Leu Lys Ser Ser Tyr Asp Ser Leu
    130                 135                 140
Leu Ser Thr Tyr Asp Ser Ile Arg Gln Glu Asn Asp Lys Leu Lys Ala
145                 150                 155                 160
Glu Leu Leu Ser Leu Asn Glu Lys Leu Gln Pro Lys Asp Asp Asp Asp
                165                 170                 175
Pro Ser Ala Glu Ile Gly Arg Asn Leu Ser Ser Ser Ser Pro Pro Val
            180                 185                 190
Asp Ala Ala Glu Pro Pro Cys Leu Lys Leu Thr Val Lys Val Glu Asp
        195                 200                 205
Arg Leu Ser Thr Gly Ser Asn Gly Ser Ala Val Met Asp Gly Asp Gly
    210                 215                 220
Pro Gln Gln Leu Leu Asp Asp Ser Gly Asp Ser Tyr Phe Glu Asn Asp
225                 230                 235                 240
Glu Glu Tyr Asp Cys Ala Ala Ala Ser Leu Ala Ala Ala Lys Glu Asp
                245                 250                 255
Asp Gly Ser Asp Glu Gly Gly Cys Tyr Phe Thr Glu Ala Leu Ala Ala
            260                 265                 270
Glu Glu Glu Glu Ala Pro Phe Ala Trp Cys Ile Trp Ser
        275                 280                 285

<210>  23
<211>  813
<212>  DNA
<213>  Gossypium hirsutum

<220>
<221>  variation
<222>  (808)..(808)
<223>  /replace="c" /replace="g" /replace="t"

<400>  23
atggagtctg gccgtctttt tttcaatccc tccactaccc accgcaacat gttgcttctc      60
gggaacactg aacccatctt tcgaggggca agaacaatgg ttagcatgga ggaaaaccca     120
aagaagcgac tgttcttcag ctcgccggag gatttgtacg acgaagagta ctacgacgag     180
cagttgcccg agaaaaagcg tcgccttacg tcggagcagg tgtatctgct agagaagagc     240
tttgaggcag agaacaagct ggagccggag aggaagagcc agttggccaa gaagttagga     300
ctgcaaccaa ggcaggtggc ggtatggttc cagaaccgcc gtgcaaggtg gaagacaaag     360
cagcttgaaa gggactatga cctcctcaaa tcttcctttg attcccttca gtccaattat     420
gacactattc tcaaagaaaa tgagaagctc aaatctgagg tagcttcctt gactgaaaaa     480
ctacaagcca aagatgtggc aacagaagca atagcaggtg aaaaggatga agggttagca     540
gctgagatgg cctccgccct ccaattcagt atgaaggtgg aggaccgtct tagtagcggc     600
agtgtcggaa gcgcggtggt ggatgaggat gccccacagc tggtggacag cggcaattcc     660
tactttccaa gcgatgaata ctccagaggc attggccctt tcgatggggt tcagtcggaa     720

gatgaggatg gcagtgataa ttgcgggagt tacttctccg atgtgttcgc aaccacagag     780
cagggagcat taggattgtg ggcctggatc taa                                  813

<210>  24
<211>  270
<212>  PRT
<213>  Gossypium hirsutum

<220>
<221>  UNSURE
<222>  (270)..(270)
<223>  Xaa can be any naturally occuring amino acid

<400>  24
Met Glu Ser Gly Arg Leu Phe Phe Asn Pro Ser Thr Thr His Arg Asn
1               5                   10                  15
Met Leu Leu Leu Gly Asn Thr Glu Pro Ile Phe Arg Gly Ala Arg Thr
            20                  25                  30
Met Val Ser Met Glu Glu Asn Pro Lys Lys Arg Leu Phe Phe Ser Ser
        35                  40                  45
Pro Glu Asp Leu Tyr Asp Glu Glu Tyr Tyr Asp Glu Gln Leu Pro Glu
    50                  55                  60
Lys Lys Arg Arg Leu Thr Ser Glu Gln Val Tyr Leu Leu Glu Lys Ser
65                  70                  75                  80
Phe Glu Ala Glu Asn Lys Leu Glu Pro Glu Arg Lys Ser Gln Leu Ala
                85                  90                  95
Lys Lys Leu Gly Leu Gln Pro Arg Gln Val Ala Val Trp Phe Gln Asn
            100                 105                 110
Arg Arg Ala Arg Trp Lys Thr Lys Gln Leu Glu Arg Asp Tyr Asp Leu
        115                 120                 125
Leu Lys Ser Ser Phe Asp Ser Leu Gln Ser Asn Tyr Asp Thr Ile Leu
    130                 135                 140
Lys Glu Asn Glu Lys Leu Lys Ser Glu Val Ala Ser Leu Thr Glu Lys
145                 150                 155                 160
Leu Gln Ala Lys Asp Val Ala Thr Glu Ala Ile Ala Gly Glu Lys Asp
                165                 170                 175
Glu Gly Leu Ala Ala Glu Met Ala Ser Ala Leu Gln Phe Ser Met Lys
            180                 185                 190
Val Glu Asp Arg Leu Ser Ser Gly Ser Val Gly Ser Ala Val Val Asp
        195                 200                 205
Glu Asp Ala Pro Gln Leu Val Asp Ser Gly Asn Ser Tyr Phe Pro Ser
    210                 215                 220
Asp Glu Tyr Ser Arg Gly Ile Gly Pro Phe Asp Gly Val Gln Ser Glu
225                 230                 235                 240
Asp Glu Asp Gly Ser Asp Asn Cys Gly Ser Tyr Phe Ser Asp Val Phe
                245                 250                 255
Ala Thr Thr Glu Gln Gly Ala Leu Gly Leu Trp Ala Trp Xaa
            260                 265                 270

<210>  25
<211>  858
<212>  DNA
<213>  Lycopersicon esculentum

<220>
<221>  variation
<222>  (294)..(294)
<223>  /replace="c" /replace="g" /replace="t"

<400>  25
atgggatctg ggcatatatt tttcgacccg tcgtcgtgtc acggcaacat gctgttcctt      60
gggagcggag atcctgtttt ccgaggacca agatcgacga tgatgaagat ggaggactcc     120
tcgaagaggc gacccttctt tagctcgccg gaggatctat atgacgagga atactacgac     180
gagcagtcac cggagaagaa gcgccgtctc actcctgagc aggtgcactt gttggagaag     240
agctttgaga cagaaaacaa gctggagccc gagcgcaaaa cccagctggc ctaaaagctg     300
gggctgcagc ccagacaggt ggctgtatgg ttccaaaacc gccgtgcccg gtggaagacc     360
aagcagctcg agagggatta tgatcagctc aaatcctctt atgactccct tctctctgat     420
tttgactccg ttcgcaaaga taacgataag ctcaaatctg aggttgtttc attgatggaa     480
aagttacagg ggaaagtggt tggaggagca gggggaaatg aaaaatctga catcttggag     540
gtggatgcta tgacgatcct tcaagtgaag gtgaaggctg gggaccggtt gagcagtggc     600
agtggtggga gcgcggtggt agatgagcat agttcacagc tggtggacag tggggactca     660
tattttcaca ctgatcatga ggagtatcca gggcctggag gatgcaatgt tcctccaccc     720
atggatggtt tacaatcgga ggaagatgat ggtagtgatg atcatggcag ttgccatggc     780
tacttctcta acgtctttgt ggcagaagag cagcaccatg aacaaggaga agagcctatt     840
ggatggttct ggtcttaa                                                   858

<210>  26
<211>  285
<212>  PRT
<213>  Lycopersicon esculentum

<220>
<221>  UNSURE
<222>  (98)..(98)
<223>  Xaa can be any naturally occuring amino acid

<400>  26
Met Gly Ser Gly His Ile Phe Phe Asp Pro Ser Ser Cys His Gly Asn
1               5                   10                  15
Met Leu Phe Leu Gly Ser Gly Asp Pro Val Phe Arg Gly Pro Arg Ser
            20                  25                  30
Thr Met Met Lys Met Glu Asp Ser Ser Lys Arg Arg Pro Phe Phe Ser
        35                  40                  45
Ser Pro Glu Asp Leu Tyr Asp Glu Glu Tyr Tyr Asp Glu Gln Ser Pro
    50                  55                  60
Glu Lys Lys Arg Arg Leu Thr Pro Glu Gln Val His Leu Leu Glu Lys
65                  70                  75                  80
Ser Phe Glu Thr Glu Asn Lys Leu Glu Pro Glu Arg Lys Thr Gln Leu
                85                  90                  95
Ala Xaa Lys Leu Gly Leu Gln Pro Arg Gln Val Ala Val Trp Phe Gln
            100                 105                 110
Asn Arg Arg Ala Arg Trp Lys Thr Lys Gln Leu Glu Arg Asp Tyr Asp
        115                 120                 125
Gln Leu Lys Ser Ser Tyr Asp Ser Leu Leu Ser Asp Phe Asp Ser Val
    130                 135                 140
Arg Lys Asp Asn Asp Lys Leu Lys Ser Glu Val Val Ser Leu Met Glu
145                 150                 155                 160
Lys Leu Gln Gly Lys Val Val Gly Gly Ala Gly Gly Asn Glu Lys Ser
                165                 170                 175
Asp Ile Leu Glu Val Asp Ala Met Thr Ile Leu Gln Val Lys Val Lys
            180                 185                 190
Ala Gly Asp Arg Leu Ser Ser Gly Ser Gly Gly Ser Ala Val Val Asp
        195                 200                 205
Glu His Ser Ser Gln Leu Val Asp Ser Gly Asp Ser Tyr Phe His Thr
    210                 215                 220
Asp His Glu Glu Tyr Pro Gly Pro Gly Gly Cys Asn Val Pro Pro Pro
225                 230                 235                 240
Met Asp Gly Leu Gln Ser Glu Glu Asp Asp Gly Ser Asp Asp His Gly

245                 250                 255
Ser Cys His Gly Tyr Phe Ser Asn Val Phe Val Ala Glu Glu Gln His
            260                 265                 270
His Glu Gln Gly Glu Glu Pro Ile Gly Trp Phe Trp Ser
        275                 280                 285

<210>  27
<211>  972
<212>  DNA
<213>  Lycopersicon esculentum

<400>  27
atggctccag ggattctcta tggtggttct tctaatttcg atggcgtttt tactcaaaaa      60
cagagagacg tgttttcttc atctactgca ccgaaagggc atcttggttc cctttttgcc    120
cctgcctctt cttcttctaa tttcttggga tccagttcta tggtgagttt tcgcggtgtt     180
aatggaggga agagatcatt ctttgattcg ttcgatcagg atgacaatga agctgatgaa     240
ttgggggaat atcttcatca agcggagaag aagaggcgac ttactgacaa ccaagttcag     300
tttcttgaga agagttttgg ggaagagaac aaacttgaac cagaaagaaa agttcagctt     360
gctaaagaac ttggtctgca gcctcgccaa attgcaattt ggtttcagaa tcgtcgtgcg     420
cgatggaaga ctaagcagct cgagaaagat tatgatgaat tgaggaatag atacgatact     480
ctgaaatcaa attacaataa tcttctcaag gaaaaagaag atcttcgaac tgaagttttc     540
cgtctcaccg gtaagctgtt tatcaaagag aaaggaaatg ggcaattgga tttgcgcgat     600
gaacacaaac actccaatgc attggcaaaa gaaaccgtgg ttgatccaat gtccaatgta     660
ccagctctgg ttgttaagca ccagcaggaa gatttaagct ctgctaagag tgatgttttc     720
gactcagaaa gcccacgtta caccagtaga atgcattcct cagtcgtaga tcaggatgat     780
tctgctcgcg catttgaaac tgatcagtcg gattcatctc aggatgatga tgaaaacttc     840
agcaagaata tgctttctac tgccaaccta cttggcaaag acgcggatga tgattatccc     900
gcgacatcat caaatttgag ttactttgga tttccagttg aagaccaagg ttttggtttc     960
tggacttatt aa                                                         972

<210>  28
<211>  323
<212>  PRT
<213>  Lycopersicon esculentum

<400>  28
Met Ala Pro Gly Ile Leu Tyr Gly Gly Ser Ser Asn Phe Asp Gly Val
1               5                   10                  15
Phe Thr Gln Lys Gln Arg Asp Val Phe Ser Ser Ser Thr Ala Pro Lys
            20                  25                  30
Gly His Leu Gly Ser Leu Phe Ala Pro Ala Ser Ser Ser Ser Asn Phe
        35                  40                  45
Leu Gly Ser Ser Ser Met Val Ser Phe Arg Gly Val Asn Gly Gly Lys
    50                  55                  60
Arg Ser Phe Phe Asp Ser Phe Asp Gln Asp Asp Asn Glu Ala Asp Glu
65                  70                  75                  80
Leu Gly Glu Tyr Leu His Gln Ala Glu Lys Lys Arg Arg Leu Thr Asp
                85                  90                  95
Asn Gln Val Gln Phe Leu Glu Lys Ser Phe Gly Glu Glu Asn Lys Leu
            100                 105                 110
Glu Pro Glu Arg Lys Val Gln Leu Ala Lys Glu Leu Gly Leu Gln Pro
        115                 120                 125
Arg Gln Ile Ala Ile Trp Phe Gln Asn Arg Arg Ala Arg Trp Lys Thr
    130                 135                 140
Lys Gln Leu Glu Lys Asp Tyr Asp Glu Leu Arg Asn Arg Tyr Asp Thr
145                 150                 155                 160
Leu Lys Ser Asn Tyr Asn Asn Leu Leu Lys Glu Lys Glu Asp Leu Arg
                165                 170                 175
Thr Glu Val Phe Arg Leu Thr Gly Lys Leu Phe Ile Lys Glu Lys Gly

180                 185                 190
Asn Gly Gln Leu Asp Leu Arg Asp Glu His Lys His Ser Asn Ala Leu
        195                 200                 205
Ala Lys Glu Thr Val Val Asp Pro Met Ser Asn Val Pro Ala Leu Val
    210                 215                 220
Val Lys His Gln Gln Glu Asp Leu Ser Ser Ala Lys Ser Asp Val Phe
225                 230                 235                 240
Asp Ser Glu Ser Pro Arg Tyr Thr Ser Arg Met His Ser Ser Val Val
                245                 250                 255
Asp Gln Asp Asp Ser Ala Arg Ala Phe Glu Thr Asp Gln Ser Asp Ser
            260                 265                 270
Ser Gln Asp Asp Asp Glu Asn Phe Ser Lys Asn Met Leu Ser Thr Ala
        275                 280                 285
Asn Leu Leu Gly Lys Asp Ala Asp Asp Asp Tyr Pro Ala Thr Ser Ser
    290                 295                 300
Asn Leu Ser Tyr Phe Gly Phe Pro Val Glu Asp Gln Gly Phe Gly Phe
305                 310                 315                 320
Trp Thr Tyr

<210>  29
<211>  1014
<212>  DNA
<213>  Medicago sativa

<400>  29
atggcgggtg ggagagtttt ttcaaatggt cctgcaaata tttcaaatat aaatatgaat      60
attttgcttc agaatcaaca acaaactcct cgtggaaact cttctcaaca acctcttgat     120
tctcttttcc tttcttcttc tgcttctttc tttggttcaa gatctatggt gagttttgaa     180
gatgttcaag gaaggaaaag gcgcaacagg tctttctttg gaggatttga tcttgacgaa     240
aacggagagg atgagatgga tgagtacttt catcaatccg agaagaaacg gcgtctctca     300
gtggatcaag ttcagtttct tgagaaaagc tttgaggagg acaacaaact tgaaccagag     360
aggaaaacca agctagctaa agaccttggt ttgcagccac ggcaagttgc tatttggttt     420
caaaaccgtc gtgcaaggtg gaagactaaa cagcttgaga aggattatga ttctcttaat     480
gatggttatg agtctcttaa gacagagtat gacaaccttc tcaaagagaa agataggtta     540
caatctgagg tggcaagcct aactgaaaag gtacttgaaa gagagaaaca agagggaaaa     600
ttcaaacaag gtgaaagtga aacaaaggaa ttcttgaagg aaccaacaat taataagcct     660
ttggttgatt cagtttctga gggtgaagga tccaaattgt caattgttga ggcttctaat     720
aataataata ataataacaa acttgaagat attagttcag caaggagtga catattggat     780
tgtgaaagtc cacgctacac tgatggagtg ttagagacat gtgattcttc ctatgtattt     840
gaacctgaat atcaatcgga cctatcacaa gatgaagaag atcacaattt attgcctcct     900
tacatcttta caaaacttga agatgtgaat tactccgacc cgccacataa ttcaacaagt     960
tatggatttc aagaggaaga tcatcatcaa gctctttggc cttggtctta ttag          1014

<210>  30
<211>  337
<212>  PRT
<213>  Medicago sativa

<400>  30
Met Ala Gly Gly Arg Val Phe Ser Asn Gly Pro Ala Asn Ile Ser Asn
1               5                   10                  15
Ile Asn Met Asn Ile Leu Leu Gln Asn Gln Gln Gln Thr Pro Arg Gly
            20                  25                  30
Asn Ser Ser Gln Gln Pro Leu Asp Ser Leu Phe Leu Ser Ser Ser Ala
        35                  40                  45
Ser Phe Phe Gly Ser Arg Ser Met Val Ser Phe Glu Asp Val Gln Gly
    50                  55                  60
Arg Lys Arg Arg Asn Arg Ser Phe Phe Gly Gly Phe Asp Leu Asp Glu

65                  70                  75                  80
Asn Gly Glu Asp Glu Met Asp Glu Tyr Phe His Gln Ser Glu Lys Lys
                85                  90                  95
Arg Arg Leu Ser Val Asp Gln Val Gln Phe Leu Glu Lys Ser Phe Glu
            100                 105                 110
Glu Asp Asn Lys Leu Glu Pro Glu Arg Lys Thr Lys Leu Ala Lys Asp
        115                 120                 125
Leu Gly Leu Gln Pro Arg Gln Val Ala Ile Trp Phe Gln Asn Arg Arg
    130                 135                 140
Ala Arg Trp Lys Thr Lys Gln Leu Glu Lys Asp Tyr Asp Ser Leu Asn
145                 150                 155                 160
Asp Gly Tyr Glu Ser Leu Lys Thr Glu Tyr Asp Asn Leu Leu Lys Glu
                165                 170                 175
Lys Asp Arg Leu Gln Ser Glu Val Ala Ser Leu Thr Glu Lys Val Leu
            180                 185                 190
Glu Arg Glu Lys Gln Glu Gly Lys Phe Lys Gln Gly Glu Ser Glu Thr
        195                 200                 205
Lys Glu Phe Leu Lys Glu Pro Thr Ile Asn Lys Pro Leu Val Asp Ser
    210                 215                 220
Val Ser Glu Gly Glu Gly Ser Lys Leu Ser Ile Val Glu Ala Ser Asn
225                 230                 235                 240
Asn Asn Asn Asn Asn Asn Lys Leu Glu Asp Ile Ser Ser Ala Arg Ser
                245                 250                 255
Asp Ile Leu Asp Cys Glu Ser Pro Arg Tyr Thr Asp Gly Val Leu Glu
            260                 265                 270
Thr Cys Asp Ser Ser Tyr Val Phe Glu Pro Glu Tyr Gln Ser Asp Leu
        275                 280                 285
Ser Gln Asp Glu Glu Asp His Asn Leu Leu Pro Pro Tyr Ile Phe Thr
    290                 295                 300
Lys Leu Glu Asp Val Asn Tyr Ser Asp Pro Pro His Asn Ser Thr Ser
305                 310                 315                 320
Tyr Gly Phe Gln Glu Glu Asp His His Gln Ala Leu Trp Pro Trp Ser
                325                 330                 335
Tyr


<210>  31
<211>  843
<212>  DNA
<213>  Aquilegia formosa x Aquilegia pubescens

<400>  31
atggattcaa caacaagccg tcttttcttt gatggttcct gccatgggaa catgttgctt      60
ttagggagtg gagatcccgt tcttcgaggt tcaagatcat tcattaatat ggaagattct     120
ttgaaaagac gtccttttta tagttcaaca gatgaactaa ttgaagagga gttttatgat     180
gaacagctac ctgaaaagaa acgtcgtctt acttctgagc aggttcatct attggagaag     240
agctttgaga cagagaacaa gctggaacca gatcgtaaga cccagcttgc taagaagctt     300
gggttgcaac cgagacaagt tgcagtttgg tttcagaata gacgagctcg ttggaagact     360
aagcaactag agagagatta tgatcttctt aaagcttctt atgattccct tcgttctgat     420
tacgatgaca ttgttaaaga gaatgagaag ctcaaatctg aggtggtttc cttaactggg     480
aagttgcagg tcaaggaggg agctgggatg gagttaaatc agatatctga cccaccactc     540
tccactgaag aaaatgttga tgtaactacg atgcaattta atgttaaggt tgaggatcgc     600
ttgagctctg gcagtggggt aagtgctgtg gttgatgagg aatgtcgaca gcttgttgac     660
agtgttgatt cctatttccc tggcgatgac tatggtcaat gcataggccc agtagatgga     720
gtccagtcag aagaagatga cattagtgac gacagccgga gctatttctc agatgtcttt     780
ccagctgcac cagagcagaa ccaccaggag agtgagacat tgggttggtg ggactgggct     840
taa                                                                   843

<210>  32

<211>  280
<212>  PRT
<213>  Aquilegia formosa x Aquilegia pubescens

<400>  32
Met Asp Ser Thr Thr Ser Arg Leu Phe Phe Asp Gly Ser Cys His Gly
1               5                   10                  15
Asn Met Leu Leu Leu Gly Ser Gly Asp Pro Val Leu Arg Gly Ser Arg
            20                  25                  30
Ser Phe Ile Asn Met Glu Asp Ser Leu Lys Arg Arg Pro Phe Tyr Ser
        35                  40                  45
Ser Thr Asp Glu Leu Ile Glu Glu Glu Phe Tyr Asp Glu Gln Leu Pro
    50                  55                  60
Glu Lys Lys Arg Arg Leu Thr Ser Glu Gln Val His Leu Leu Glu Lys
65                  70                  75                  80
Ser Phe Glu Thr Glu Asn Lys Leu Glu Pro Asp Arg Lys Thr Gln Leu
                85                  90                  95
Ala Lys Lys Leu Gly Leu Gln Pro Arg Gln Val Ala Val Trp Phe Gln
            100                 105                 110
Asn Arg Arg Ala Arg Trp Lys Thr Lys Gln Leu Glu Arg Asp Tyr Asp
        115                 120                 125
Leu Leu Lys Ala Ser Tyr Asp Ser Leu Arg Ser Asp Tyr Asp Asp Ile
    130                 135                 140
Val Lys Glu Asn Glu Lys Leu Lys Ser Glu Val Val Ser Leu Thr Gly
145                 150                 155                 160
Lys Leu Gln Val Lys Glu Gly Ala Gly Met Glu Leu Asn Gln Ile Ser
                165                 170                 175
Asp Pro Pro Leu Ser Thr Glu Glu Asn Val Asp Val Thr Thr Met Gln
            180                 185                 190
Phe Asn Val Lys Val Glu Asp Arg Leu Ser Ser Gly Ser Gly Val Ser
        195                 200                 205
Ala Val Val Asp Glu Glu Cys Arg Gln Leu Val Asp Ser Val Asp Ser
    210                 215                 220
Tyr Phe Pro Gly Asp Asp Tyr Gly Gln Cys Ile Gly Pro Val Asp Gly
225                 230                 235                 240
Val Gln Ser Glu Glu Asp Asp Ile Ser Asp Asp Ser Arg Ser Tyr Phe
                245                 250                 255
Ser Asp Val Phe Pro Ala Ala Pro Glu Gln Asn His Gln Glu Ser Glu
            260                 265                 270
Thr Leu Gly Trp Trp Asp Trp Ala
        275                 280

<210>  33
<211>  2193
<212>  DNA
<213>  Oryza sativa

<400>  33
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660

tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga attttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 34
<211> 49
<212> DNA
<213> Artificial sequence

<220>
<223> primer: prm6000

<400> 34
ggggacaagt ttgtacaaaa aagcaggctt aaacaatgga tcccggccg 49

<210> 35
<211> 48
<212> DNA
<213> Artificial sequence

<220>
<223> primer: prm6001

<400> 35
ggggaccact ttgtacaaga aagctgggtg atcagctcca gaaccagg 48

<210> 36
<211> 834
<212> DNA
<213> Oryza sativa

<400> 36
atgaagcgac ccggcggtgc cggcggcggc ggaggcagcc catcgctcgt cacgatggct 60
aattctagtg atgatggata tggaggggtt gggatggagg cggaggggga cgtggaggag 120
gagatgatgg cgtgcggcgg cggcggggag aagaagcggc ggctgagcgt ggagcaggtt 180
cgcgcgctgg agcggagctt cgaggtggag aacaagcttg agcctgagcg gaaggcgcgg 240

ctggcgcgcg acctcggcct gcagccgcgc caggtcgccg tctggttcca gaaccgccgc 300
gcgcggtgga agaccaagca gctcgagcgc gactacgccg cgctccgcca ttcctacgac 360
tccctgcgcc tcgatcacga cgcgctccgc cgcgacaagg acgccctcct cgccgagatc 420
aaggagctga aggcgaagct cggggacgag gaggcggcgg cgagcttcac gtcggtgaag 480
gaggagccgg cggcctccga cgggccaccg gcggcgggat ttgggtcgtc cgacagcgac 540
tcaagcgcgg tgctgaacga cgtggacgcg gccggcgccg cgcccgcggc gacggacgcg 600
ctggctccgg aggcgtgcac gtttctcggt gcgccgcccg ccgcgggcgc gggcgcgggc 660
gcagcggcgg cggcgagcca cgaggaggtg ttcttccacg gcaatttcct caaggtggag 720
gaggacgaga cggggttcct cgacgacgac gagccgtgcg gcgggttctt cgccgacgat 780
cagcccccgc cgctctcgtc gtggtgggcc gaacccacgg agcactggaa ctga 834

<210> 37
<211> 277
<212> PRT
<213> Oryza sativa

<400> 37
Met Lys Arg Pro Gly Gly Ala Gly Gly Gly Gly Gly Ser Pro Ser Leu
1               5                   10                  15
Val Thr Met Ala Asn Ser Ser Asp Asp Gly Tyr Gly Gly Val Gly Met
            20                  25                  30
Glu Ala Glu Gly Asp Val Glu Glu Glu Met Met Ala Cys Gly Gly Gly
        35                  40                  45
Gly Glu Lys Lys Arg Arg Leu Ser Val Glu Gln Val Arg Ala Leu Glu
    50                  55                  60
Arg Ser Phe Glu Val Glu Asn Lys Leu Glu Pro Glu Arg Lys Ala Arg
65                  70                  75                  80
Leu Ala Arg Asp Leu Gly Leu Gln Pro Arg Gln Val Ala Val Trp Phe
                85                  90                  95
Gln Asn Arg Arg Ala Arg Trp Lys Thr Lys Gln Leu Glu Arg Asp Tyr
            100                 105                 110
Ala Ala Leu Arg His Ser Tyr Asp Ser Leu Arg Leu Asp His Asp Ala
        115                 120                 125
Leu Arg Arg Asp Lys Asp Ala Leu Leu Ala Glu Ile Lys Glu Leu Lys
    130                 135                 140
Ala Lys Leu Gly Asp Glu Glu Ala Ala Ala Ser Phe Thr Ser Val Lys
145                 150                 155                 160
Glu Glu Pro Ala Ala Ser Asp Gly Pro Pro Ala Ala Gly Phe Gly Ser
                165                 170                 175
Ser Asp Ser Asp Ser Ser Ala Val Leu Asn Asp Val Asp Ala Ala Gly
            180                 185                 190
Ala Ala Pro Ala Ala Thr Asp Ala Leu Ala Pro Glu Ala Cys Thr Phe
        195                 200                 205
Leu Gly Ala Pro Pro Ala Ala Gly Ala Gly Ala Gly Ala Ala Ala Ala
    210                 215                 220
Ala Ser His Glu Glu Val Phe Phe His Gly Asn Phe Leu Lys Val Glu
225                 230                 235                 240
Glu Asp Glu Thr Gly Phe Leu Asp Asp Asp Glu Pro Cys Gly Gly Phe
                245                 250                 255
Phe Ala Asp Asp Gln Pro Pro Pro Leu Ser Ser Trp Trp Ala Glu Pro
            260                 265                 270
Thr Glu His Trp Asn
        275

<210> 38
<211> 750
<212> DNA
<213> Oryza sativa

<400>  38
atggatgggg aggaggacag cgagtggatg atgatggacg ttggagggaa gggcgggaag      60
ggcggcggcg gcggcggcgc ggcggacagg aagaagcggt tcagcgagga gcagatcaag     120
tcgctggagt ccatgttcgc gacgcagacc aagctggagc cgaggcagaa gctgcagctc     180
gccagggagc tcggcctgca gcctcgccag gtcgccatct ggttccagaa caagcgcgcg     240
cggtggaagt ccaagcagct cgagcgcgag tactccgccc tccgcgacga ctacgacgcc     300
ctcctctgca gctacgagtc cctcaagaag gagaagctcg ccctcatcaa gcagctggag     360
aagctggcgg agatgctgca ggagccacgg gggaagtacg gcgataatgc cggggacgac     420
gcgcggtcgg gcggcgtcgc cggcatgaag aaggaggagt tcgtcggcgc gggcggcgcc     480
gccacgctct actcgtcggc cgagggtggc gggacgacga cgacgacgac ggccaagttg     540
atgccccact tcggcagcga cgacgtcgac gcggggctct tcctccggcc gtcgtcgcag     600
catcatccgc cgccgccgca cgccggtgcc ggcttcacgt cctccgagcc ggccgccgac     660
caccagtcct tcaacttcca ctcgagctgg ccgtcgtcca cggagcagac ctgcagcagc     720
acgccatggt gggaattcga gagcgagtga                                      750

<210>  39
<211>  249
<212>  PRT
<213>  Oryza sativa

<400>  39
Met Asp Gly Glu Glu Asp Ser Glu Trp Met Met Met Asp Val Gly Gly
1               5                   10                  15
Lys Gly Gly Lys Gly Gly Gly Gly Gly Gly Ala Ala Asp Arg Lys Lys
            20                  25                  30
Arg Phe Ser Glu Glu Gln Ile Lys Ser Leu Glu Ser Met Phe Ala Thr
        35                  40                  45
Gln Thr Lys Leu Glu Pro Arg Gln Lys Leu Gln Leu Ala Arg Glu Leu
    50                  55                  60
Gly Leu Gln Pro Arg Gln Val Ala Ile Trp Phe Gln Asn Lys Arg Ala
65                  70                  75                  80
Arg Trp Lys Ser Lys Gln Leu Glu Arg Glu Tyr Ser Ala Leu Arg Asp
                85                  90                  95
Asp Tyr Asp Ala Leu Leu Cys Ser Tyr Glu Ser Leu Lys Lys Glu Lys
            100                 105                 110
Leu Ala Leu Ile Lys Gln Leu Glu Lys Leu Ala Glu Met Leu Gln Glu
        115                 120                 125
Pro Arg Gly Lys Tyr Gly Asp Asn Ala Gly Asp Asp Ala Arg Ser Gly
    130                 135                 140
Gly Val Ala Gly Met Lys Lys Glu Glu Phe Val Gly Ala Gly Gly Ala
145                 150                 155                 160
Ala Thr Leu Tyr Ser Ser Ala Glu Gly Gly Gly Thr Thr Thr Thr Thr
                165                 170                 175
Thr Ala Lys Leu Met Pro His Phe Gly Ser Asp Asp Val Asp Ala Gly
            180                 185                 190
Leu Phe Leu Arg Pro Ser Ser Gln His His Pro Pro Pro Pro His Ala
        195                 200                 205
Gly Ala Gly Phe Thr Ser Ser Glu Pro Ala Ala Asp His Gln Ser Phe
    210                 215                 220
Asn Phe His Ser Ser Trp Pro Ser Ser Thr Glu Gln Thr Cys Ser Ser
225                 230                 235                 240
Thr Pro Trp Trp Glu Phe Glu Ser Glu
                245

<210>  40
<211>  945
<212>  DNA
<213>  Populus tremuloides

<400>  40
atggcgggtg gtaccggtgg ttctaattcc aatttgtctg ttttgcttca aagccaaaga      60
ggcccttgtg ctgcttcaca acctcttgaa tcttttttcc tttctggctc ttctccttct      120
tttcttggtt caagatccat gatgagtttt gaagatgttc atcaagcaaa cggatcaacc      180
aggccttttt tccgctcgtt tgatcacgaa gacaatggag acgatgatct ggatgaatat      240
tttcatcaac ctgaaaagaa gaggagactt actgttgatc aagttcagtt tcttgaaaag      300
agttttgagc ttgagaacaa gcttgaacct gaaaggaaaa tccagcttgc aaaggatctt      360
ggccttcagc cgcgtcaggt tgctatatgg tttcaaaacc gccgagcaag atggaagact      420
aaacagctgg aaaaggatta tgacgttttg caatctagct acaatagcct taaggctgac      480
tatgacaacc tcctcaagga gaaggagaaa ctaaaagctg aggttaatct tctcaccgac      540
aagttgctcc tcaaagagaa agagaaggga atctcagaat tgtctgataa agatgcatta      600
tcgcaagagc cacctaaaag ggctatagct gattcagctt ccgagggtga agtgtcgaaa      660
atctcaacag tggcctgtaa gcaggaagat atcagctcag ccaaaagcga catatttgat      720
tcagacagcc cacattacgc tgatggggtg cattcctcac tcttagaggc aggagattct      780
tcatatgttt tcgaacccga tcaatcagat ttgtcacaag atgaagaaga taactttagc      840
aagagcttat tgcctccata cgtctttccg aagcttgaag atgacgatta ctctgacccg      900
cctgcaagtt ttgaagatca tgccttttgg tcctggtcat actaa                     945

<210>  41
<211>  314
<212>  PRT
<213>  Populus tremuloides

<400>  41
Met Ala Gly Gly Thr Gly Gly Ser Asn Ser Asn Leu Ser Val Leu Leu
1               5                   10                  15
Gln Ser Gln Arg Gly Pro Cys Ala Ala Ser Gln Pro Leu Glu Ser Phe
            20                  25                  30
Phe Leu Ser Gly Ser Ser Pro Ser Phe Leu Gly Ser Arg Ser Met Met
        35                  40                  45
Ser Phe Glu Asp Val His Gln Ala Asn Gly Ser Thr Arg Pro Phe Phe
    50                  55                  60
Arg Ser Phe Asp His Glu Asp Asn Gly Asp Asp Asp Leu Asp Glu Tyr
65                  70                  75                  80
Phe His Gln Pro Glu Lys Lys Arg Arg Leu Thr Val Asp Gln Val Gln
                85                  90                  95
Phe Leu Glu Lys Ser Phe Glu Leu Glu Asn Lys Leu Glu Pro Glu Arg
            100                 105                 110
Lys Ile Gln Leu Ala Lys Asp Leu Gly Leu Gln Pro Arg Gln Val Ala
        115                 120                 125
Ile Trp Phe Gln Asn Arg Arg Ala Arg Trp Lys Thr Lys Gln Leu Glu
    130                 135                 140
Lys Asp Tyr Asp Val Leu Gln Ser Ser Tyr Asn Ser Leu Lys Ala Asp
145                 150                 155                 160
Tyr Asp Asn Leu Leu Lys Glu Lys Glu Lys Leu Lys Ala Glu Val Asn
                165                 170                 175
Leu Leu Thr Asp Lys Leu Leu Leu Lys Glu Lys Glu Lys Gly Ile Ser
            180                 185                 190
Glu Leu Ser Asp Lys Asp Ala Leu Ser Gln Glu Pro Pro Lys Arg Ala
        195                 200                 205
Ile Ala Asp Ser Ala Ser Glu Gly Glu Val Ser Lys Ile Ser Thr Val
    210                 215                 220
Ala Cys Lys Gln Glu Asp Ile Ser Ser Ala Lys Ser Asp Ile Phe Asp
225                 230                 235                 240
Ser Asp Ser Pro His Tyr Ala Asp Gly Val His Ser Ser Leu Leu Glu
                245                 250                 255
Ala Gly Asp Ser Ser Tyr Val Phe Glu Pro Asp Gln Ser Asp Leu Ser
            260                 265                 270
Gln Asp Glu Glu Asp Asn Phe Ser Lys Ser Leu Leu Pro Pro Tyr Val

        275                 280                 285
Phe Pro Lys Leu Glu Asp Asp Asp Tyr Ser Asp Pro Pro Ala Ser Phe
    290                 295                 300
Glu Asp His Ala Phe Trp Ser Trp Ser Tyr
305                 310

<210>  42
<211>  954
<212>  DNA
<213>  Populus tremuloides

<400>  42
atggcggctt gtggtggtgg tggtggtggt tctaatccca atttgtctgt tttagttcaa      60
agccaaagag gcccttgtgc tgcttctcaa cctcttgaag cttttttcct ttctggctct     120
tctccttctt ttcttggttc aagatccatg atgagttttg cagatgttca ccaagcaaat     180
ggatcaacta gaccgttttt ccgcccatat gatcacgaag acaacggcga cgatgatttg     240
gatgaatatt ttcatcaacc tgaaaagaag aggagactta ctgttgatca agttcagttt     300
cttgaaagaa gttttgaggt tgagaacaag cttgaacccg aaaggaaaat ccagctggcg     360
aaggatcttg gcttgcagcc tcggcaggtt gccatatggt ttcaaaaccg ccgggcaaga     420
tggaagacga aacagcttga aaaagattat gaggttctgc aatctagcta caatggcctt     480
aaggctgact acgacaacct cttcaaggag aaggagaaac taaaagctga ggttaatctt     540
ctcaccaacg agttgctcct taaagagaaa gagaaaggaa gctcagaatt gtctgataaa     600
gatgcattat ctcaagagcc acccaaaaag gcaatagccg attcagcttc agagggtgaa      660
gtgtcgaaaa cttcaaccgt ggcctgccag caggaagata ttagctcagc caaaagtgat     720
atgtttgatt cagacagccc acattttgcg gatggggtac attcctcact cttagaggca     780
ggtgattctt cacatgtctt cgagcccgac caatcggatt tatcacaaga tgaagaagat     840
aacttgagca agagtctttt gcctccgtac gtctttccaa agcttgaaga tggtgattac     900
tctgacccgc cagcaagttt tgaagatcat gccttttggt gctggtcata ctaa           954

<210>  43
<211>  317
<212>  PRT
<213>  Populus tremuloides

<400>  43
Met Ala Ala Cys Gly Gly Gly Gly Gly Gly Ser Asn Pro Asn Leu Ser
1               5                   10                  15
Val Leu Val Gln Ser Gln Arg Gly Pro Cys Ala Ala Ser Gln Pro Leu
            20                  25                  30
Glu Ala Phe Phe Leu Ser Gly Ser Ser Pro Ser Phe Leu Gly Ser Arg
        35                  40                  45
Ser Met Met Ser Phe Ala Asp Val His Gln Ala Asn Gly Ser Thr Arg
    50                  55                  60
Pro Phe Phe Arg Pro Tyr Asp His Glu Asp Asn Gly Asp Asp Asp Leu
65                  70                  75                  80
Asp Glu Tyr Phe His Gln Pro Glu Lys Lys Arg Arg Leu Thr Val Asp
                85                  90                  95
Gln Val Gln Phe Leu Glu Arg Ser Phe Glu Val Glu Asn Lys Leu Glu
            100                 105                 110
Pro Glu Arg Lys Ile Gln Leu Ala Lys Asp Leu Gly Leu Gln Pro Arg
        115                 120                 125
Gln Val Ala Ile Trp Phe Gln Asn Arg Arg Ala Arg Trp Lys Thr Lys
    130                 135                 140
Gln Leu Glu Lys Asp Tyr Glu Val Leu Gln Ser Ser Tyr Asn Gly Leu
145                 150                 155                 160
Lys Ala Asp Tyr Asp Asn Leu Phe Lys Glu Lys Glu Lys Leu Lys Ala
                165                 170                 175
Glu Val Asn Leu Leu Thr Asn Glu Leu Leu Leu Lys Glu Lys Glu Lys
            180                 185                 190

Gly Ser Ser Glu Leu Ser Asp Lys Asp Ala Leu Ser Gln Glu Pro Pro
        195                 200                 205
Lys Lys Ala Ile Ala Asp Ser Ala Ser Glu Gly Glu Val Ser Lys Thr
    210                 215                 220
Ser Thr Val Ala Cys Gln Gln Glu Asp Ile Ser Ser Ala Lys Ser Asp
225                 230                 235                 240
Met Phe Asp Ser Asp Ser Pro His Phe Ala Asp Gly Val His Ser Ser
                245                 250                 255
Leu Leu Glu Ala Gly Asp Ser Ser His Val Phe Glu Pro Asp Gln Ser
            260                 265                 270
Asp Leu Ser Gln Asp Glu Glu Asp Asn Leu Ser Lys Ser Leu Leu Pro
        275                 280                 285
Pro Tyr Val Phe Pro Lys Leu Glu Asp Gly Asp Tyr Ser Asp Pro Pro
    290                 295                 300
Ala Ser Phe Glu Asp His Ala Phe Trp Cys Trp Ser Tyr
305                 310                 315

<210>  44
<211>  909
<212>  DNA
<213>  Populus tremuloides

<400>  44
atggcgggtg ataaagactg tggcagttct aaaatgacca tttttcttcg aaacggcagg      60
ctccctcctt gtgaatctct ctgtattctc acctctttta gcactcttca tggtgcaaaa     120
tctatggtta attttaggaa tgatggagga gacactgtag acatgtcttt tttccaacca     180
catgtcaaag aagaaagtag cgatgaggat tatgatgcgc accttaagcc atctgaaaag     240
aaaaggcggc ttacagctgc tcaagtccag tttcttgaga agagctttga ggcggagaat     300
aagcttgaac cagagaggaa gatgcagctt gctaaagaac tcggcttgca gcctcgccag     360
gttgcaatat ggtttcaaaa ccgtagagct cggttcaaga acaagcagct ggaaagggac     420
tacgactcct tgagaatcag ctttgacaaa ctcaaggctg attatgacaa actcctcctc     480
gagaagcaga atttgaaaaa cgagcttctt tcactgaaag aaaaattgct tagcagagag     540
gaaagtatgg aaagttcaga accatttgat gtcatccatt caccggatgc agaacttgag     600
cctattcctg atacagtgtc tgaaaatgtt tccgccattg tgccaatggt gacacccaaa     660
caagaagaaa gttcagctaa aaatgatgtt ttcaactcag acagcccacg ttcatttttg     720
gagccccgtg attgttatcg tgttttcgag tcagaccaac cagatttttc ccaagttgaa     780
gaagataatc tcaccaggag ctttctaccc cctccgtact ttccaaaact ctaccgagag     840
ccacctgcaa gttcacgtaa ttttgaattc tcagcggaag atcagccctt ttggtcctgg     900
atttactga                                                             909

<210>  45
<211>  302
<212>  PRT
<213>  Populus tremuloides

<400>  45
Met Ala Gly Asp Lys Asp Cys Gly Ser Ser Lys Met Thr Ile Phe Leu
1               5                   10                  15
Arg Asn Gly Arg Leu Pro Pro Cys Glu Ser Leu Cys Ile Leu Thr Ser
            20                  25                  30
Phe Ser Thr Leu His Gly Ala Lys Ser Met Val Asn Phe Arg Asn Asp
        35                  40                  45
Gly Gly Asp Thr Val Asp Met Ser Phe Phe Gln Pro His Val Lys Glu
    50                  55                  60
Glu Ser Ser Asp Glu Asp Tyr Asp Ala His Leu Lys Pro Ser Glu Lys
65                  70                  75                  80
Lys Arg Arg Leu Thr Ala Ala Gln Val Gln Phe Leu Glu Lys Ser Phe
                85                  90                  95
Glu Ala Glu Asn Lys Leu Glu Pro Glu Arg Lys Met Gln Leu Ala Lys

                100                 105                 110
Glu Leu Gly Leu Gln Pro Arg Gln Val Ala Ile Trp Phe Gln Asn Arg
        115                 120                 125
Arg Ala Arg Phe Lys Asn Lys Gln Leu Glu Arg Asp Tyr Asp Ser Leu
    130                 135                 140
Arg Ile Ser Phe Asp Lys Leu Lys Ala Asp Tyr Asp Lys Leu Leu Leu
145                 150                 155                 160
Glu Lys Gln Asn Leu Lys Asn Glu Leu Leu Ser Leu Lys Glu Lys Leu
                165                 170                 175
Leu Ser Arg Glu Glu Ser Met Glu Ser Ser Glu Pro Phe Asp Val Ile
            180                 185                 190
His Ser Pro Asp Ala Glu Leu Glu Pro Ile Pro Asp Thr Val Ser Glu
        195                 200                 205
Asn Val Ser Ala Ile Val Pro Met Val Thr Pro Lys Gln Glu Glu Ser
    210                 215                 220
Ser Ala Lys Asn Asp Val Phe Asn Ser Asp Ser Pro Arg Ser Phe Leu
225                 230                 235                 240
Glu Pro Arg Asp Cys Tyr Arg Val Phe Glu Ser Asp Gln Pro Asp Phe
                245                 250                 255
Ser Gln Val Glu Glu Asp Asn Leu Thr Arg Ser Phe Leu Pro Pro Pro
            260                 265                 270
Tyr Phe Pro Lys Leu Tyr Arg Glu Pro Pro Ala Ser Ser Arg Asn Phe
        275                 280                 285
Glu Phe Ser Ala Glu Asp Gln Pro Phe Trp Ser Trp Ile Tyr
    290                 295                 300

<210>  46
<211>  975
<212>  DNA
<213>  Medicago truncatula

<400>  46
atggcaggtg gcaagctttt tggtggttct aatatgtcac ttttgcttca aaatgaaaga      60
ctcccttgta cttctgaagt ccttgaatct ctttgggttc acacccctgc ttcttttcaa     120
ggttcaaatt cagtggttaa ttttgagaat ggtggtggta gcaacagagt ggtaacagat     180
agacccttct ttcaacaact tgagaaagaa gagaattgtg gtgatgaaga ttatgaagca     240
tgctaccatc aacaaggaaa gaaaaggagg ctttcaagtg aacaagttca atttcttgaa     300
aagagttttg aggtagaaaa caagcttgaa cctgatagga aagttcaact tgcaaaagag     360
cttggtttgc aaccaagaca agttgctata tggtttcaaa acagaagggc aaggttcaaa     420
actaaacagc ttgaaaaaga ttatggcaca ttgaaagcta gctttgatag tctcaaagat     480
gattatgata atcttcttca agagaatgac aagttaaaag aagaggtgaa ttctctcaag     540
aacaaattga tcccaagaga taaagaaaaa gtgaattcag aagacaaatc atcaccagaa     600
gcaatcaatt cacctcataa caacatagat ccaatggata taatttcaat tacaaattca     660
gaaaatgggt ccaaaatgtc actccctaat atggtactaa aatgtaagca agaagatgcc     720
aattcagcta aagtgatgtt gcttgattct gatagcccac attgcaatga tgggaacaat     780
ctttcttctt tcatagagcc tacagattca gatttctcac aagatgaaga ggataatgat     840
aacttgagtc ataatctttt gactcttcct tgcttaccaa aagttgaaga tgtttgctat     900
gatgacccac atgaaaattc ttgtaatttt gggttccctg ttgaagatca aaccttttgt     960
ttctggcctt attga                                                      975

<210>  47
<211>  324
<212>  PRT
<213>  Medicago truncatula

<400>  47
Met Ala Gly Gly Lys Leu Phe Gly Gly Ser Asn Met Ser Leu Leu Leu
1               5                   10                  15
Gln Asn Glu Arg Leu Pro Cys Thr Ser Glu Val Leu Glu Ser Leu Trp

                20                  25                  30
Val His Thr Pro Ala Ser Phe Gln Gly Ser Asn Ser Val Val Asn Phe
        35                  40                  45
Glu Asn Gly Gly Gly Ser Asn Arg Val Val Thr Asp Arg Pro Phe Phe
    50                  55                  60
Gln Gln Leu Glu Lys Glu Glu Asn Cys Gly Asp Glu Asp Tyr Glu Ala
65                  70                  75                  80
Cys Tyr His Gln Gln Gly Lys Lys Arg Arg Leu Ser Ser Glu Gln Val
                85                  90                  95
Gln Phe Leu Glu Lys Ser Phe Glu Val Glu Asn Lys Leu Glu Pro Asp
            100                 105                 110
Arg Lys Val Gln Leu Ala Lys Glu Leu Gly Leu Gln Pro Arg Gln Val
        115                 120                 125
Ala Ile Trp Phe Gln Asn Arg Arg Ala Arg Phe Lys Thr Lys Gln Leu
    130                 135                 140
Glu Lys Asp Tyr Gly Thr Leu Lys Ala Ser Phe Asp Ser Leu Lys Asp
145                 150                 155                 160
Asp Tyr Asp Asn Leu Leu Gln Glu Asn Asp Lys Leu Lys Glu Glu Val
                165                 170                 175
Asn Ser Leu Lys Asn Lys Leu Ile Pro Arg Asp Lys Glu Lys Val Asn
            180                 185                 190
Ser Glu Asp Lys Ser Ser Pro Glu Ala Ile Asn Ser Pro His Asn Asn
        195                 200                 205
Ile Asp Pro Met Asp Ile Ile Ser Ile Thr Asn Ser Glu Asn Gly Ser
    210                 215                 220
Lys Met Ser Leu Pro Asn Met Val Leu Lys Cys Lys Gln Glu Asp Ala
225                 230                 235                 240
Asn Ser Ala Lys Ser Asp Val Leu Asp Ser Asp Ser Pro His Cys Asn
                245                 250                 255
Asp Gly Asn Asn Leu Ser Ser Phe Ile Glu Pro Thr Asp Ser Asp Phe
            260                 265                 270
Ser Gln Asp Glu Glu Asp Asn Asp Asn Leu Ser His Asn Leu Leu Thr
        275                 280                 285
Leu Pro Cys Leu Pro Lys Val Glu Asp Val Cys Tyr Asp Asp Pro His
    290                 295                 300
Glu Asn Ser Cys Asn Phe Gly Phe Pro Val Glu Asp Gln Thr Phe Cys
305                 310                 315                 320
Phe Trp Pro Tyr

<210>  48
<211>  984
<212>  DNA
<213>  Phaseolus vulgaris

<400>  48
atggcgggtg gcaagcttca tcctggttca aacatgtcac ttctcctcca aaacgacagg      60
ctcccttgct cctctgaagt ccttgagtct ctttgggctc acacctctaa cgctgcttcc     120
ttccaaggtt caaaatctat ggttgatttt gagaatgtta gtgggggcag ggtgacggat     180
aggccctttt ttcaagcgtt ggagaaggaa gataactgtg atgatgatta tgagggttgc     240
ttccatcaac cgggtaagaa aaggaggctc acaagcgaac aagttcagtt ccttgaaagg     300
aactttgagg tcgagaacaa gcttgaacct gaaaggaagg tccaacttgc aaaggagctt     360
ggcttgcagc caaggcaagt ggctatatgg ttccaaaacc gaagggcaag gttcaagacc     420
aagcagctag aaaaagatta tggcacattg aaagctagct atgacagact caaaggtgac     480
tatgaaagtc ttcttcaaga gaatgacaag ttaaaagcag aggtgaattc tctggagagc     540
aaattgattc ttagagataa agagaaggag aattcggacg acaagtcatc tcctgatgct     600
gtcaattcac cccacaaaga gcctatggat ttaatttcaa attcaacatc tgaaaatggg     660
accaaagtgt cactccctat tatggtaaca tgcaagcaag aagatgccaa ttcagccaaa     720
agtgatgtgc ttgattcgga cagcccacat tgcactgatg ggaaccatcc ctcttcattc     780

gtggagcctg ctgattcctc ccatgctttt gaaccagacc actccgactt ctcccaagat 840
gaagaggata atcttagtga aagccttttg accctccctt gcttaccaaa ggttgaagaa 900
gcctgctatg atgaccctcc tgaaaaccct tgtaattttg gcttccatgt cgaggatcaa 960
accttctgtt tctggcccta ttga 984

<210> 49
<211> 327
<212> PRT
<213> Phaseolus vulgaris

<400> 49
Met Ala Gly Gly Lys Leu His Pro Gly Ser Asn Met Ser Leu Leu Leu
1               5                   10                  15
Gln Asn Asp Arg Leu Pro Cys Ser Ser Glu Val Leu Glu Ser Leu Trp
            20                  25                  30
Ala His Thr Ser Asn Ala Ala Ser Phe Gln Gly Ser Lys Ser Met Val
        35                  40                  45
Asp Phe Glu Asn Val Ser Gly Gly Arg Val Thr Asp Arg Pro Phe Phe
    50                  55                  60
Gln Ala Leu Glu Lys Glu Asp Asn Cys Asp Asp Asp Tyr Glu Gly Cys
65                  70                  75                  80
Phe His Gln Pro Gly Lys Lys Arg Arg Leu Thr Ser Glu Gln Val Gln
                85                  90                  95
Phe Leu Glu Arg Asn Phe Glu Val Glu Asn Lys Leu Glu Pro Glu Arg
            100                 105                 110
Lys Val Gln Leu Ala Lys Glu Leu Gly Leu Gln Pro Arg Gln Val Ala
        115                 120                 125
Ile Trp Phe Gln Asn Arg Arg Ala Arg Phe Lys Thr Lys Gln Leu Glu
    130                 135                 140
Lys Asp Tyr Gly Thr Leu Lys Ala Ser Tyr Asp Arg Leu Lys Gly Asp
145                 150                 155                 160
Tyr Glu Ser Leu Leu Gln Glu Asn Asp Lys Leu Lys Ala Glu Val Asn
                165                 170                 175
Ser Leu Glu Ser Lys Leu Ile Leu Arg Asp Lys Glu Lys Glu Asn Ser
            180                 185                 190
Asp Asp Lys Ser Ser Pro Asp Ala Val Asn Ser Pro His Lys Glu Pro
        195                 200                 205
Met Asp Leu Ile Ser Asn Ser Thr Ser Glu Asn Gly Thr Lys Val Ser
    210                 215                 220
Leu Pro Ile Met Val Thr Cys Lys Gln Glu Asp Ala Asn Ser Ala Lys
225                 230                 235                 240
Ser Asp Val Leu Asp Ser Asp Ser Pro His Cys Thr Asp Gly Asn His
                245                 250                 255
Pro Ser Ser Phe Val Glu Pro Ala Asp Ser Ser His Ala Phe Glu Pro
            260                 265                 270
Asp His Ser Asp Phe Ser Gln Asp Glu Glu Asp Asn Leu Ser Glu Ser
        275                 280                 285
Leu Leu Thr Leu Pro Cys Leu Pro Lys Val Glu Glu Ala Cys Tyr Asp
    290                 295                 300
Asp Pro Pro Glu Asn Pro Cys Asn Phe Gly Phe His Val Glu Asp Gln
305                 310                 315                 320
Thr Phe Cys Phe Trp Pro Tyr
                325

<210> 50
<211> 957
<212> DNA
<213> Lotus corniculatus

<400>  50
atggcgggag ggagggtctt tagcggcggt tctgctgctc ctgcaaatgt ttccgatacc      60
agtcttttgc ttcagaatca acctcctgat tcttctctct tcctctctac ctctgcttct     120
tttctcggtt caagatccat ggtgagcttc gcagataata aattagggca aacgcggtcg     180
ttcttctccg cgtttgacct cgatgagaac ggcgatgagg tcatggacga gtactttcac     240
caatcggaga agaagcgccg tctctctgtt gaccaagttc agtttctgga gaagagcttc     300
gaggtggata acaagctcga acctgacagg aaaaccaaga ttgccaagga ccttggtttg     360
cagccacgcc aagtcgcaat ctggttccag aaccgccgtg cacggtggaa gacgaaacag     420
cttgagaagg attatgattc tctgcatagt agctttgaga gtctcaaatc caactatgat     480
aatcttctca aggagaaaga catgttaaaa gctgaggtgg caagtctcac tgagaaggtg     540
cttgcaagag agaatttgaa acaagttgaa agtgaaacaa agggattggt tgaaccaccc     600
caaaggcctt tacttgattc agtttcagag ggtgaagaat ctaaagtctc tgttggggct     660
tgtaaacatg aggatatcag ttcagccagg agtgagagtt tggattctga tagcccacgt     720
tacagggatg gatatggagt taactcagca gtgctagaga catgtgattc ttcttatgtg     780
gttgaacctg atcaatcgga tatgtcacag gatgaggaag acaacctgac caagaccctg     840
ttgcctccat acatgttttc caaacttgga gatatggatt actccgaccc gcctgaaagt     900
tcatgtaatt tcggatttcc ggaggaagat catgcccttt ggtcatggtc ttactga        957

<210>  51
<211>  318
<212>  PRT
<213>  Lotus corniculatus

<400>  51
Met Ala Gly Gly Arg Val Phe Ser Gly Gly Ser Ala Ala Pro Ala Asn
1               5                   10                  15
Val Ser Asp Thr Ser Leu Leu Leu Gln Asn Gln Pro Pro Asp Ser Ser
            20                  25                  30
Leu Phe Leu Ser Thr Ser Ala Ser Phe Leu Gly Ser Arg Ser Met Val
        35                  40                  45
Ser Phe Ala Asp Asn Lys Leu Gly Gln Thr Arg Ser Phe Phe Ser Ala
    50                  55                  60
Phe Asp Leu Asp Glu Asn Gly Asp Glu Val Met Asp Glu Tyr Phe His
65                  70                  75                  80
Gln Ser Glu Lys Lys Arg Arg Leu Ser Val Asp Gln Val Gln Phe Leu
                85                  90                  95
Glu Lys Ser Phe Glu Val Asp Asn Lys Leu Glu Pro Asp Arg Lys Thr
            100                 105                 110
Lys Ile Ala Lys Asp Leu Gly Leu Gln Pro Arg Gln Val Ala Ile Trp
        115                 120                 125
Phe Gln Asn Arg Arg Ala Arg Trp Lys Thr Lys Gln Leu Glu Lys Asp
    130                 135                 140
Tyr Asp Ser Leu His Ser Ser Phe Glu Ser Leu Lys Ser Asn Tyr Asp
145                 150                 155                 160
Asn Leu Leu Lys Glu Lys Asp Met Leu Lys Ala Glu Val Ala Ser Leu
                165                 170                 175
Thr Glu Lys Val Leu Ala Arg Glu Asn Leu Lys Gln Val Glu Ser Glu
            180                 185                 190
Thr Lys Gly Leu Val Glu Pro Pro Gln Arg Pro Leu Leu Asp Ser Val
        195                 200                 205
Ser Glu Gly Glu Glu Ser Lys Val Ser Val Gly Ala Cys Lys His Glu
    210                 215                 220
Asp Ile Ser Ser Ala Arg Ser Glu Ser Leu Asp Ser Asp Ser Pro Arg
225                 230                 235                 240
Tyr Arg Asp Gly Tyr Gly Val Asn Ser Ala Val Leu Glu Thr Cys Asp
                245                 250                 255
Ser Ser Tyr Val Val Glu Pro Asp Gln Ser Asp Met Ser Gln Asp Glu
            260                 265                 270
Glu Asp Asn Leu Thr Lys Thr Leu Leu Pro Pro Tyr Met Phe Ser Lys

        275                 280                 285
Leu Gly Asp Met Asp Tyr Ser Asp Pro Pro Glu Ser Ser Cys Asn Phe
    290                 295                 300
Gly Phe Pro Glu Glu Asp His Ala Leu Trp Ser Trp Ser Tyr
305                 310                 315

## Description

The present invention relates generally to the field of molecular biology and concerns a method for improving plant growth characteristics relative to corresponding wild type plants or other control plants. More specifically, the present invention concerns a method for improving plant growth characteristics comprising modulating expression in a plant of a nucleic acid encoding a class I homeodomain leucine zipper (HDZip) hox5 polypeptide or a homologue thereof. The present invention also concerns plants having modulated expression of a nucleic acid encoding a class I HDZip hox5 polypeptide or a homologue thereof, which plants have improved growth characteristics relative to corresponding wild type or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production leaf scenescence and more. Root development, nutrient uptake and stress tolerance and early vigour may also be important factors in determining yield. Optimizing one of the abovementioned factors may therefore contribute to increasing crop yield.

Seed yield is a particularly important trait, since the seeds of many plants are important for human and animal nutrition. Crops such as, corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another trait of particular economic interest is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

The ability to increase plant yield would have many applications in areas such as agriculture, including in the production of ornamental plants, arboriculture, horticulture and forestry. Increasing yield may also find use in the production of algae for use in bioreactors (for the biotechnological production of substances such as pharmaceuticals, antibodies or vaccines, or for the bioconversion of organic waste) and other such areas.

### BACKGROUND

Homeodomain leucine zipper (HDZip) proteins constitute a family of transcription factors characterized by the presence of a DNA-binding domain (HD) and an adjacent leucine zipper (Zip) motif. The homeodomain usually consists of 60 conserved amino acid residues that form a helix1-loop-helix2-turn-helix3 that binds DNA. This DNA binding site is usually pseudopalindromic. The leucine zipper, adjacent to the C-terminal end of the homeodomain, consists of several heptad repeats (at least four) in which usually a leucine (occasionally a valine or an isoleucine) appears every seventh amino acid. The leucine zipper is important for protein dimerisation. This dimerisation is a prerequisite for DNA binding (Sessa et al. (1993) EMBO J 12(9): 3507-3517), and may proceed between two identical HDZip proteins (homodimer) or between two different HDZip proteins (heterodimer).

Homeodomain genes are present in all eucaryotes, and constitute a gene family of at least 89 members in Arabidopsis thaliana. The leucine zipper is also found by itself in eukaryotes other than plants. However, the presence of both a homeodomain and a leucine zipper is plant-specific (found in at least 47 out of the 89 proteins in Arabidopsis), and has been encountered in moss in addition to vascular plants (Sakakibara et al. (2001) Mol Biol Evol 18(4): 491-502). The leucine zipper is then located at the C-terminal end of the homeodomain, these two features overlapping by three amino acids.

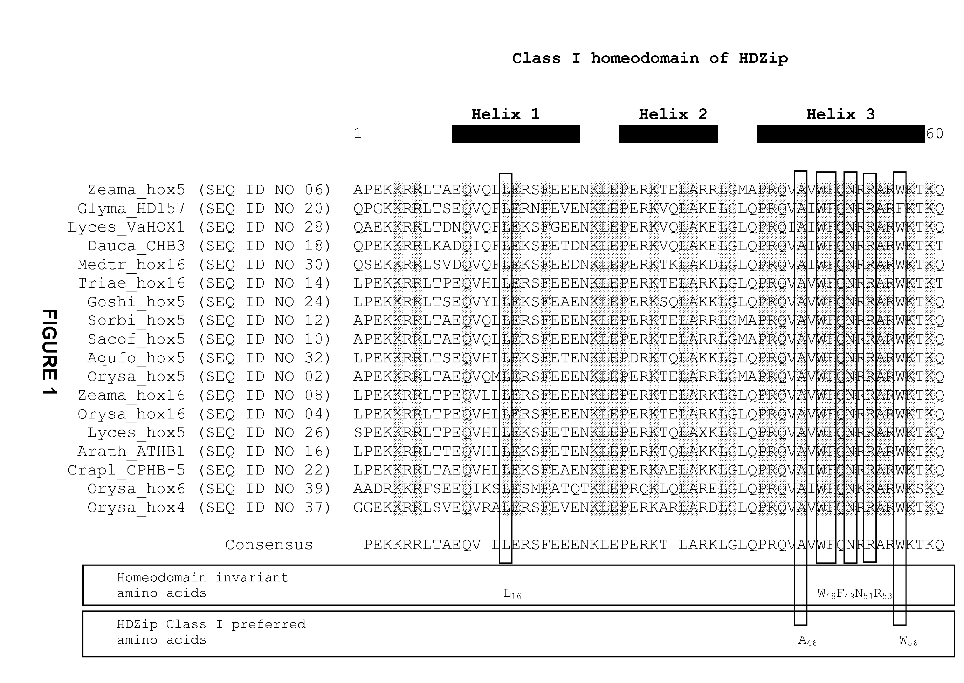

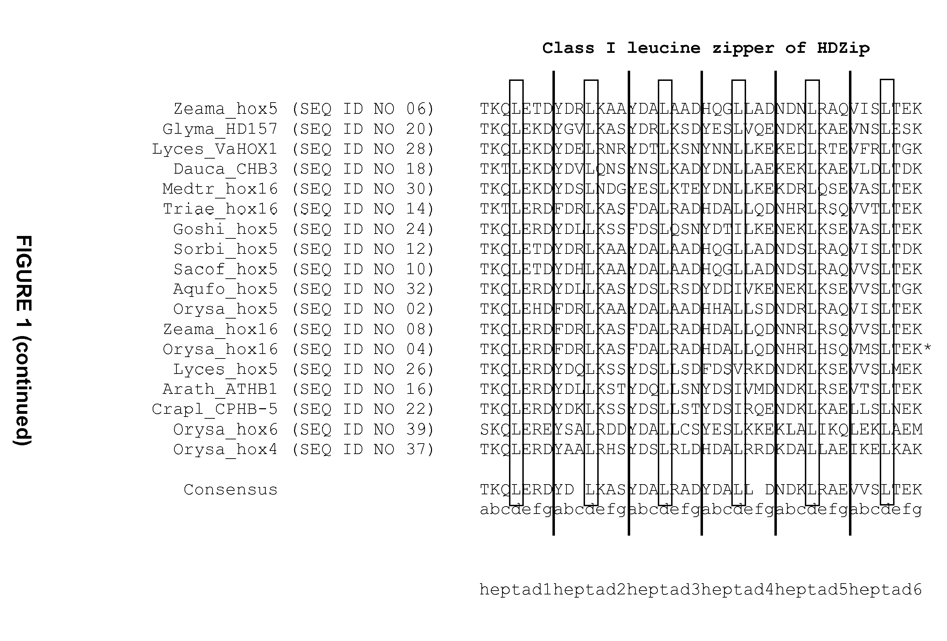

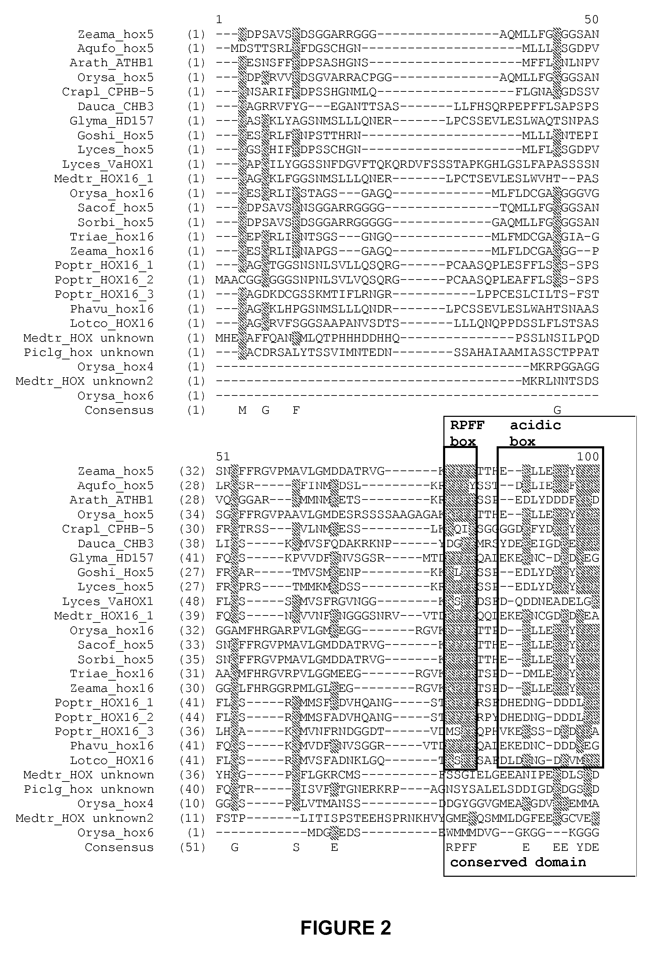

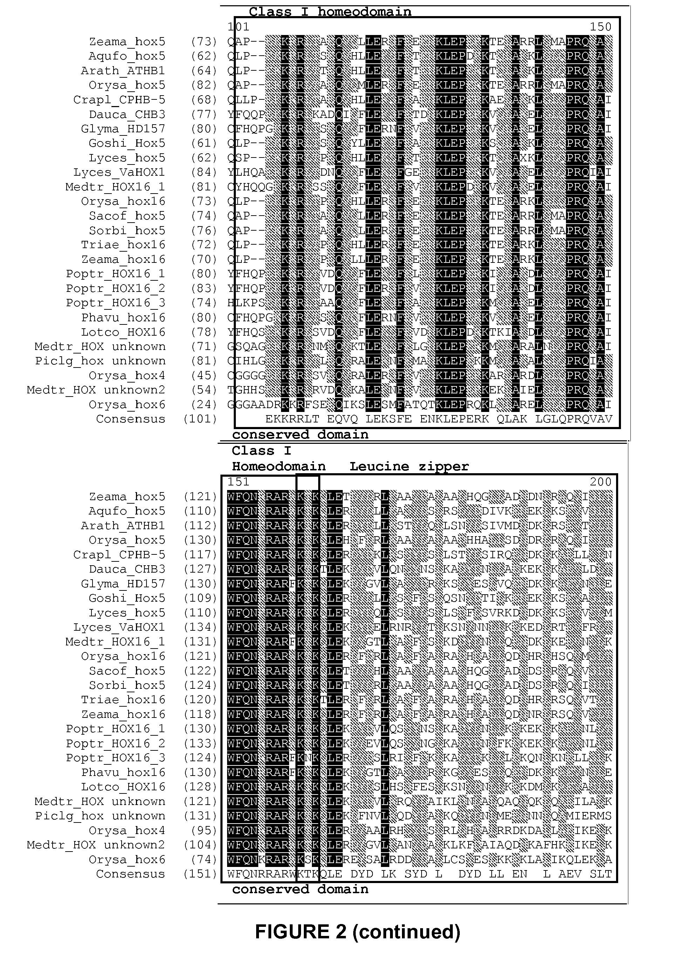

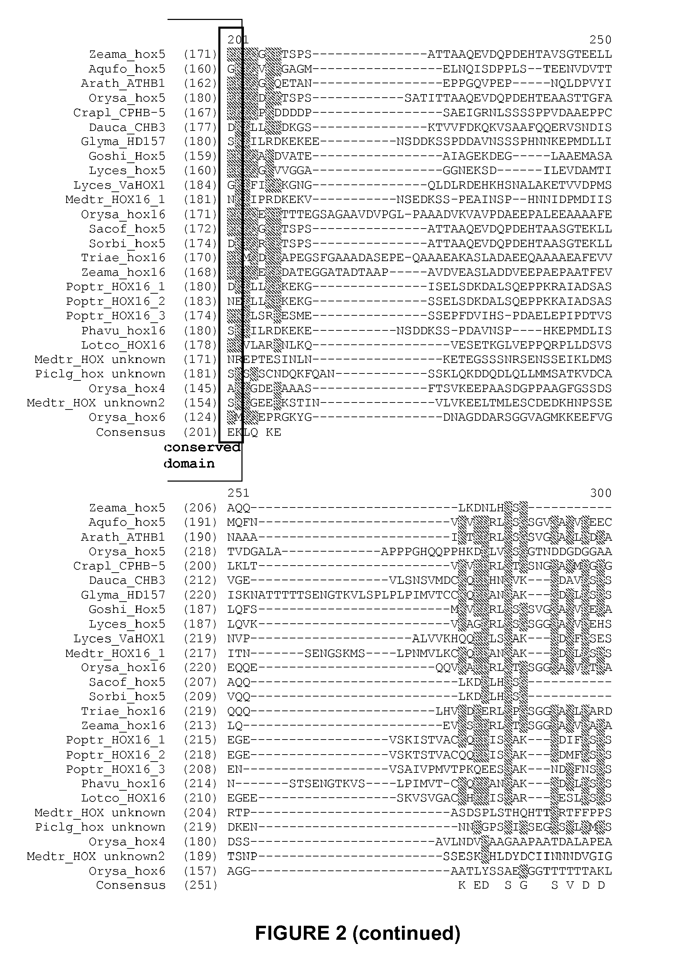

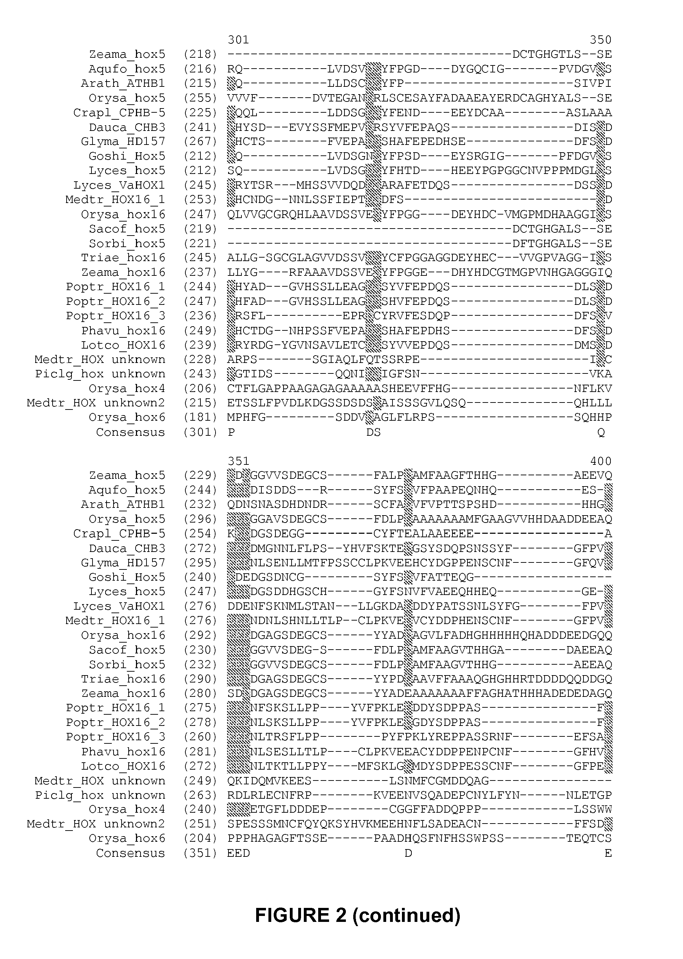

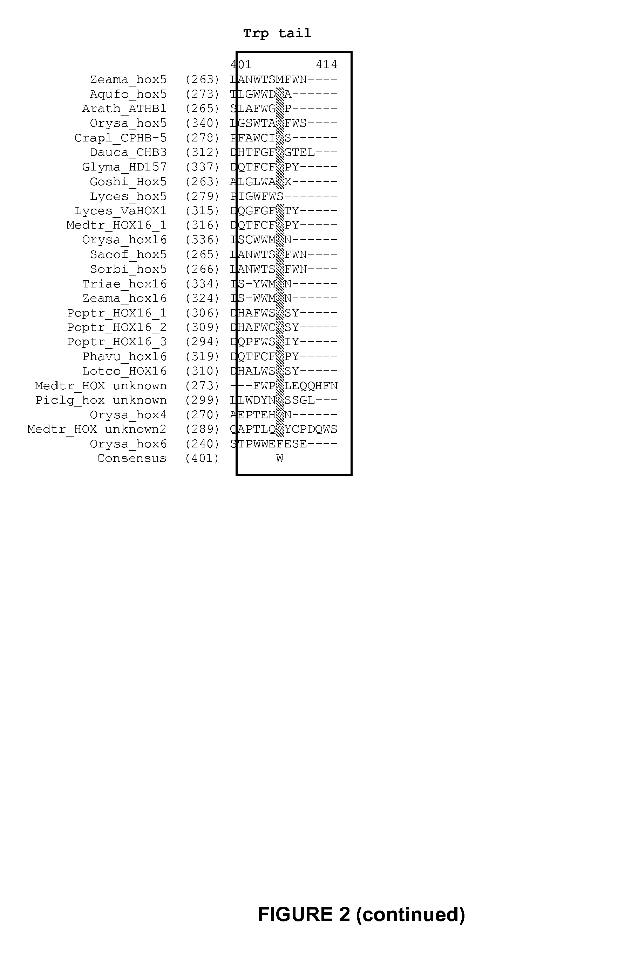

The Arabidopsis HDZip genes have been classified into four different classes, HDZip I to IV, based on sequence similarity criteria (Sessa et al. (1994) In Plant Molec Biol, pp412-426). Like the HD-Zip proteins from the three other classes, class I HDZip proteins are quite divergent in their primary amino structure outside of the homeodomain and the leucine zipper. Within both the homeodomain and the leucine zipper, class I HDZip proteins are further characterized by two specific features:
1) in the homeodomain, in addition to the invariant amino acids Leu16Trp48Phe49Asn51Arg53, position 46 is occupied by an Ala (A) and position 56 by a Try (W) (or occasionally by a Phe (F)) (Sessa et al. (1997) J Mol Biol 274(3):303-309; see Figure 1), referred to as a class I homeodomain, and
2) the leucine zipper comprises six heptads, except for the fern Ceratopteris richardii which presents seven heptads (within each heptad, positions are named a, b, c, d, e, f and g, the conserved leucine being at position d; Sakakibara et al. (2001) Mol Biol Evol18(4): 491-502; see Figure 2). HDZip 11, III and IV present a leucine zipper with five heptads only.

Concerning their DNA binding properties, class I HDZip proteins preferably bind to 5 bp half-sites that overlap at a central position, CAA(A/T)ATTG (Sessa et al. (1993) EMBO J 12(9): 3507-3517).

Different HDZip proteins have been shown to either activate or repress transcription. In Arabidopsis, the class I HDZip ATHB1, -5, -6, and -16 were shown to act as transcriptional activators in transient expression assays on Arabidopsis leaves using a reporter gene (luciferase; Henriksson et al. (2005) Plant Phys 139: 509-518). Two rice class I HDZip proteins, Oshox4 and Oshox5, acted as activators in transient expression assays on rice cell suspension cultures using another reporter gene (glucuronidase; Meijer et al. (2000) Mol Gen Genet 263:12-21). In contrast, two rice class II HDZip proteins, Oshox1 and Oshox3, acted as transcriptional repressors in the same experiments (Meijer et al. (1997) Plant J 11: 263-276; Meijer et al. (2000) supra).

Several class I HDZip proteins have been shown to be involved in light response and in abscisic acid (ABA)/ water deficit related response (Hjellström et al. (2003) Plant Cell Environ 26: 1127-1136). Transgenic Arabidopsis overexpressing class I HDZip ATHB1, - 3, -13, -20, and -23 suggest that these genes are involved in the regulation of cotyledon and leaf development (Aoyama et al. (1995) Plant Cell 7: 1773-1785; Hanson (2000) In Comprehensive summaries of Uppsala Dissertations from the Faculty of Science and Technology, Uppsala). The ATHB3, -13, -20, and -23 genes are similar and form a distinct subclass within the class I HDZip. Since these genes cause similar alterations in cotyledon shape when expressed constitutively, they are referred to as the pointed cotyledon (POC) HDZip genes. Hanson concludes that class I HDZip proteins that are closely related phylogenetically are also functionally related, in most cases.

It has now been found that modulating expression in a plant of a nucleic acid encoding a class I HDZip hox5 polypeptide or a homologue thereof gives plants having improved growth characteristics relative to corresponding wild type plants or other control plants.

The present invention therefore provides a method for improving plant growth characteristics comprising modulating expression in a plant of a nucleic acid encoding a class I HDZip hox5 polypeptide or a homologue thereof.

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. A "control plant" as used herein refers not only to whole plants, but also to plant parts, including seeds and seed parts.

Advantageously, performance of the methods according to the present invention results in plants having improved growth characteristics, particularly one or more of increased yield, improved growth, improved biomass, improved architecture, improved cell division and improved tolerance to abiotic stress relative to corresponding wild type or other control plants.

The term "increased yield" as defined herein is taken to mean an increase in any one or more of the following, each relative to corresponding wild type or other control plants: (i) increased biomass (weight) of one or more parts of a plant, particularly aboveground (harvestable) parts, or increased root biomass, increased root volume, increased root number, increased root diameter or increased root length (of thick or thin roots), or increased biomass of any other harvestable part; (ii) increased total seed yield, which includes an increase in seed biomass (seed weight) and which may be an increase in the seed weight per plant or on an individual seed basis and/or per hectare or acre; (iii) increased number of flowers (florets) per panicle, which is expressed as a ratio of number of filled seeds over number of primary panicles; (iv) increased seed fill rate (expressed in percentage terms as the proportion of the number of filled seeds over the number of florets); (v) increased number of (filled) seeds; (vi) increased seed size, which may also influence the composition of seeds; (vii) increased seed volume, which may also influence the composition of seeds (including oil, protein and carbohydrate total content and composition); (viii) increased (individual or average) seed area; (ix) increased (individual or average) seed length; (x) increased (individual or average) seed width; (xi) increased (individual or average) seed perimeter; (xii) increased harvest index (HI), which is expressed as a ratio of the yield of harvestable parts, such as seeds, over the total biomass; and (xiii) increased thousand kernel weight (TKW), which is extrapolated from the number of filled seeds counted and their total weight. An increased TKW may result from an increased seed size and/or seed weight. An increased TKW may result from an increase in embryo size and/or endosperm size.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of rice plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the invention gives plants having an increased growth rate. Therefore, according to the present invention, there is provided a method for increasing the growth rate of plants which method comprises modulating expression in a plant of a nucleic acid encoding a class I HDZip hox5 polypeptide or a homologue thereof.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

Performance of the methods according to the present invention results in plants having increased tolerance to abiotic stress. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. For example, drought and/or salinisation are manifested primarily as osmotic stress, resulting in the disruption of homeostasis and ion distribution in the cell. Oxidative stress, which frequently accompanies high or low temperature, salinity or drought stress may cause denaturation of functional and structural proteins. As a consequence, these diverse environmental stresses often activate similar cell signaling pathways and cellular responses, such as the production of stress proteins, up-regulation of anti-oxidants, accumulation of compatible solutes and growth arrest.

Since diverse environmental stresses activate similar pathways, the exemplification of the present invention with drought stress (insofar as the invention concerns the use of class I HDZip hox5 polypeptides and their encoding nucleic acids) should not be seen as a limitation to drought stress, but more as a screen to indicate the involvement of class I HDZip hox5 polypeptides or a homologues thereof in abiotic stresses in general. Furthermore, the methods of the present invention may be performed under non-stress conditions or under conditions of mild drought to give plants having improved growth characteristics (particularly increased yield) relative to corresponding wild type or other control plants. The term "non-stress" conditions as used herein are preferably those environmental conditions that do not significantly go beyond the everyday climatic and other abiotic conditions that plants may encounter most preferably those conditions that allow optimal growth of plants. Persons skilled in the art are aware of normal soil conditions and climatic conditions for a given location.

A particularly high degree of "cross talk" is reported between drought stress and high-salinity stress (Rabbani et al. (2003) Plant Physiol 133: 1755-1767). Therefore, it would be apparent that a class I HDZip hox5 polypeptide or a homologue thereof would, along with its usefulness in conferring drought-tolerance in plants, also find use in protecting the plant against various other abiotic stresses. Furthermore, Rabbani et al. (2003, Plant Physiol 133: 1755-1767) report that similar molecular mechanisms of stress tolerance and responses exist between dicots and monocots. The methods of the invention are therefore advantageously applicable to any plant.

The term "abiotic stress" as defined herein is taken to mean any one or more of: water stress (due to drought or excess water), anaerobic stress, salt stress, temperature stress (due to hot, cold or freezing temperatures), chemical toxicity stress and oxidative stress. According to one aspect of the invention, the abiotic stress is an osmotic stress, selected from water stress, salt stress, oxidative stress and ionic stress. Preferably, the water stress is drought stress. The term salt stress is not restricted to common salt (NaCI), but may be any one or more of: NaCl, KCI, LiCl, MgCl₂, CaCl₂, amongst others.

Increased tolerance to abiotic stress is manifested by increased plant yield in abiotic stress conditions. Particularly insofar as the invention concerns the use of class I HDZip hox5 polypeptides and their encoding nucleic acids, such increased yield may include one or more of the following: increased number of filled seeds, increased total seed yield, increased number of flowers per panicle, increased seed fill rate, increased Hl, increased TKW, increased root length or increased root diameter, each relative to corresponding wild type plants.

Performance of the methods of the invention gives plants having increased tolerance to abiotic stress. Performance of the methods of the invention gives plants grown under non-stress conditions or under mild drought conditions improved growth characteristics (particularly increased yield) relative to corresponding wild type plants or other control plants grown under comparable conditions.

According to the present invention, there is provided a method for increasing abiotic stress tolerance in plants which method comprises modulating expression in a plant of a nucleic acid encoding a class I HDZip hox5 polypeptide or a homologue thereof. According to one aspect of the invention, the abiotic stress is an osmotic stress, selected from one or more of the following: water stress, salt stress, oxidative stress and ionic stress. Preferably, the water stress is drought stress.

Particularly insofar as the invention concerns the use of class I HDZip hox5 polypeptides and their encoding nucleic acids, performance of the methods of the invention gives an increased greenness index relative to corresponding wild type plants. The greenness index as defined herein is the proportion (expressed as %) of yellow pixels in plant images recorded by a digital camera. An increased greenness index may indicate reduced or delayed senescence which in turn allows prolongation of the photosynthetic activity of a plant, which in turn leads to various beneficial effects well known in the art.

The invention therefore provides a method for increasing greenness index in plants which method comprises modulating expression in a plant of a nucleic acid encoding a class I HDZip hox5 polypeptide or a homologue thereof. Preferably, the greenness index is increased in abiotic stress conditions, more preferably in water stress conditions, further preferably in drought stress conditions.

Preferably, where the method of the invention comprises modulating expression in a plant of a nucleic acid encoding a class I HDZip hox5 polypeptide or a homologue thereof, the increased yield includes one or more of the following: increased number of filled seeds, increased total seed yield, increased number of flowers per panicle, increased seed fill rate, increased Hl, increased TKW, increased root length or increased root diameter, each relative to corresponding wild type or other control plants.

According to a preferred feature of the present invention, there is provided a method for increasing plant yield relative to corresponding wild type or other control plants, which method comprises modulating expression in a plant of a nucleic acid encoding a class I HDZip hox5 polypeptide or a homologue thereof.

The methods of the invention are therefore advantageously applicable to any plant.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acacia spp.,Acer spp., Actinidia spp., Aesculus spp., Agathis australis, Albizia amara, Alsophila tricolor, Andropogon spp., Arachis spp, Areca catechu, Astelia fragrans, Astragalus cicer, Baikiaea plurijuga, Betula spp., Brassica spp., Bruguiera gymnorrhiza, Burkea africana, Butea frondosa, Cadaba farinosa, Calliandra spp, Camellia sinensis, Canna indica, Capsicum spp., Cassia spp., Centroema pubescens, Chaenomeles spp., Cinnamomum cassia, Coffea arabica, Colophospermum mopane, Coronillia varia, Cotoneaster serotina, Crataegus spp., Cucumis spp., Cupressus spp., Cyathea dealbata, Cydonia oblonga, Cryptomeria japonica, Cymbopogon spp., Cynthea dealbata, Cydonia oblonga, Dalbergia monetaria, Davallia divaricata, Desmodium spp., Dicksonia squarosa, Diheteropogon amplectens, Dioclea spp, Dolichos spp., Dorycnium rectum, Echinochloa pyramidalis, Ehrartia spp., Eleusine coracana, Eragrestis spp., Erythrina spp., Eucalyptus spp., Euclea schimperi, Eulalia villosa, Fagopyrum spp., Feijoa sellowiana, Fragaria spp., Flemingia spp, Freycinetia banksii, Geranium thunbergii, Ginkgo biloba, Glycine javanica, Gliricidia spp, Gossypium hirsutum, Grevillea spp., Guibourtia coleosperma, Hedysarum spp., Hemarthia altissima, Heteropogon contortus, Hordeum vulgare, Hyparrhenia rufa, Hypericum erectum, Hyperthelia dissoluta, Indigo incarnata, Iris spp., Leptarrhena pyrolifolia, Lespediza spp., Lettuca spp., Leucaena leucocephala, Loudetia simplex, Lotonus bainesii, Lotus spp., Macrotyloma axillare, Malus spp., Manihot esculenta, Medicago sativa, Metasequoia glyptostroboides, Musa sapientum, Nicotianum spp., Onobrychis spp., Ornithopus spp., Oryza spp., Peltophorum africanum, Pennisetum spp., Persea gratissima, Petunia spp., Phaseolus spp., Phoenix canariensis, Phormium cookianum, Photinia spp., Picea glauca, Pinus spp., Pisum sativum, Podocarpus totara, Pogonarthria fleckii, Pogonarthria squarrosa, Populus spp., Prosopis cineraria, Pseudotsuga menziesii, Pterolobium stellatum, Pyrus communis, Quercus spp., Rhaphiolepsis umbellata, Rhopalostylis sapida, Rhus natalensis, Ribes grossularia, Ribes spp., Robinia pseudoacacia, Rosa spp., Rubus spp., Salix spp., Schyzachyrium sanguineum, Sciadopitys verticillata, Sequoia sempervirens, Sequoiadendron giganteum, Sorghum bicolor, Spinacia spp., Sporobolus fimbriatus, Stiburus alopecuroides, Stylosanthos humilis, Tadehagi spp, Taxodium distichum, Themeda triandra, Trifolium spp., Triticum spp., Tsuga heterophylla, Vaccinium spp., Vicia spp., Vitis vinifera, Watsonia pyramidata, Zantedeschia aethiopica, Zea mays, amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugar cane, sunflower, tomato, squash, tea and algae, amongst others.

According to a preferred embodiment of the present invention, the plant is a crop plant such as soybean, sunflower, canola, alfalfa, rapeseed, cotton, tomato, potato or tobacco. Further preferably, the plant is a monocotyledonous plant, such as sugarcane. More preferably the plant is a cereal, such as rice, maize, wheat, barley, millet, rye, sorghum or oats.

### Class I HDZip hox5 polypeptides and homologues thereof, and class I HDZip hox5 nucleic acids/genes useful in the methods of the invention

The term "class I HDZip hox5 polypeptide or homologue thereof" as defined herein refers to a polypeptide comprising from N-terminal to C-terminal: (i) an acidic box; and (ii) a class I homeodomain; and (iii) a leucine zipper with more than 5 heptads.

Additionally, the class I HDZip hox5 polypeptide or a homologue thereof may comprise any one or both of the following: (a) a Trp tail; and (b) the RPFF amino acid motif, where R is Arg, P Pro and F Phe. Within this motif, are allowed one or more conservative change(s) at any position, and/or one or two non-conservative change(s) at any position. The motif of (b) precedes the acidic box, when examining the protein from N-terminal to C-terminal.

An example of a class I HDZip hox5 polypeptide as defined hereinabove comprising from N-terminal to C-terminal: (i) an acidic box; and (ii) a class I homeodomain; and (iii) a leucine zipper with more than 5 heptads; and additionally comprising: (a) a Trp tail; and (b) the RPFF amino acid motif, where R is Arg, P Pro and F Phe, is represented as in SEQ ID NO: 2. Further such examples are given in Table A of Example 1 herein.

A class I HDZip hox5 polypeptide or homologue thereof is encoded by a class I HDZip hox5 nucleic acid/gene. Therefore the term "class I HDZip hox5 nucleic acid/gene" as defined herein is any nucleic acid/gene encoding a class I HDZip hox5 polypeptide or a homologue thereof as defined hereinabove.

Class I HDZip hox5 polypeptides or homologues thereof may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues of class I HDZip hox5 comprising a class I homeodomain and a leucine zipper with more than 5 heptads may readily be identified using, for example, the ClustalW multiple sequence alignment algorithm (version 1.83) available at http://clustalw.genome.jp/sit-bin/nph-ClustalW, with the default pairwise alignment parameters, and a scoring method in percentage. Minor manual editing may be performed to optimise alignment between conserved motifs, as would be apparent to a person skilled in the art.

The various structural domains in a class I HDZip hox5 protein, such as the homeodomain and the leucine zipper, may be identified using specialised databases e.g. SMART (Schultz et al. (1998) Proc. Natl. Acad. Sci. USA 95, 5857-5864; Letunic et al. (2002) Nucleic Acids Res 30, 242-244; http://smart.embl-heidelberg.de/), InterPro (Mulder et al., (2003) Nucl. Acids. Res. 31, 315-318; http://www.ebi.ac.uk/interpro/), Prosite (Bucher and Bairoch (1994), A generalized profile syntax for biomolecular sequences motifs and its function in automatic sequence interpretation. (in) ISMB-94; Proceedings 2nd International Conference on Intelligent Systems for Molecular Biology. Altman R., Brutlag D., Karp P., Lathrop R., Searls D., Eds., pp53-61, AAAlPress, Menlo Park; Hulo et al., Nucl. Acids. Res. 32:D134-D137, (2004), http://www.expasy.org/prosite/) or Pfam (Bateman et al., Nucleic Acids Research 30(1):276-280 (2002), http://www.sanger.ac.uk/Software/Pfam/). Leucine zipper prediction and heptad identification may be done using specialised software such as 2ZIP, which combines a standard coiled coil prediction algorithm with an approximate search for the characteristic leucine repeat (Bornberg-Bauer et al. (1998) Computational Approaches to Identify Leucine Zippers, Nucleic Acids Res., 26(11): 2740-2746, http://2zip.molgen.mpg.de).

Furthermore, the presence of an acidic box may also readily be identified. Primary amino acid composition (in %) to determine if a polypeptide domain is rich in specific amino acids may be calculated using software programs from the ExPASy server, in particular the ProtParam tool (Gasteiger E et al. (2003) ExPASy: the proteomics server for in-depth protein knowledge and analysis. Nucleic Acids Res 31:3784-3788). The composition of the protein of interest may then be compared to the average amino acid composition (in %) in the Swiss-Prot Protein Sequence data bank. Within this databank, the average Asp (D) and Glu (E) content are of 5.3 % and of 6.6 % respectively, the combined average being of 11.9 %. As an example, the acidic box of SEQ ID NO: 2 contains 9.1 % of D and 54.5 % of E, the combined average being of 63.6 %. As defined herein, an acidic rich box has a combined Asp (D) and Glu (E) content (in % terms) above that found in the average amino acid composition (in % terms) of the proteins in the Swiss-Prot Protein Sequence database. An acidic box may be part of a transcription activation domain. Eukaryotic transcription activation domains have been classified according to their amino acid content, and major categories include acidic, glutamine-rich and proline-rich activation domains (Rutherford et al. (2005) Plant J. 43(5):769-88, and references therein).

A selected number of proteins amongst the class I HDZip hox5 polypeptides or homologues thereof further comprise the RPFF amino acid motif, where R is Arg, P Pro and F Phe. Within this motif, are allowed one or more conservative change(s) at any position, and/or one or two non-conservative change(s) at any position. This motif precedes the acidic box, when examining the protein from N-terminal to C-terminal (see Figure 2). The presence of the RPFF may be identified using methods for the alignment of sequences for comparison as described hereinabove. In some instances, the default parameters may be adjusted to modify the stringency of the search. For example using BLAST, the statistical significance threshold (called "expect" value) for reporting matches against database sequences may be increased to show less stringent matches. This way, short nearly exact matches may be identified.

A selected number of proteins amongst the class I HDZip hox5 polypeptides or homologues thereof may further comprise a Trp tail. A Trp tail as defined herein is the last 10 amino acids of the C-terminal of the protein comprising at least one Trp residue (see Figure 2).

Examples of class I HDZip hox5 polypeptides or homologues thereof (encoded by polynucleotide sequence accession number in parenthesis) are given in Table A.

It is to be understood that sequences falling under the definition of "class I HDZip hox5 polypeptide or homologue thereof" are not to be limited to the sequences given in Table A, but that any polypeptide comprising from N-terminal to C-terminal: (i) an acidic box; and (ii) a class I homeodomain; and (iii) a leucine zipper with more than 5 heptads, may be suitable for use in performance of the methods of the invention.

Class I HDZip hox5 polypeptides or homologues thereof have DNA binding activity, preferably to 5 bp half-sites that overlap at a central position, CAA(A/T)ATTG, as detected in yeast one-hybrid assays (Meijer et al. (2000) Mol Gen Genet 263:12-21). In transient assays on rice cell suspensions, co-bombardement of a class I HDZip hox5 polypeptide with the GUS reporter gene resulted in an increase number of stained spots, which were also more intense in color (Meijer et al, supra). This assay is useful to demonstrate the activator function of class I HDZip hox5 polypeptides or homologues.

Sequences useful in the methods of the invention are not limited to the aforementioned class I homeodomain leucine zipper (HDZip) hox5 nucleic acids and polypeptides. The methods according to the present invention may also be performed using variants of nucleic acids encoding class I homeodomain leucine zipper (HDZip) hox5 polypeptides or homologues thereof.

Examples of such variants include portions, hybridizing sequences, allelic variants, splice variants and variants obtained by gene shuffling.

A portion may be prepared, for example, by making one or more deletions to a nucleic acid. The portions may be used in isolated form or they may be fused to other coding (or non coding) sequences in order to, for example, produce a protein that combines several activities. When fused to other coding sequences, the resulting polypeptide produced upon translation may be bigger than that predicted for the portion.

Where the sequence useful in the methods of the invention is a class I HDZip hox5, the portion encodes a polypeptide comprising from N-terminal to C-terminal: (i) an acidic box; and (ii) a class I homeodomain; and (iii) a leucine zipper with more than 5 heptads. Preferably, the portion is a portion of one of the nucleic acids given in Table A. Most preferably the portion is a portion of a nucleic acid as represented by SEQ ID NO: 1.

The invention therefore provides a method for improving plant growth characteristics comprising modulating expression in a plant of a portion of a nucleic acid encoding a class I homeodomain leucine zipper (HDZip) hox5 polypeptide or a homologue.

Another nucleic acid variant is a nucleic acid capable of hybridising under reduced stringency conditions, preferably under stringent conditions, with a nucleic acid encoding a class I HDZip hox5 nucleic acid/gene or homologue thereof.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and are different under different environmental parameters. The skilled artisan is aware of various parameters which may be altered during hybridisation and washing and which will either maintain or change the stringency conditions.

The Tₘ is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisaton to be performed at 30 to 45°C, though the rate of hybridisation will be lowered. Base pair mismatches reduce the hybridisation rate and the thermal stability of the duplexes. On average and for large probes, the Tₘ decreases about 1°C per % base mismatch. The Tₘ may be calculated using the following equations, depending on the types of hybrids:
1. DNA-DNA hybrids (Meinkoth and Wahl, Anal. Biochem., 138: 267-284, 1984):
   Tₘ= 81.5°C + 16.6x1og[Na⁺]a + 0.41x%[G/C^{b}] - 500x[L^{c}]⁻¹ - 0.61x% formamide
2. DNA-RNA or RNA-RNA hybrids:
   Tₘ₌ 79.8 + 18.5 (log₁₀[Na⁺]^{a}) + 0.58 (%G/C^{b}) + 11.8 (%G/C^{b})² - 820/L^{c}
3. oligo-DNA or oligo-RNA^{d} hybrids:
   For <20 nucleotides: Tₘ= 2 (lₙ)
   For 20-35 nucleotides: Tₘ= 22 + 1.46 (lₙ)
   a or for other monovalent cation, but only accurate in the 0.01-0.4 M range.
   ^{b}only accurate for %GC in the 30% to 75% range.
   ^{c}L = length of duplex in base pairs.
   ^{d} Oligo, oligonucleotide; In, effective length of primer = 2x(no. of G/C)+(no. of A/T). Note: for each 1 % formamide, the Tₘ is reduced by about 0.6 to 0.7°C, while the presence of 6 M urea reduces the Tₘ by about 30°C

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. Conditions of greater or less stringency may also be selected. Generally, low stringency conditions are selected to be about 50°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with RNase. Examples of hybridisation and wash conditions are listed in Table 2 below.

**Table 2: Examples of hybridisation and wash conditions**

| Stringency Condition | Polynucleotide Hybrid ^{±} | Hybrid Length (bp) ^{‡} | ybridisation Temperature and Buffer ^{†} | Wash Temperature and Buffert |
|---|---|---|---|---|
| A | DNA:DNA | or equal to 50 | 65°C 1 xSSC; or 42°C, 1xSSCand50% formamide | 65°C1; 0.3xSSC |
| B | DNA:DNA | <50 | Tb*;1xSSC | Tb*;1xSSC |
| C | DNA:RNA | > or equal to 50 | 67°C 1 xSSC; or 45°C, 1×SSC and 50% formamide | 67°C; 0.3×SSC |
| D | DNA:RNA | <50 | Td*;1×SSC | Td*;1×SSC |
| E | RNA:RNA | > or equal to 50 | 70°C 1 ×SSC; or 50°C, 1×SSC and 50% formamide | 70°C; 0.3×SSC |
| F | RNA:RNA | <50 | Tf*;1×SSC | Tf*; 1×SSC |
| G | DNA:DNA | > or equal to 50 | 65°C 4×SSC; or 45°C, 4×SSC and 50% formamide | 65°C;1×SSC |
| H | DNA:DNA | <50 | Th*; 4 ×SSC | Th*;4×SSC |
| I | DNA:RNA | > or equal to 50 | 67°C 4×SSC; or 45°C, 4×SSC and 50% formamide | 67°C;1×SSC |
| J | DNA:RNA | <50 | Tj*;4 ×SSC | Tj*; 4 ×SSC |
| K | RNA:RNA | > or equal to 50 | 70°C 4×SSC; or 40°C, 6×SSC and 50% formamide | 67°C;1×SSC |
| L | RNA:RNA | <50 | Tj*; 2×SSC | Tj*; 2×SSC |
| M | DNA:DNA | > or equal to 50 | 50°C 4×SSC; or 40°C, 6×SSC and 50% formamide | 50°C; 2×SSC |
| N | DNA:DNA | <50 | Tn*; 6 ×SSC | Tn*; 6×SSC |
| O | DNA:RNA | > or equal to 50 | 55°C 4×SSC; or 42°C, 6×SSC and 50% formamide | 55°C; 2×SSC |
| P | DNA:RNA | <50 | Tp*; 6 ×SSC | Tp*; 6×SSC |
| Q | RNA:RNA | > or equal to 50 | 60°C 4×SSC; or 45°C, 6×SSC and 50% formamide | 60°C.; 2×SSC |
| R | RNA:RNA | <50 | Tr*; 4 ×SSC | Tr*; 4×SSC |

| | | | | |
|---|---|---|---|---|
| t The "hybrid length" is the anticipated length for the hybridising nucleic acid. When nucleic acids of known sequence are hybridised, the hybrid length may be determined by aligning the sequences and identifying the conserved regions described herein. t SSPE (1 XSSPE is 0.15M NaCl, 10mM NaH₂P0₄, and 1.25mM EDTA, pH7.4) may be substituted for SSC (1 xSSC is 0.15M NaCl and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 µg/ml denatured, fragmented salmon sperm DNA, 0.5% sodium pyrophosphate, and up to 50% formamide. * Tb-Tr: The hybridisation temperature for hybrids anticipated to be less than 50 base pairs in length should be 5-10°C less than the melting temperature Tₘ of the hybrids; the Tₘ is determined according to the above-mentioned equations. ± The present invention also encompasses the substitution of any one, or more DNA or RNA hybrid partners with either a PNA, or a modified nucleic acid. | | | | |

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

Where the sequence useful in the methods of the invention is a class I HDZip hox5, the hybridizing sequence encodes a polypeptide comprising from N-terminal to C-terminal: (i) an acidic box; and (ii) a class I homeodomain; and (iii) a leucine zipper with more than 5 heptads. Preferably, the hybidising sequence is capable of hybridizing under reduced stringency conditions, preferably under stringent conditions, with one of the nucleic acids given in Table A or a portion thereof as defined herein. Most preferably the portion is a portion of a nucleic acid as represented by SEQ ID NO: 1.

The invention therefore provides a method for improving plant growth characteristics comprising modulating expression in a plant of a nucleic acid capable of hybridising under reduced stringency, preferably under stringent conditions to a nucleic acid encoding a class I homeodomain leucine zipper (HDZip) hox5 polypeptide or a homologue thereof.

The nucleic acids or variant thereof may be derived from any natural or artificial source. The nucleic acid/gene or variant thereof may be isolated from a microbial source, such as yeast or fungi, or from a plant, algae or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a monocotyledonous species, preferably from the family Poaceae, further preferably from Oryza genus, most preferably from Oryza sativa. The nucleic acid may be isolated from a dicotyledonous species, preferably from the family Brassicaceae, further preferably from Arabidopsis thaliana.

The expression of a nucleic acid may be modulated by introducing a genetic modification (preferably in the locus of the gene in question). The locus of a gene as defined herein is taken to mean a genomic region, which includes the gene of interest and 10 kb up- or down stream of the coding region.

The genetic modification may be introduced, for example, by any one (or more) of the following methods: T-DNA activation, TILLING, site-directed mutagenesis, directed evolution and homologous recombination or by introducing and expressing in a plant a nucleic acid encoding a class I HDZip hox5 polypeptide or a homologue thereof. Following introduction of the genetic modification, there follows a step of selecting for modulated expression of the nucleic acid, which modulation in expression gives plants having improved growth characteristics, particularly increased yield.

T-DNA activation tagging (Hayashi et al. Science (1992) 1350-1353) involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through Agrobacterium infection and leads to overexpression of genes near the inserted T-DNA. The resulting transgenic plants show dominant phenotypes due to overexpression of genes close to the introduced promoter. The promoter to be introduced may be any promoter capable of directing expression of a gene in the desired organism, in this case a plant. For example, constitutive, tissue-preferred, cell type-preferred and inducible promoters are all suitable for use in T-DNA activation.

A genetic modification may also be introduced in the locus of the gene in question using the technique of TILLING (Targeted Induced Local Lesions In Genomes). This is a mutagenesis technology useful to generate and/or identify and isolate mutagenised variants. TILLING also allows selection of plants carrying such mutant variants. These mutant variants may even exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Site-directed mutagenesis may be used to generate variant nucleic acids. Several methods are available to achieve site-directed mutagenesis, the most common being PCR based methods (Current Protocols in Molecular Biology, Wiley Eds http://www.4ulr.com/products/currentprotocols/index.html).

Directed evolution or gene shuffling may also be used to generate nucleic acid variants. This consists of iterations of DNA shuffling followed by appropriate screening and/or selection to generate variants having a modified biological activity (Castle et al., (2004) Science 304(5674): 1151-4; US patents 5,811,238 and 6,395,547).

T-DNA activation, TILLING, site-directed mutagenesis and directed evolution are examples of technologies that enable the generation of novel alleles and variants.

Homologous recombination allows introduction in a genome of a selected nucleic acid at a defined selected position. Homologous recombination is a standard technology used routinely in biological sciences for lower organisms such as yeast or the moss Physcomitrella. Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8). The nucleic acid to be targeted need not be targeted to the locus of the gene in question, but may be introduced in, for example, regions of high expression. The nucleic acid to be targeted may be an improved allele used to replace the endogenous gene or may be introduced in addition to the endogenous gene.

A preferred method for introducing a genetic modification is to introduce and express in a plant a nucleic acid encoding a class I HDZip hox5 polypeptide or a homologue thereof. The nucleic acid to be introduced into a plant may be a full-length nucleic acid or may be a portion or a hybridising sequence as hereinbefore defined.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break α-helical structures or β-sheet structures). Conservative substitution tables are well known in the art (see for example Creighton (1984) Proteins. W.H. Freeman and Company and Table 4 below). The homologues useful in the methods according to the invention are preferably polypeptides have in increasing order of preference at least 30%, 40%, 50%, 60%, 70%, 80%, 85%, 90%, 95%, 96%, 97%, 98% or 99% or more sequence identity or similarity (functional identity) to the sequences mentioned herein as being useful in the methods of the invention, for example the sequences given in Table A and I herein

Also encompassed by the term "homologues" are two special forms of homology, which include orthologous sequences and paralogous sequences, which encompass evolutionary concepts used to describe ancestral relationships of genes. The term "paralogous" relates to gene-duplications within the genome of a species leading to paralogous genes. The term "orthologous" relates to homologous genes in different organisms due to speciation.

Orthologues in, for example, monocot plant species may easily be found by performing a so-called reciprocal blast search. Taking the example of finding an orthologue or pralogue of a a class I HDZip hox5 nucleic acid or class I HDZip hox5 polypeptide, this may be done by a first blast involving blasting the sequence in question (for example, SEQ ID NO: 1 or SEQ ID NO: 2) against any sequence database, such as the publicly available NCBI database which may be found at: http://www.ncbi.nlm.nih.gov. BLASTN or TBLASTX may be used when starting from nucleotide sequence, or BLASTP or TBLASTN when starting from the protein, with standard default values. The BLAST results may be filtered. The full-length sequences of either the filtered results or the non-filtered results are then BLASTed back (second BLAST) against the sequences of the organism from which the sequence in question is derived. The results of the first and second BLASTs are then compared. When the results of the second BLAST give as hits with the highest similarity a class I HDZip hox5 nucleic acid or class I HDZip hox5 polypeptide, then a paralogue has been found, if it originates from the same organism as for the sequence used in the first BLAST. In case it originates from an organism other than that of the sequence used in the first BLAST, then an orthologue has been found. In the case of large families, ClustalW may be used, followed by a neighbour joining tree, to help visualize the clustering.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions. Conservative substitution tables are readily available in the art. The table below gives examples of conserved amino acid substitutions.

**Table 4: Examples of conserved amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

A homologue may also be in the form of an "insertional variant" of a protein, i.e. where one or more amino acid residues are introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tang-100 epitope, c-myc epitope, FLAG@-epitope, lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

The class I HDZip hox5 polypeptide or homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein. The Derivatives may comprise naturally occurring altered, glycosylated, acylated, prenylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The class I HDZip hox5 polypeptide or homologue thereof may be encoded by an alternative splice variant.

Where the sequence useful in the methods of the invention is a nucleic acid encoding a class I HDZip hox5 polypeptide or homologue thereof, the splice variant encodes a polypeptide comprising from N-terminal to C-terminal: (i) an acidic box; and (ii) a class I homeodomain; and (iii) a leucine zipper with more than 5 heptads. Additionally, the class I HDZip hox5 polypeptide or a homologue thereof may comprise one or both of the following: (a) a Trp tail; and (b) the RPFF amino acid motif, where R is Arg, P Pro and F Phe. Within this motif, are allowed one or more conservative change(s) at any position, and/or one or two non-conservative change(s) at any position. The motif of (b) precedes the acidic box, when examining the protein from N-terminal to C-terminal. Further preferred are splice variants of nucleic acid sequences given in Table A. Most preferred is a splice variant of a nucleic acid sequence as represented by SEQ ID NO: 1.

The present invention therefore provides a method for improving plant growth characteristics comprising modulating expression in a plant of an alternative splice variant of a nucleic acid encoding a class I homeodomain leucine zipper (HDZip) hox5 polypeptide or a homologue thereof.

The term "alternative splice variant" as used herein encompasses variants of a nucleic acid sequence in which selected introns and/or exons have been excised, replaced or added, or in which introns have been shortened or lengthened. Such variants will be ones in which the biological activity of the protein is retained, which may be achieved by selectively retaining functional segments of the protein. Such splice variants may be found in nature or may be manmade. Methods for making such splice variants are well known in the art.

The homologue may also be encoded by an allelic variant of a nucleic acid encoding a class I HDZip hox5 polypeptide or a homologue thereof.

Where the sequence useful in the methods of the invention is a nucleic acid encoding a class I HDZip hox5 polypeptide or homologue thereof, the allelic variant encodes a polypeptide comprising from N-terminal to C-terminal: (i) an acidic box; and (ii) a class I homeodomain; and (iii) a leucine zipper with more than 5 heptads. Additionally, the class I HDZip hox5 polypeptide or a homologue thereof may comprise one or both of the following: (a) a Trp tail; and (b) the RPFF amino acid motif, where R is Arg, P Pro and F Phe. Within this motif, are allowed one or more conservative change(s) at any position, and/or one or two non-conservative change(s) at any position. The motif of (b) precedes the acidic box, when examining the protein from N-terminal to C-terminal. Further preferred are allelic variants of nucleic acid sequences given in Table A. Most preferred is an allelic variant of a nucleic acid sequence as represented by SEQ ID NO: 1.

The present invention therefore provides a method for improving plant growth characteristics comprising modulating expression in a plant of an allelic variant of a nucleic acid encoding a class I homeodomain leucine zipper (HDZip) hox5 polypeptide or a homologue thereof.

Allelic variants exist in nature, and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

According to the present invention, modulated expression of the nucleic acid or variant thereof is envisaged. Methods for modulating expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of the nucleic acid or variant thereof. For example, endogenous promoters may be altered in vivo by mutation, deletion, and/or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

Methods for reducing the expression of genes or gene products are well documented in the art.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

The invention also provides genetic constructs and vectors to facilitate introduction and/or expression of the nucleotide sequences useful in the methods according to the invention.

Therefore, there is provided a gene construct comprising:
(i) A class I HDZip hox5 nucleic acid or variant thereof;
(ii) One or more control sequences capable of driving expression of the nucleic acid sequence of (i); and optionally
(iii) A transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest. The sequence of interest is operably linked to one or more control sequences (at least to a promoter). The terms "regulatory element", "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence. The promoter may be an inducible promoter, i.e. having induced or increased transcription initiation in response to a developmental, chemical, environmental or physical stimulus. An example of an inducible promoter being a stress-inducible promoter, i.e. a promoter activated when a plant is exposed to various stress conditions. Additionally or alternatively, the promoter may be a tissue-preferred promoter, i.e. one that is capable of preferentially initiating transcription in certain tissues, such as the leaves, roots, seed tissue etc. Promoters able to initiate transcription in certain tissues only are referred to herein as "tissue-specific".

Preferably, the class I HDZip hox5 nucleic acid or variant thereof is operably linked to a constitutive promoter. A constitutive promoter is transcriptionally active during most but not necessarily all phases of growth and development and is substantially ubiquitously expressed. The constitutive promoter is preferably a GOS2 promoter, more preferably the constitutive promoter is a rice GOS2 promoter, further preferably the constitutive promoter is represented by a nucleic acid sequence substantially similar to SEQ ID NO: 33, most preferably the constitutive promoter is as represented by SEQ ID NO: 33. It should be clear that the applicability of the present invention is not restricted to the class I HDZip hox5 nucleic acid or variant thereof when driven by a GOS2 promoter. Examples of other constitutive promoters which may also be used perform the methods of the invention are shown in Table 5 below.

**Table 5: Examples of constitutive promoters**

| Gene Source | Reference |
|---|---|
| Actin | McElroy et al, Plant Cell, 2: 163-171, 1990 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| Nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

For the identification of functionally equivalent promoters, the promoter strength and/or expression pattern of a candidate promoter may be analysed for example by operably linking the promoter to a reporter gene and assay the expression level and pattern of the reporter gene in various tissues of the plant. Suitable well-known reporter genes include for example beta-glucuronidase or beta galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1,000 transcripts per cell.

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene that confers a phenotype on a cell in which it is expressed to facilitate the identification and/or selection of cells that are transfected or transformed with a nucleic acid construct of the invention.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. As used herein, the term "selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xlose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the lo×P sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

In a preferred embodiment, there is provided a gene construct comprising:
(i) A class I HDZip hox5 nucleic acid or variant thereof;
(ii) A constitutive promoter capable of driving expression of the nucleic acid sequence of (i); and optionally
(iii) A transcription termination sequence.

The constitutive promoter is preferably a GOS2 promoter, more preferably the constitutive promoter is the rice GOS2 promoter, further preferably the constitutive promoter is represented by a nucleic acid sequence substantially similar to SEQ ID NO: 33, most preferably the constitutive promoter is as represented by SEQ ID NO: 33. The invention further provides use of a construct as defined hereinabove in the methods of the invention.

The present invention also encompasses plants obtainable by the methods according to the present invention. The present invention therefore provides plants, plant parts (including plant cells) obtainable by the methods according to the present invention, which plants or parts (including cells) comprise a transgene class I HDZip hox5 nucleic acid or variant thereof.

The invention also provides a method for the production of transgenic plants having improved growth characteristics, particularly increased yield, relative to corresponding wild type or other control plants, comprising introduction and expression in a plant of any of the nucleic acids described herein as being useful in the methods of the invention.

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
a) the nucleic acid sequences encoding proteins useful in the methods of the invention, or
b) genetic control sequence(s) which is operably linked with the nucleic acid sequence according to the invention, for example a promoter, or
c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

More specifically, the present invention provides a method for the production of transgenic plants having improved growth characteristics (particularly increased yield) which method comprises:
(i) introducing and expressing in a plant, plant part or plant cell a class I HDZip hox5 nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al. (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al. (1986) Mol. Gen Genet 202: 179-185); DNA or RNA-coated particle bombardment (Klein TM et al. (1987) Nature 327: 70) infection with (non-integrative) viruses and the like. Transgenic rice plants are preferably produced via Agrobacterium-mediated transformation using any of the well known methods for rice transformation, such as described in any of the following: published European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells comprising an isolated class IHDZiphox5 nucleic acid or variant thereof. Preferred host cells are plant cells.

The invention also extends to harvestable parts of a plant such as, but not limited to seeds, leaves, fruits, flowers, stems, rhizomes, tubers and bulbs. The invention furthermore relates to products derived from a harvestable part of such a plant, such as dry pellets or powders, oil, fat and fatty acids, starch or proteins.

The present invention also encompasses use of class I HDZip hox5 nucleic acids and variants thereof and use of class I HDZip hox5 polypeptides and homologues thereof. Such uses relate to improving any of the plant growth characteristics as defined hereinabove.

Class I HDZip hox5 nucleic acids or variants thereof, or class I HDZip hox5 polypeptides or homologues thereof may find use in breeding programmes in which a DNA marker is identified which may be genetically linked to one of the aforementioned nucleic acids or variants. The nucleic acids or variants, or polypeptides or homologues thereof may be used to define a molecular marker. This DNA or protein marker may then be used in breeding programmes to select plants having improved growth characteristics (such as increased yield). The nucleic acids or variants are as defined hereinabove.

Allelic variants of a class I HDZip hox5 nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give improved growth characteristics, such as increased yield. Selection is typically carried out by monitoring growth performance of plants containing different allelic variants of the sequence in question, for example, different allelic variants of any of the nucleic acids/genes described herein as being useful in the methods of the invention. Growth performance may be monitored in a greenhouse or in the field. Further optional steps include crossing plants in which the superior allelic variant was identified with another plant. This could be used, for example, to make a combination of interesting phenotypic features.

The aforementioned nucleic acid and variants may also be used as probes for genetically and physically mapping the genes that they are a part of, and as markers for traits linked to those genes. Such information may be useful in plant breeding in order to develop lines with desired phenotypes. Such use of the nucleic acids or variants requires only a nucleic acid sequence of at least 15 nucleotides in length. The nucleic acids or variants may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids or variants. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid or variant in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art. The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having improved growth characteristics, as described hereinbefore. These improved growth characteristics may also be combined with other economically advantageous traits, such as further yield-enhancing traits, tolerance to other abiotic and biotic stresses, traits modifying various architectural features and/or biochemical and/or physiological features.

### Items

1. Method for increasing plant yield relative to corresponding wild type plants comprising modulating expression in a plant of a nucleic acid encoding class I homeodomain leucine zipper (HDZip) hox5 polypeptide or homologue thereof, and optionally selecting for plants having increased yield, wherein said class I HDZip hox5 polypeptide or homologue comprises from N-terminal to C-terminal: (i) an acidic box; and (ii) a class I homeodomain; and (iii) a leucine zipper with more than 5 heptads.
2. Method according to item 1, wherein said class I HDZip hox5 polypeptide or homologue thereof further comprises one or both of the following: (i) a Trp tail; and (ii) a RPFF amino acid motif, where R is Arg, P Pro and F Phe, and within this motif, allowing one or more conservative change(s) at any position, and/or one or two non-conservative change(s) at any position.
3. Method according to item 1 or 2, wherein said modulated expression is effected by introducing a genetic modification preferably in the locus of a gene encoding a class I HDZip hox5 polypeptide or a homologue thereof.
4. Method according to item 3, wherein said genetic modification is effected by one of:
   T-DNA activation, TILLING, site-directed mutagenesis or directed evolution.
5. Method for increasing plant yield relative to corresponding wild type plants comprising introducing and expressing in a plant a class I HDZip hox5 nucleic acid or a variant thereof.
6. Method according to item 5, wherein said variant is a portion of a class I HDZip hox5 nucleic acid, which portion encodes a polypeptide comprising from N-terminal to C-terminal: (i) an acidic box; and (ii) a class I homeodomain; and (iii) a leucine zipper with more than 5 heptads.
7. Method according to item 5, wherein said variant is a sequence capable of hybridising to a class I HDZip hox5 nucleic acid, which hybridising sequence encodes a polypeptide comprising from N-terminal to C-terminal: (i) an acidic box; and (ii) a class I homeodomain; and (iii) a leucine zipper with more than 5 heptads.
8. Method according to items 5 to 7, wherein said class I HDZip hox5 nucleic acid or variant thereof is overexpressed in a plant.
9. Method according to any one of items 5 to 8, wherein said class I HDZip hox5 nucleic acid or variant thereof is of plant origin, preferably from a monocotyledon plant, further preferably from the family Poaceae, more preferably from the genus Oryza, most preferably from Oryza sativa.
10. Method according to any one of items 5 to 9, wherein said variant encodes an orthologue or paralogue of the class I HDZip hox5 protein of SEQ ID NO: 2.
11. Method according to any one of items 5 to 10, wherein said class I HDZip hox5 nucleic acid or variant thereof is operably linked to a constitutive promoter.
12. Method according to item 11, wherein said constitutive promoter is a GOS2 promoter, more preferably the constitutive promoter is a rice GOS2 promoter, further preferably the constitutive promoter is represented by a nucleic acid sequence substantially similar to SEQ ID NO: 33, most preferably the constitutive promoter is as represented by SEQ ID NO: 33.
13. Method according to any one of items 1 to 12, wherein said increased yield is selected from one or more of: increased number of filled seeds, increased total seed yield, increased number of flowers per panicle, increased seed fill rate, increased harvest index (HI), increased thousand kernel weight (TKW), increased root length or increased root diameter, each relative to corresponding wild type plants.
14. Method according to any one of items 1 to 13, wherein said increased yield occurs under abiotic stress.
15. Method according to item 14, wherein said abiotic stress is an osmotic stress, selected from one or more of: water stress, salt stress, oxidative stress and ionic stress, preferably wherein said water stress is drought stress.
16. Method according to item 14 or 15, wherein said abiotic stress tolerance is manifested as increased yield selected from one or more of: increased number of filled seeds, increased total seed yield, increased number of flowers per panicle, increased seed fill rate, increased HI, increased TKW, increased root length or increased root diameter, each relative to corresponding wild type plants.
17. Method according to items 13 to 16, wherein said increased yield comprises increased greenness index.
18. Plant, plant part or plant cell obtainable by a method according to any one of items 1 to 17.
19. Use of a construct comprising:
   (i) a class I HDZip hox5 nucleic acid or variant thereof
   (ii) one or more control sequences capable of driving expression of the nucleic acid sequence of (i), and optionally
   (iii) a transcription termination sequence
      in a method according to any one of items 5 to 17.
20. Construct according to item 19, wherein said control sequence is a constitutive promoter.
21. Construct according to item 20, wherein said constitutive promoter is a GOS2 promoter, preferably the rice GOS2 promoter, further preferably the constitutive promoter is represented by a nucleic acid sequence substantially similar to SEQ ID NO: 33, most preferably the constitutive promoter is as represented by SEQ ID NO: 33.
22. Plant, plant part or plant cell transformed with a construct comprising a class I HDZip hox5 nucleic acid or variant thereof under the control of a GOS2 promoter.
23. Method for the production of a transgenic plant having increased yield relative corresponding wild type plant, which method comprises:
   (i) introducing and expressing in a plant or plant cell a class I HDZip hox5 nucleic acid or variant thereof;
   (ii) cultivating the plant cell under conditions promoting plant growth and development.
24. Method according to item 23 wherein said increased yield occurs under increased abiotic stress.
25. Transgenic plant having increased yield relative to corresponding wild type plant, said increased yield resulting from a class I HDZip hox5 nucleic acid or a variant thereof introduced into said plant.
26. Transgenic plant according to item 18, 22 or 25, wherein said plant is a monocotyledonous plant, such as sugar cane or wherein the plant is a cereal, such as rice, maize, wheat, barley, millet, rye, oats or sorghum.
27. Harvestable parts of a plant according to any one of items 18, 22, 25 or 26.
28. Harvestable parts of a plant according to item 27, wherein said harvestable parts are seeds.
29. Products derived from a plant according to item 26 and/or from harvestable parts of a plant according to items 27 or 28.
30. Use of a class I HDZip hox5 nucleic acid/gene or variant thereof, or use of a class I HDZip hox5 polypeptide or homologue thereof, in increasing plant yield relative to corresponding wild type plants.
31. Use according to item 30, wherein said increased yield is selected from one or more of: increased number of filled seeds, increased total seed yield, increased number of flowers per panicle, increased seed fill rate, increased HI, increased TKW, increased root length or increased root diameter, each relative to corresponding wild type plants.
32. Use according to items 30 or 31, wherein said increased yield occurs under increased abiotic stress.
33. Use according to item 32, wherein said increased yield comprises increased greenness index.
34. Use of a class I HDZip hox5 nucleic acid/gene or variant thereof, or use of a class I HDZip hox5 polypeptide or homologue thereof, as a molecular marker.

### Description of figures

The present invention will now be described with reference to the following figures in which:
Fig. 1 shows a multiple alignment of class I HDZip homeodomains from different plant sources, using VNTI AlignX multiple alignment program, based on a modified ClustalW algorithm (InforMax, Bethesda, MD, http://www.informaxinc.com), with default settings for gap opening penalty of 10 and a gap extension of 0.05). The homeodomain invariant amino acids L₁₆, W₄₈, F₄₉, N₅₁ and R₅₃ are boxed vertically. HDZip Class I preferred amino acids A₄₆ and W₅₆ are equally boxed vertically. The three helixes necessary for DNA binding are marked as black boxes above the alignment. The six heptads are separated by a vertical line. The seven positions within each heptad are named a, b, c, d, e, f and g. The Leu occupies the d position within each heptad, and is boxed vertically.
Fig. 2 shows a multiple alignment of several plant class I HDZip hox5 polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The three main characterized domains, from N-terminal to C-terminal, are heavily boxed and identified as the acidic box, the class I homeodomain and the six heptad- leucine zipper. Additionally, the Trp tail and the RPFF amino acid motif are lightly boxed.

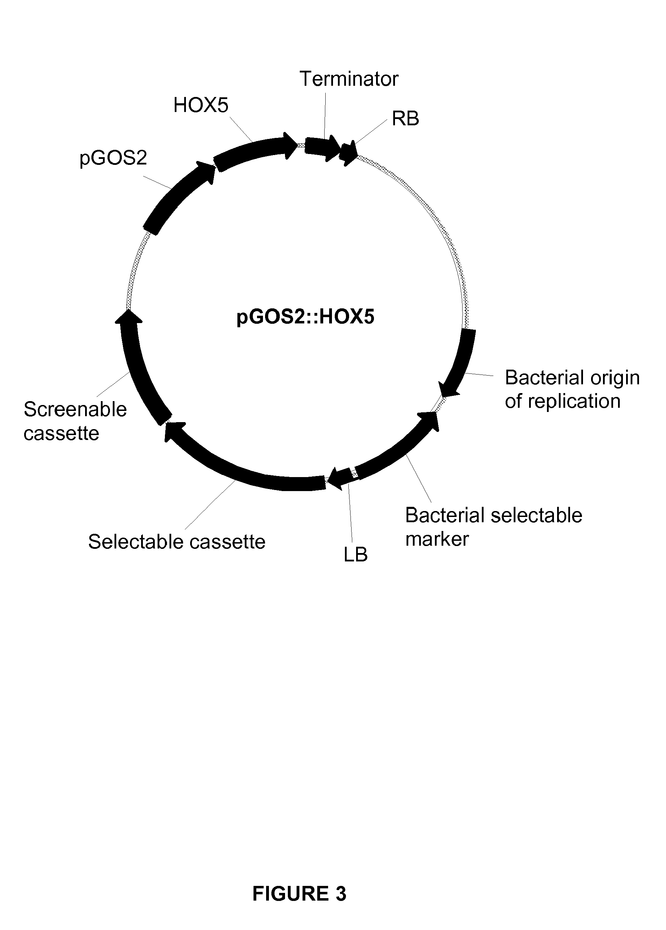

Fig. 3 shows a binary vector for expression in Oryza sativa of an Oryza sativa class I HDZip hox5 under the control of a GOS2 promoter.

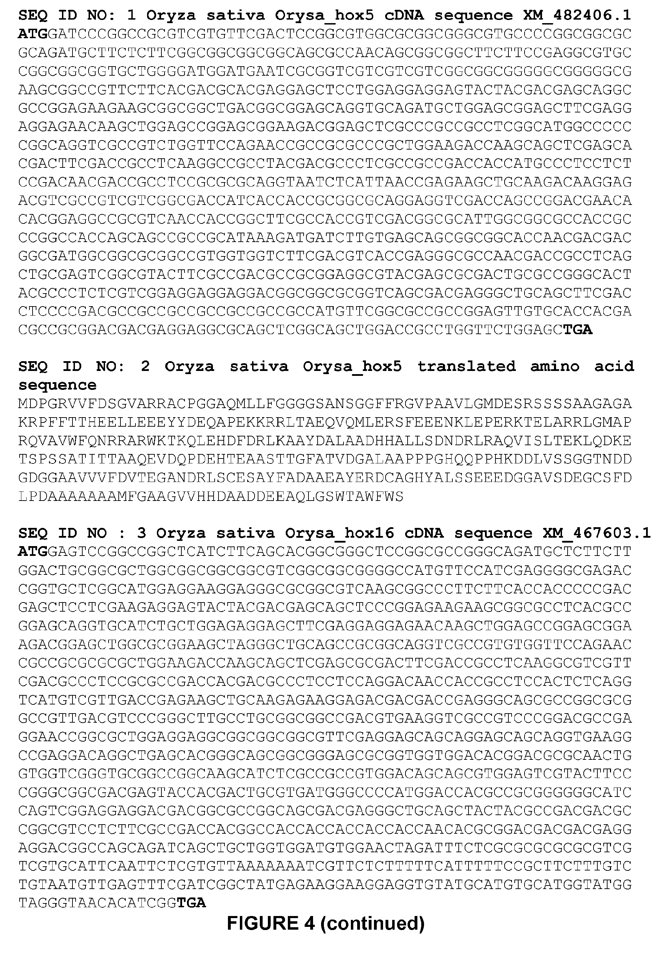

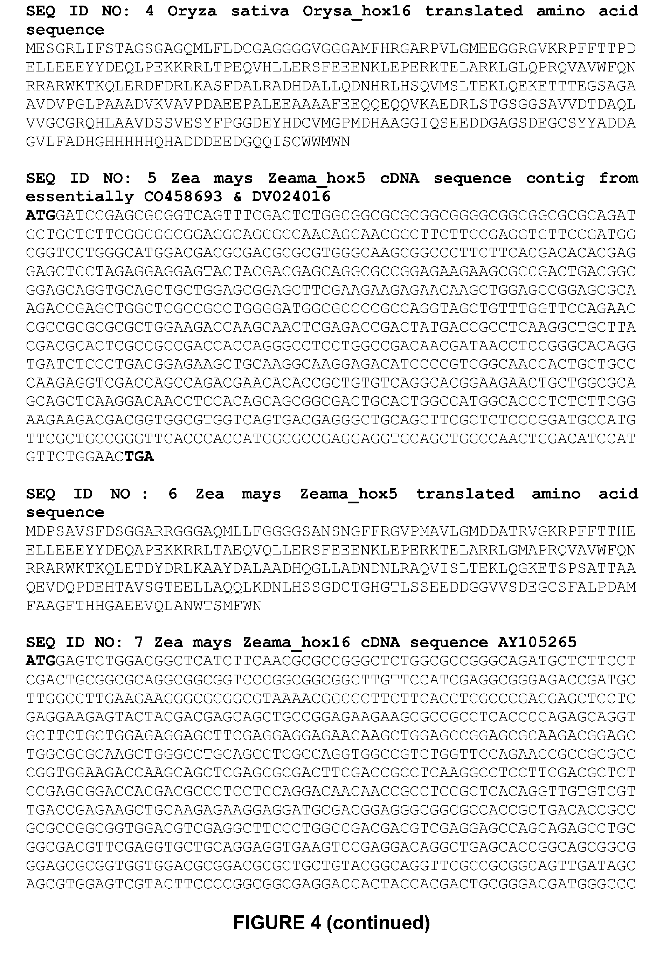

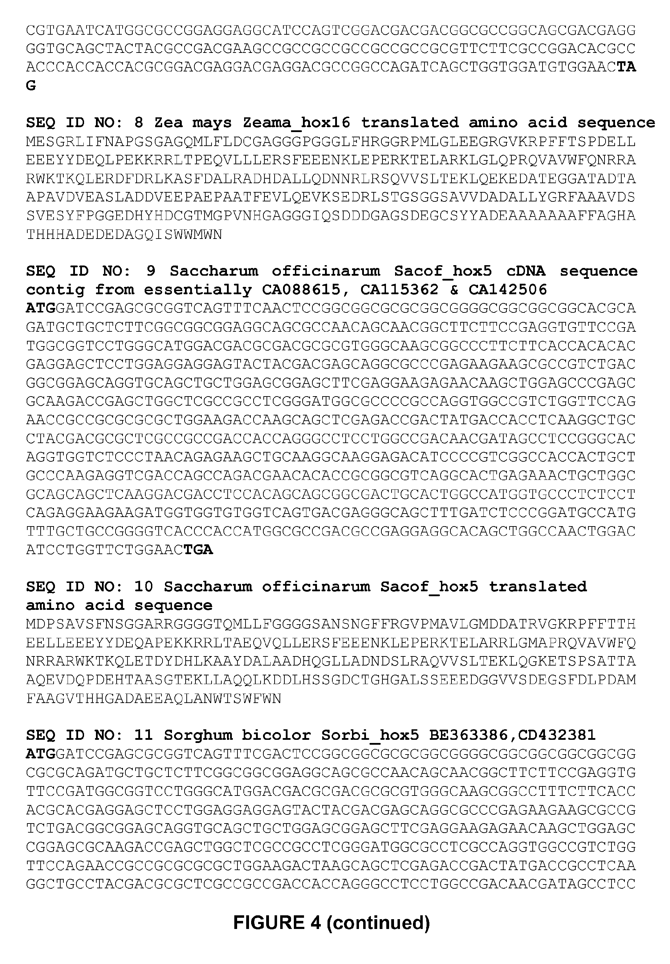

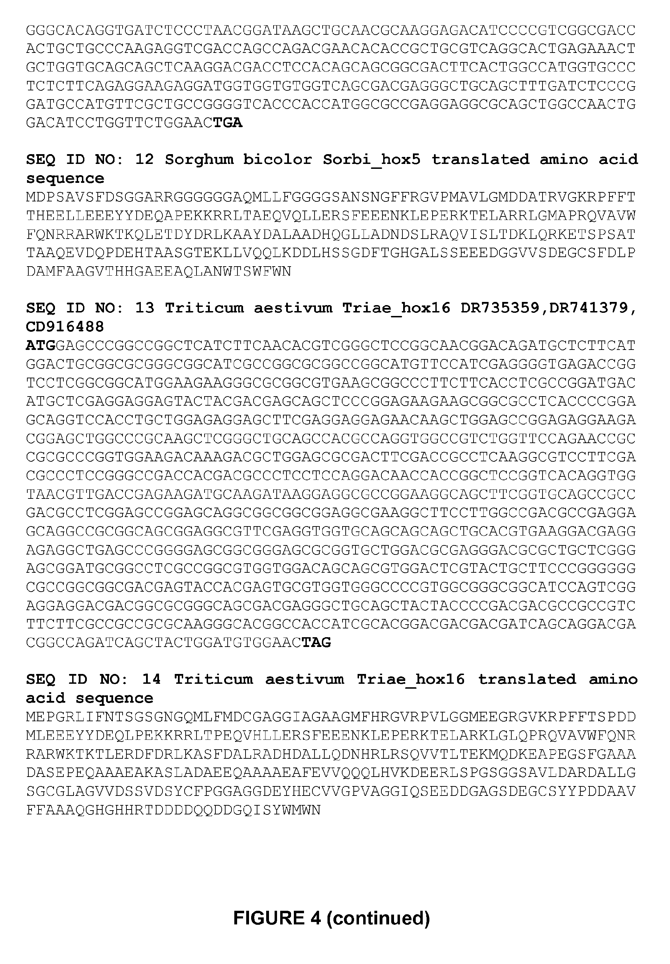

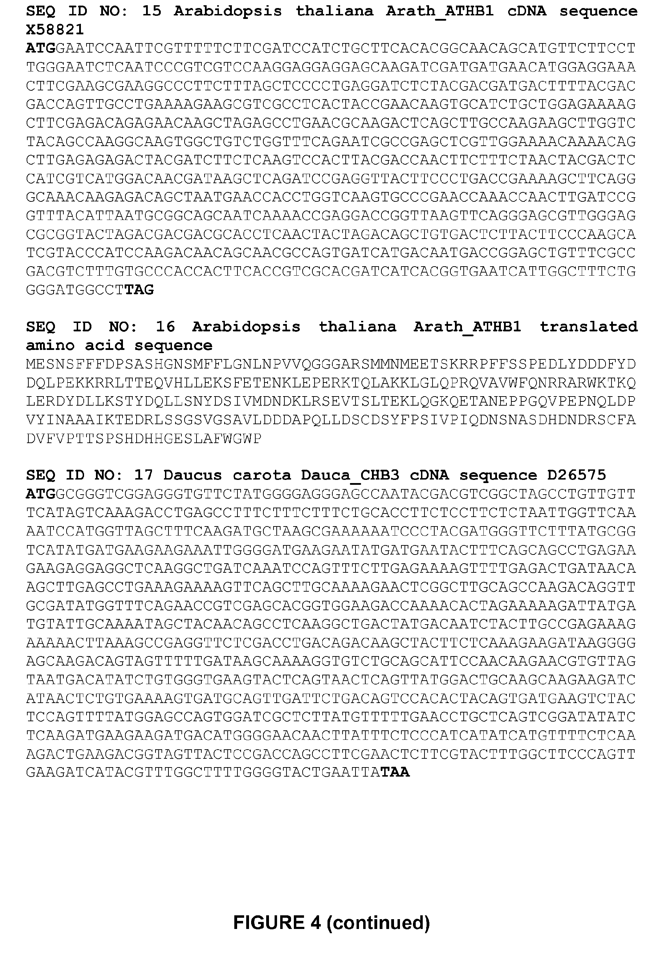

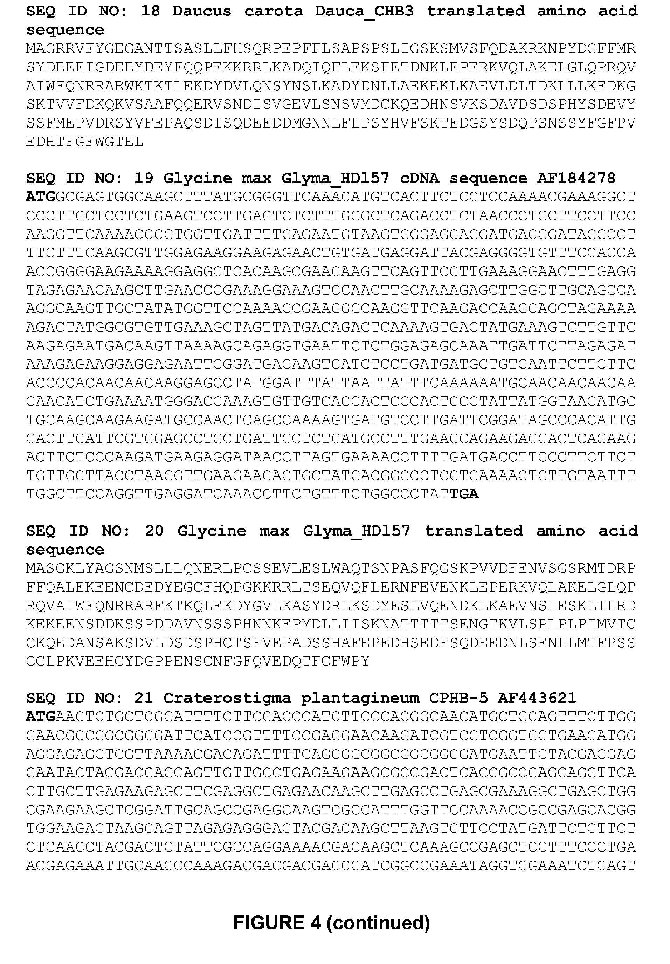

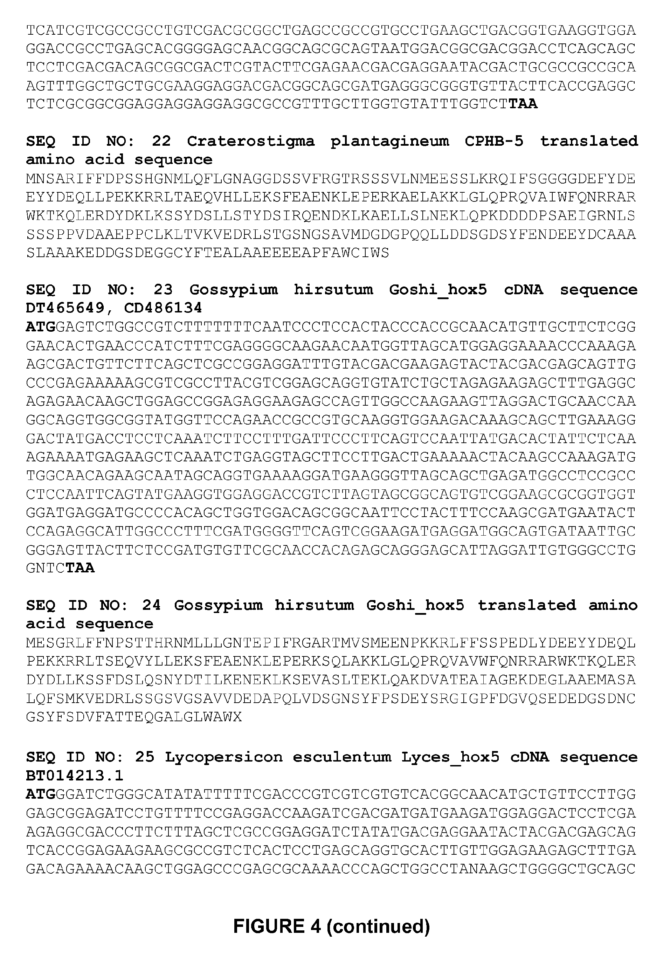

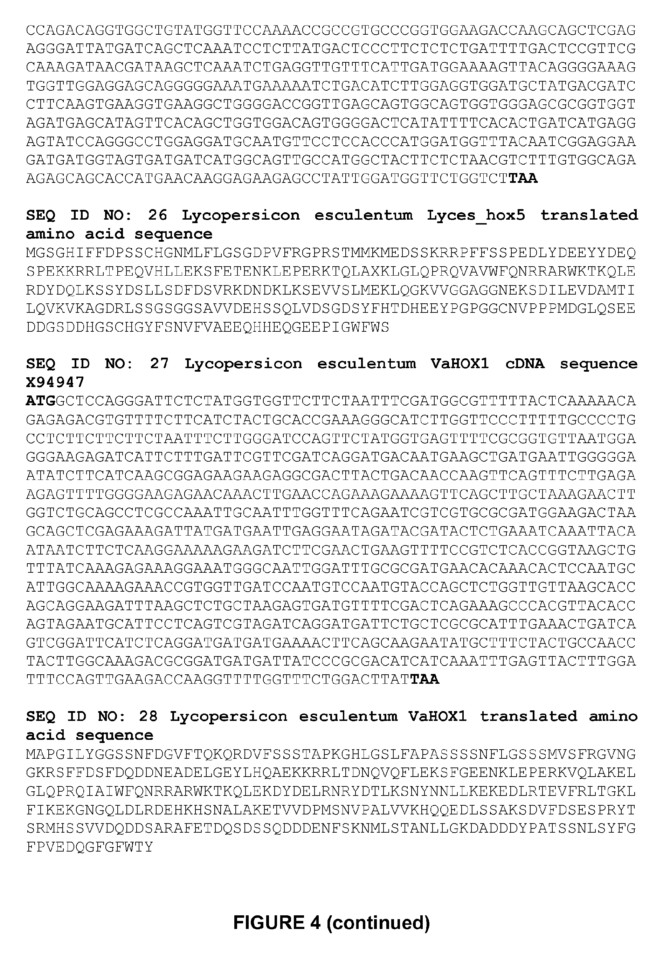

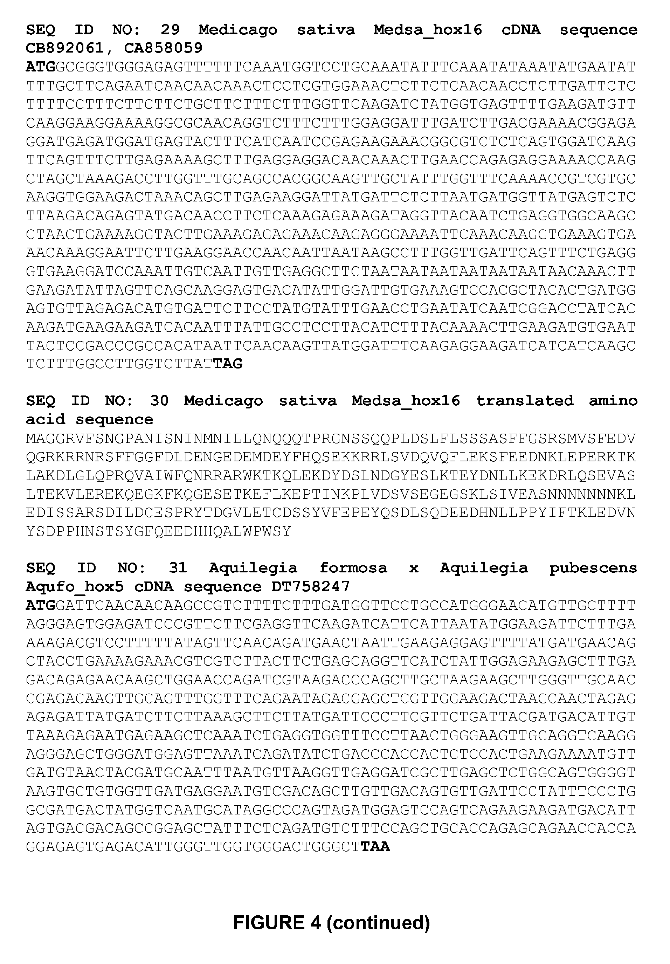

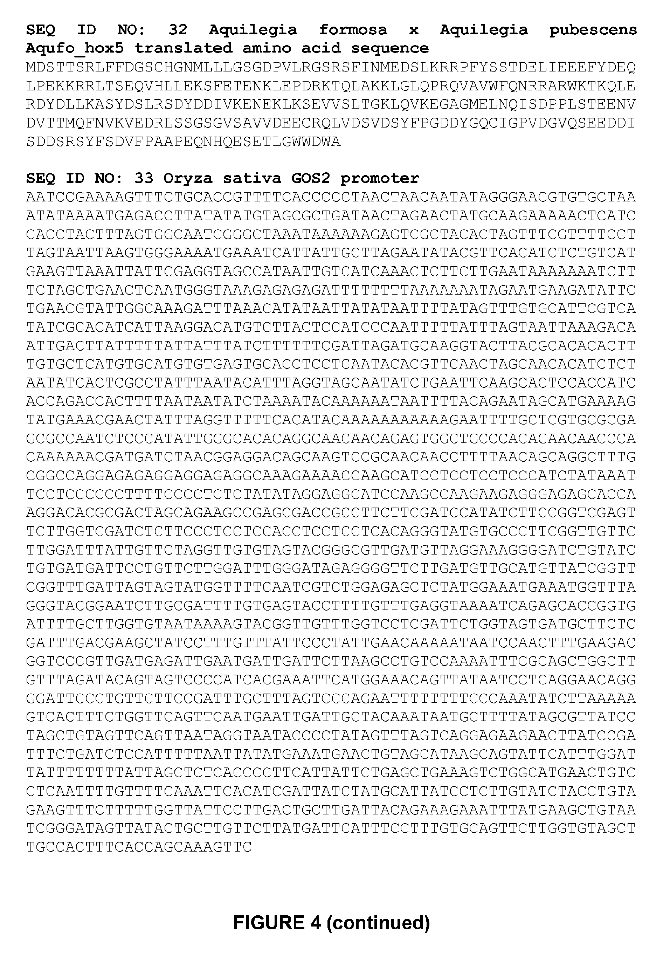

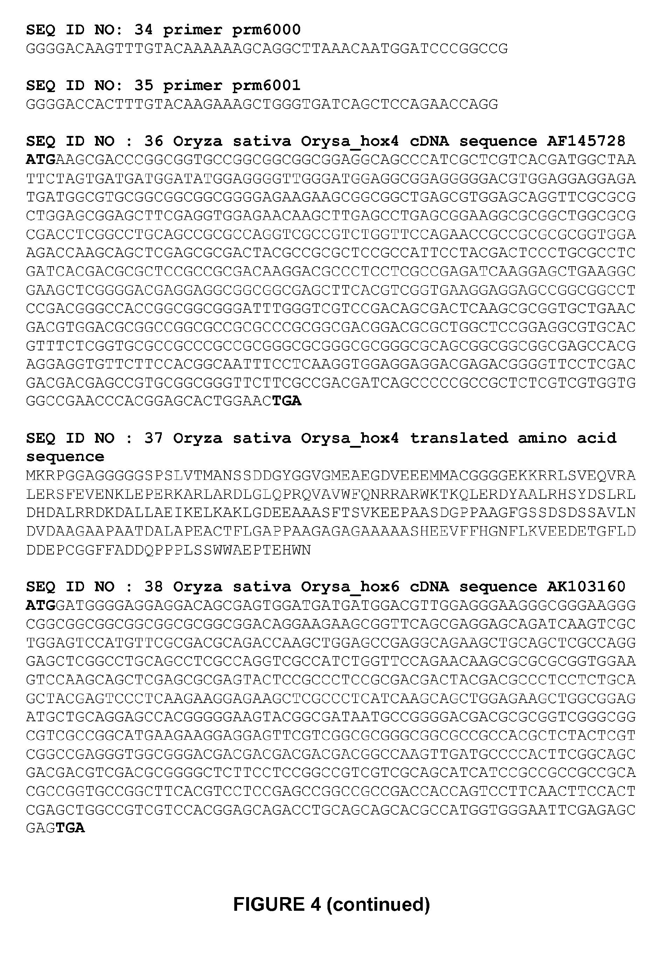

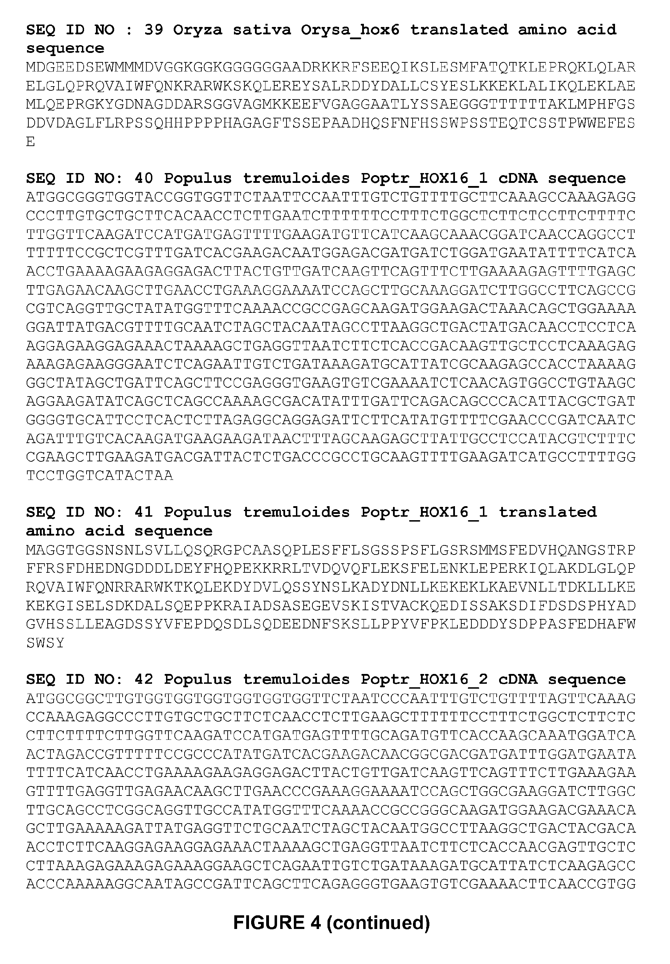

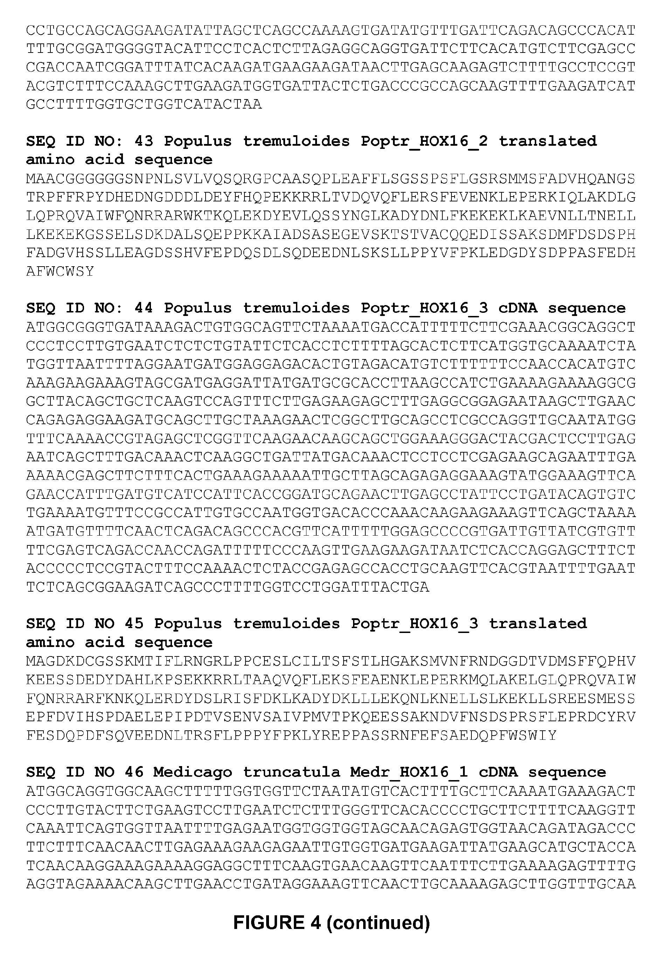

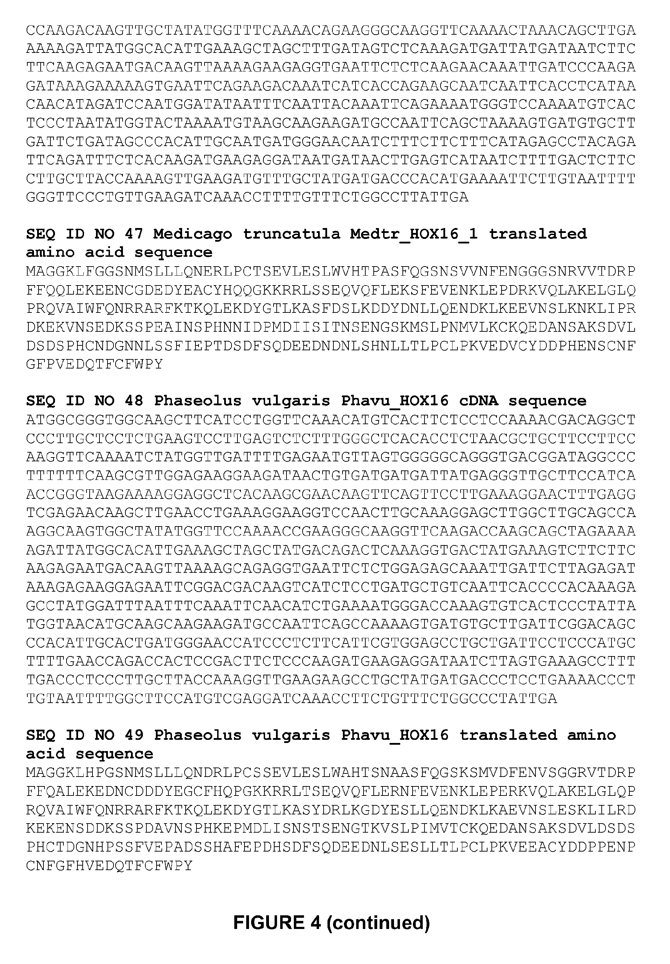

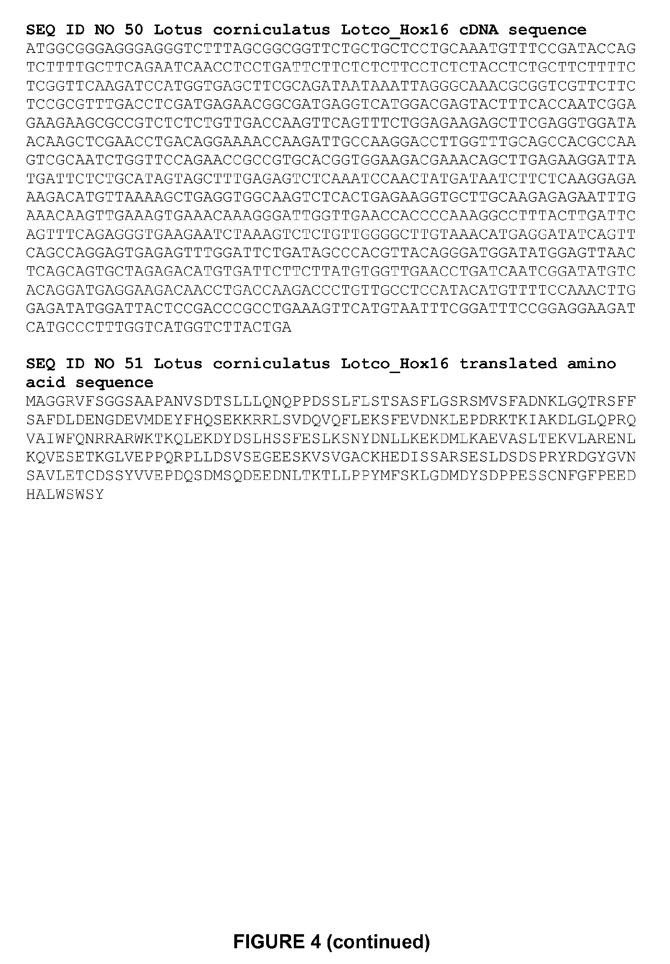

Fig. 4 details examples of class I homeodomain leucine zipper (HDZip) hox5 sequences useful in performing the methods according to the present invention. Several sequences result from public EST assemblies (see Table A), with lesser quality sequencing. As a consequence, a few nucleic acid substitutions may be expected. The start (ATG) and stop codons delimit the nucleic acid sequences.

### Examples

The present invention will now be described with reference to the following examples, which are by way of illustration alone and are not intended to completely define or to otherwise limit the scope of the invention.

Unless otherwise stated, recombinant DNA techniques were performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### Example 1: Identification of sequences related to SEQ ID NO: 1 and SEQ ID NO: 2

Sequences (full length cDNA, ESTs or genomic) were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program was used to find regions of local similarity between sequences by comparing nucleic acid or polypeptide sequences to sequence databases and by calculating the statistical significance of matches. For example, the polypeptide encoded by the nucleic acid of SEQ ID NO: 1 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.
Table A below provides a list of nucleic acid sequences related to the nucleic acid sequence of SEQ ID NO: 1.

**Table A: Examples of sequences related to the nucleic acid sequence of SEQ ID NO: 1**

| Name | NCBI nucleotide accession number | Nucleotide SEQ ID NO | Translated polypeptide SEQ ID NO | Source |
|---|---|---|---|---|
| Orysa_hox5 | XM_482406 | 1 | 2 | Oryza sativa |
| Orysa_hox16 | XM_467603 | 3 | 4 | Oryza sativa |
| Zeama_hox5^{*} | CO458693 DV024016 | 5 | 6 | Zea mays |
| Zeama_hox16 | AY105265 | 7 | 8 | Zea mays |
| Sacof_hox5^{*} | CA088615 CA115362 CA142506 | 9 | 10 | Saccharum officinarum |
| Sorbi_hox5^{*} | BE363386 CD432381 | 11 | 12 | Sorghum bicolor |
| Triae_hox16^{*} | DR735359 DR741379 CD916488 | 13 | 14 | Triticum aestivum |
| Arath_ATHB1 | X58821 | 15 | 16 | Arabidopsis thaliana |
| Dauca_CHB3^{**} | D26575 | 17 | 18 | Daucus carota |
| Glyma_HD157^{**} | AF184278 | 19 | 20 | Glycine max |
| Crapl_CPHB-5 | AF443621 | 21 | 22 | Craterostigma plantagineum |
| Goshi-hox5* | DT465649 CD486134 | 23 | 24 | Gossypium hirsutum |
| Lyces_hox5 | BT014213.1 | 25 | 26 | Lycopersicon esculentum |
| Lyces_VaHOX1 | X94947 | 27 | 28 | Lycopersicon esculentum |
| Medsa_hox16^{*} | CB892061 CA858059 | 29 | 30 | Medicago sativa |
| Aqufo_hox5 | DT758247 | 31 | 32 | Aquilegia formosa x Aquilegia pubescens |
| Poptr_hox16_1 | scaff_XV.439 | 40 | 41 | Populus tremuloides |
| Poptr_hox16_2 | scaff_XII.649 | 42 | 43 | Populus tremuloides |
| Poptr_hox16_3 | lcl\|scaff_VIII.183 9 | 44 | 45 | Populus tremuloides |
| Medtr_hox16_1 | CR954197.2 | 46 | 47 | Medicago truncatula |
| Phavu_hox16 | AF402605 | 48 | 49 | Phaseolus vulgaris |
| Lotco_hox16 | AP006364 | 50 | 51 | Lotus corniculatus |

| | | | | |
|---|---|---|---|---|
| ^{*}Contig compiled from several EST accessions (main ones shown); EST sequencing quality being usually lower, a few nucleic acid substitutions may be expected. ^{**}Sequences from Daucus carota and Glycine max have been corrected compared to their accession number. | | | | |

### Example 2: Alignment of class I HDZip hox5 polypeptide sequences

AlignX from the Vector NTI (Invitrogen) based on the popular Clustal algorithm of progressive alignment (Thompson et al. (1997) Nucleic Acids Res 25:4876-4882; Chenna et al. (2003). Nucleic Acids Res 31:3497-3500) was used. A phylogenetic tree can be constructed using a neighbour-joining clustering algorithm. Default values are for the gap open penalty of 10, for the gap extension penalty of 0,1 and the selected weight matrix is Blosum 62 (if polypeptides are aligned).

The result of the multiple sequence alignment is shown in Figure 2. The three main characterized domains, from N-terminal to C-terminal, are heavily boxed and identified as the acidic box, the class I homeodomain and the six heptad- leucine zipper. The "Conserved Domain" comprises these three domains. Additionally, the Trp tail and the RPFF amino acid motif are lightly boxed.

### Example 3: Calculation of global percentage identity between class I HDZip hox5 polypeptides sequences

Global percentages of similarity and identity between full length class I HDZip hox5 polypeptide sequences were determined using the Matrix Global Alignment Tool (MatGAT) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:
Scoring matrix: Blosum62
First Gap: 12
Extending gap: 2

Results of the software analysis are shown in Table B 1 for the global similarity and identity over the full length of the polypeptide sequences (excluding the partial polypeptide sequences). Percentage identity is given above the diagonal and percentage similarity is given below the diagonal.

The percentage identity between the polypeptide sequences shown can be as low as 29% amino acid identity compared to SEQ ID NO: 2.

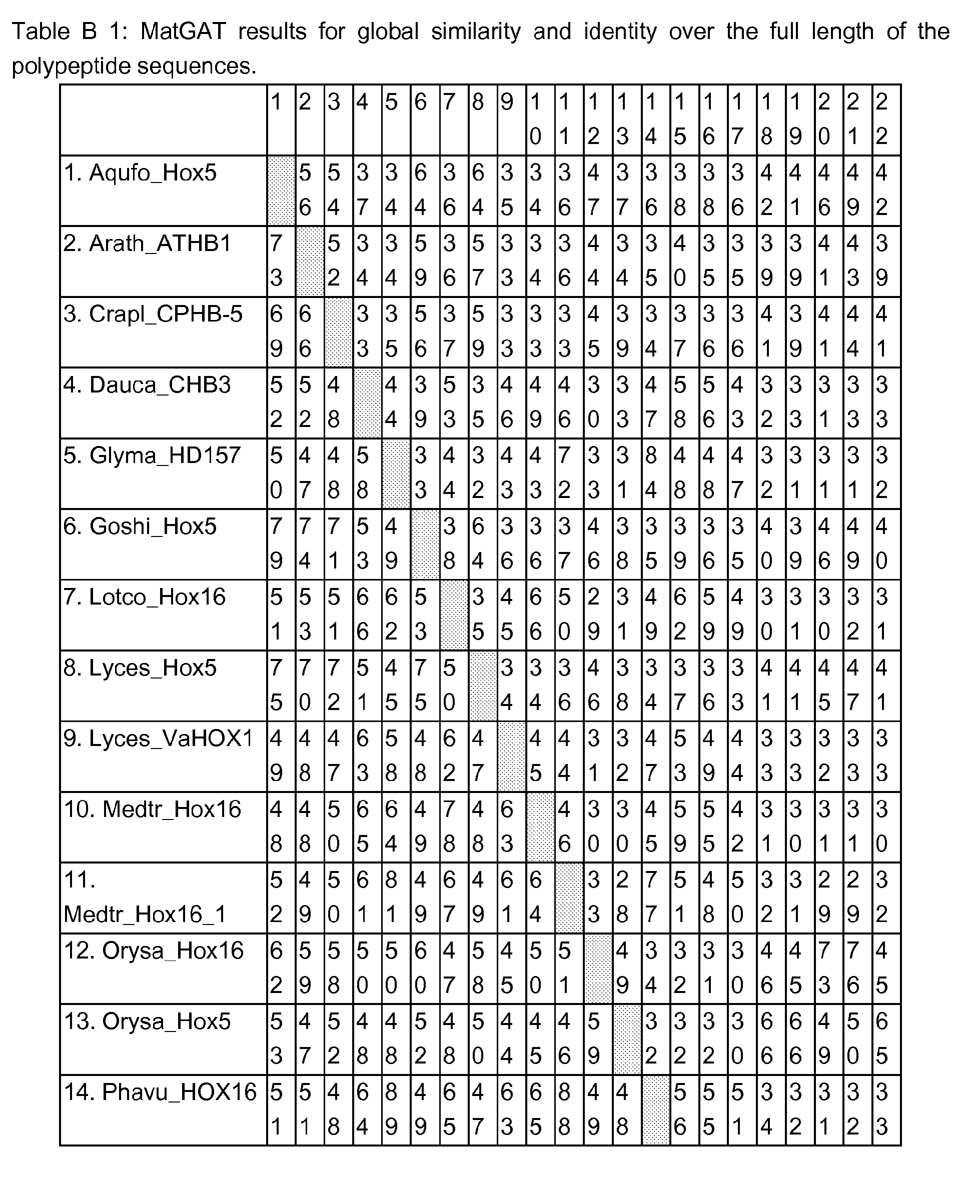

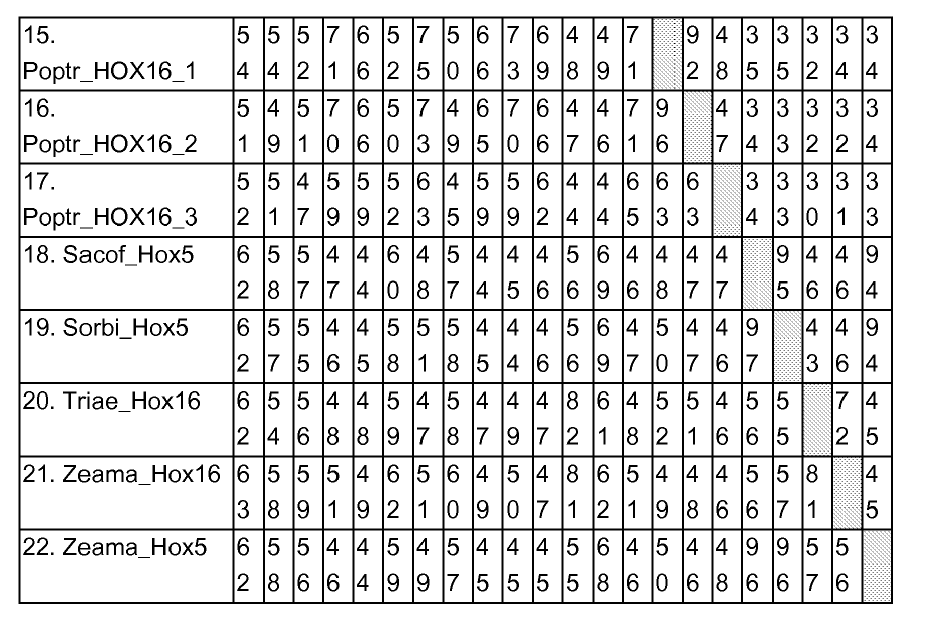

The "Conserved Domain" of class I HDZip hox5 polypeptide sequences comprises from N-terminal to C-terminal, an acidic box, a class I homeodomain and the six heptad-leucine zipper (see Figure 2), as defined hereinabove. When percentage identity analysis is performed on the conserved domains instead of on the full length polypeptide sequences, an increase in percentage identity is observed, as shown in Table B 2. Lowest values are now above 50% amino acid identity compared to SEQ ID NO: 2.

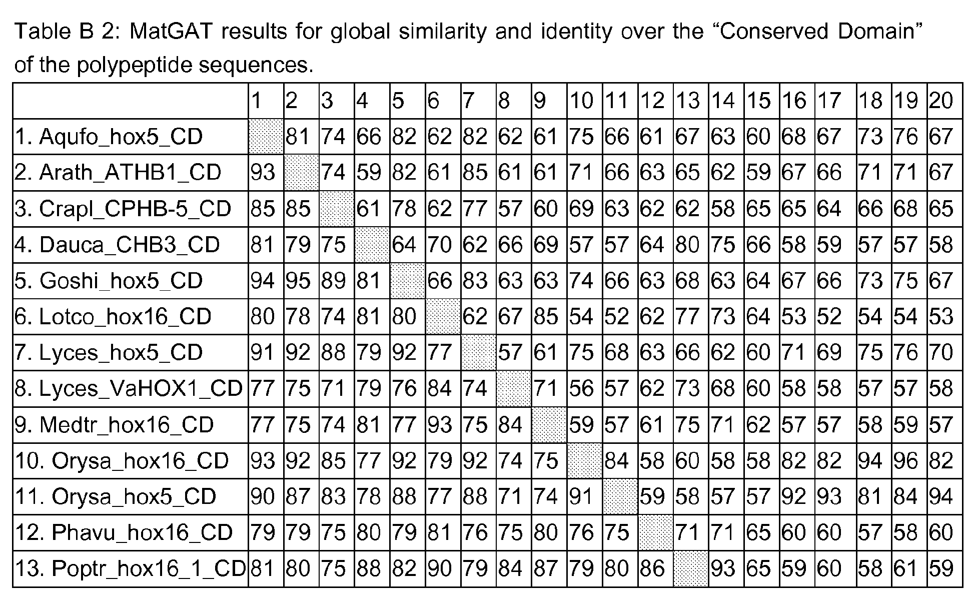

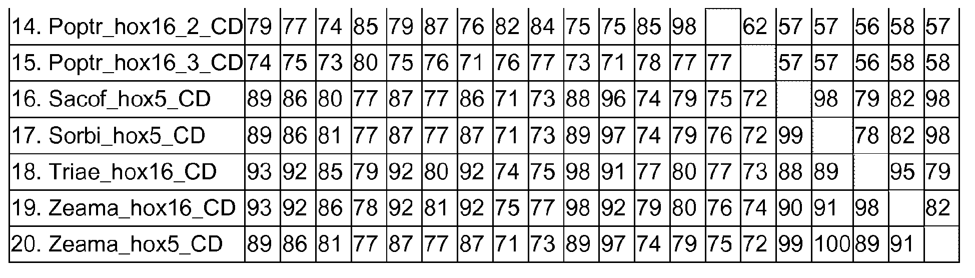

### Example 4: Identification of domains comprised in class I HDZip hox5 polypeptide sequences

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 2 are presented in Table C.

**Table C: InterPro scan results of the polypeptide sequence as represented by SEQ ID NO: 2**

| | | | |
|---|---|---|---|
| InterPro | IPR000047 | Helix-turn-helix motif, lambda-like repressor | |
| | PRINTS | PR00031 | HTHREPRESSR |
| | | | |
| InterPro | IPR001356 | Homeobox | |
| | PRODOM | PD000010 | Homeobox |
| | PRINTS | PR00024 | HOMEOBOX |
| | PFAM | PF00046 | Homeobox |
| | SMART | SM00389 | HOX |
| | PROFILE | PS00027 | HOMEOBOX_1 |
| | PROFILE | PS50071 | HOMEOBOX_2 |
| | | | |
| InterPro | IPR003106 | Leucine zipper, homeobox-associated | |
| | PFAM | PF02183 | HALZ |
| | | | |
| InterPro | IPR009057 | Homeodomain-like | |
| | SUPERFAMILY | SSF46689 | Homeodomain_lik e |
| InterPro | IPR012287 | Homeodomain-related | |
| | GENE3D | G3DSA:1.10.10.60 | Homeodomain-rel |

Primary amino acid composition (in %) to determine if a polypeptide domain is rich in specific amino acids (for example in an acidic box) may be calculated using software programs from the ExPASy server, in particular the ProtParam tool (Gasteiger E et al. (2003) ExPASy: the proteomics server for in-depth protein knowledge and analysis. Nucleic Acids Res 31:3784-3788). The composition of the polypeptide sequence of interest may then be compared to the average amino acid composition (in %) in the Swiss-Prot Protein Sequence data bank.

In the Table below (Table D), are compared the % Asp (D), % Glu (E) and their combined content in the acidic box of SEQ ID NO: 2 with the average in Swiss-Prot Protein Sequence databank.

**Table D**

| | % Asp (D) | % Glu (E) | % Asp (D) + % Glu (E) |
|---|---|---|---|
| Average in Swiss-Prot Protein Sequence databank | 5.3% | 6.6% | 11.9% |
| Acidic box of SEQ ID NO: 2 | 9.1 % | 54.5% | 63.6% |

An acidic box may be part of a transcription activation domain. Eukaryotic transcription activation domains have been classified according to their amino acid content, and major categories include acidic, glutamine-rich and proline-rich activation domains (Rutherford et al. (2005) Plant J. 43(5):769-88, and references therein).

The Gene Ontology (GO) Consortium is an international collaboration among scientists at various biological databases, with an Editorial Office based at the European Bioinformatics Institute. The objective of GO is to provide controlled vocabularies for the description of the molecular function, biological process and cellular component of gene products. When performing an InterPro scan as described above, the GO database is also searched. The class HDZip hox5 polypeptide sequences have as molecular function transcription factor and sequence-specific DNA binding activity, and localised in the nucleus of the plant cell (see Table below (Table E)).

**Table E**

| | Gene Ontology Entry |
|---|---|
| Homeodomain | Molecular Function: transcription factor activity (GO:0003700) Cellular Component: nucleus (GO:0005634) Molecular Function: sequence-specific DNA binding (GO:0043565) |
| Leucine zipper, homeobox-associated | Molecular Function: DNA binding (GO:0003677) Cellular Component: nucleus (GO:0005634) |

### Example 5: Topology prediction of class I HDZip hox5 polypeptide sequences

Leucine zipper prediction and heptad identification was carried out using specialised software such as 2ZIP, which combines a standard coiled coil prediction algorithm with an approximate search for the characteristic leucine repeat (Bornberg-Bauer et al. (1998) Nucleic Acids Res 26(11): 2740-2746; hosted at Max Planck Institut, Golm in Germany). A potential leucine zipper, a repeat of leucines or a coiled coil may be identified using this software.

The class I HDZip hox5 polypeptide sequences comprise a leucine zipper prediction, with at least 5, preferably 6 heptads. When the polypeptide of SEQ ID NO: 2 is submitted to this algorithm, a potential leucine zipper is between positions 143 and 178, as shown in the output below (numbers reflect amino acid position, C the coiled coil region, and L the leucine within the heptad):

1---------11--------21--------31--------41--------51--------
MDPGRVVFDSGVARRACPGGAQMLLFGGGGSANSGGFFRGVPAAVLGMDESRSSSSAAGA

61--------71--------81--------91--------101-------111-------
GAKRPFFTTHEELLEEEYYDEQAPEKKRRLTAEQVQMLERSFEEENKLEPERKTELARRL

121-------131-------141-------151-------161-------171-------
GMAPRQVAVWFQNRRARWKTKQLEHDFDRLKAAYDALAADHHALLSDNDRLRAQVISLTE
            CCCCCCCCCCCCCCCCCCCCCCCCCCCCCCCCCCCCCCCCCCCCCCCC
                      L------L------L------L------L------L
                      LZLZLZLZLZLZLZLZLZLZLZLZLZLZLZLZLZLZ

181-------191-------201-------211-------221-------231-------
KLQDKETSPSSATITTAAQEVDQPDEHTEAASTTGFATVDGALAAPPPGHQQPPHKDDLV
CCCCCCCC

241-------251-------261-------271-------281-------291-------
SSGGTNDDGDGGAAVVVFDVTEGANDRLSCESAYFADAAEAYERDCAGHYALSSEEEDGG

301-------311-------321-------331-------341------
AVSDEGCSFDLPDAAAAAAAMFGAAGVVHHDAADDEEAQLGSWTAWFWS

### Example 6: Assay for class I HDZip hox5 polypeptide sequences

Class I HDZip hox5 polypeptides or homologues thereof have DNA binding activity, preferably to 5 bp half-sites that overlap at a central position, CAA(A/T)ATTG, as detected in yeast one-hybrid assays (Meijer et al. (2000) Mol Gen Genet 263:12-21). In transient assays on rice cell suspensions, co-bombardement of a class I HDZip hox5 polypeptide with the GUS reporter gene reportedly resulted in an increased number of stained spots, which were also more intense in color (Meijer et al, supra). This assay is useful to demonstrate the activator function of class I HDZip hox5 polypeptides or homologues.

### Example 7: Cloning of Oryza sativa class I HDZip hox5 nucleic acid sequence

The Oryza sativa class I HDZip hox5 nucleic acid sequence was amplified by PCR using as template an Oryza sativa seedling cDNA library (Invitrogen, Paisley, UK). After reverse transcription of RNA extracted from seedlings, the cDNAs were cloned into pCMV Sport 6.0. Average insert size of the bank was 1.6 kb and the original number of clones was of the order of 1.67x10⁷cfu. Original titer was determined to be 3.34 x10⁶ cfu/ml after first amplification of 6x10¹⁰ cfu/ml. After plasmid extraction, 200 ng of template was used in a 50 µlPCR mix. Primers prm06000 (SEQ ID NO: 34; sense, start codon in bold, AttB1 site in italic: 5'- GGGGACAAGTTTGTACAAAAAAGCAGGCTTAAACAATGGATCCCGGCCG 3') and prm06001 (SEQ ID NO: 35; reverse, complementary, AttB2 site in italic: 5' GGGGACCACTTTGTACAAGAAAGCTGGGTGATCAGCTCCAGAACCAGG 3'), which include the AttB sites for Gateway recombination, were used for PCR amplification. PCR was performed using Hifi Taq DNA polymerase in standard conditions. A PCR fragment of 1116 bp (including attB sites; from start to stop 1050 bp) was amplified and purified also using standard methods. The first step of the Gateway procedure, the BP reaction, was then performed, during which the PCR fragment recombines in vivo with the pDONR201 plasmid to produce, according to the Gateway terminology, an "entry clone". Plasmid pDONR201 was purchased from Invitrogen, as part of the Gateway@ technology.

### Example 8: Vector Construction

The entry clone comprising the nucleic acid sequences was subsequently used in an LR reaction with a "destination" vector used for Oryza sativa transformation. This vector contained as functional elements within the T-DNA borders: a plant selectable marker; a screenable marker expression cassette; and a Gateway cassette intended for LR in vivo recombination with the sequence of interest already cloned in the entry clone. A rice GOS2 promoter (SEQ ID NO: 33 OR SEQ ID NO: 178) for constitutive expression was located upstream of this Gateway cassette.

After the LR recombination step, the resulting expression vector (Figure 3) was transformed into Agrobacterium strain LBA4044 according to methods well known in the art.

### Example 9: Plant transformation Rice transformation

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2%HgCI2, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

Agrobacterium strain LBA4404 containing the expression vector was used for cocultivation. Agrobacterium was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent TO rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### Example 10: Phenotypic evaluation procedure 10.1 Evaluation setup

Approximately 35 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

All T1 events were further evaluated in the T2 generation following the same evaluation procedure as for the T1 generation. From the stage of sowing until the stage of maturity the plants were passed several times through a digital imaging cabinet. At each time point, digital images (2048x1536 pixels, 16 million colours) were taken of each plant from at least 6 different angles.

### Salt stress screen

Plants from 4 events (T2 seeds) were grown on a substrate made of coco fibers and argex (3 to 1 ratio). A normal nutrient solution was used during the first two weeks after transplanting the plantlets in the greenhouse. After the first two weeks, 25 mM of salt (NaCI) was added to the nutrient solution, until the plants were harvested.

### Drought screen

Plants from five events (T2 seeds) were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. A confirmation round was performed consisting of repeating the screen with T2 seeds not harvested from plants of the first drought screen, but from plants grown under normal conditions.

### Reduced nutrient (nitrogen) availability screen

The rice plants are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Seed-related parameters are then measured

### 10.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

### 10.3 Parameters measured

### 10.3.1 Biomass-related parameter measurement

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The aboveground area is the time point at which the plant had reached its maximal leafy biomass. The early vigour is the plant (seedling) aboveground area three weeks post-germination.

An additional parameter was used to measure abiotic stress tolerance when necessary: the greenness index after drought, which gives an indication of the senescence of the plant. It is the proportion (expressed as %) of yellow pixels in the first imaging after drought.

To measure root-related parameters, plants were grown in specially designed pots with transparent bottoms to allow visualization of the roots. A digital camera recorded images through the bottom of the pot during plant growth. Root features such as total projected area (which can be correlated to total root volume), average diameter and length of roots above a certain thickness threshold (length of thick roots, or thick root length) were deduced from the picture using of appropriate software. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

### 10.3.2 Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area (mm²), multiplied by a factor 106. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Example 11: Results of transgenic rice plants expressing a class I HDZip hox5 nucleic acid under normal growth conditions

The results of the evaluation of transgenic rice plants expressing the class I HDZip hox5 nucleic acid under normal growth conditions are presented in Table F. The percentage difference between the transgenics and the corresponding nullizygotes is shown, with a P value from the F test below 0.05.

**Table F: Results of the evaluation of transgenic rice plants expressing a class I HDZip hox5 nucleic acid under normal growth conditions.**

| Trait | % Difference in T1 | % Difference in T2 |
|---|---|---|
| Average root diameter | 2 | 1 |
| Length of thick roots | 9 | 17 |
| Total seed yield | 17 | 19 |
| Number of filled seeds | 15 | 17 |
| Fill rate | 7 | 9 |
| Total number seeds | 6 | 9 |
| Number of flowers per panicle | 8 | 9 |
| TKW | 2 | 2 |
| Harvest index | 16 | 16 |
| Greenness index before flowering | 2 | 4 |

Example 12: Results of transgenic rice plants expressing the nucleic acid sequence useful in performing the methods of the invention, under salt stress growth conditions The results of the evaluation of transgenic rice plants expressing the nucleic acid encoding a class I HDZip hox5 polypeptide sequence under salt stress growth conditions are presented in Table G. The percentage difference between the transgenics and the corresponding nullizygotes is shown, with a P value from the F test below 0.05.

**Table G: Results of the evaluation of transgenic rice plants expressing the class I HDZip hox5 nucleic acid under salt stress growth conditions.**

| Trait | % Difference in T1 |
|---|---|
| Total seed yield | 41 |
| Number of filled seeds | 40 |
| Fill rate | 30 |
| TKW | 1 |
| Harvest index | 36 |
| Greenness index before flowering | 7 |

### Example 13: Results of transgenic rice plants expressing the class I HDZip hox5 nucleic acid under drought stress growth conditions

The results of the evaluation of transgenic rice plants expressing the nucleic acid sequence useful in performing the methods of the invention under drought stress growth conditions are presented in Table H. The percentage difference between the transgenics and the corresponding nullizygotes is shown, with a P value from the F test below 0.05.

**Table H: Results of the evaluation of transgenic rice plants expressing the nucleic acid sequence useful in performing the methods of the invention, under drought stress growth conditions.**

| Trait | % Difference in T1 | % Difference in T2 |
|---|---|---|
| Average root diameter | 2 | 1 |
| Length of thick roots | 9 | 14 |
| Total seed yield | 18 | 31 |
| Number of filled seeds | 21 | 33 |
| Fill rate | 15 | 33 |
| Harvest index | 18 | 34 |
| Greenness index after drought | 2 | 3 |

### Transformation of corn, wheat, soybean, canola, alfalfa with sequences useful in the methods of the invention Corn transformation

Transformation of maize (Zea mays) is performed with a modification of the method described by lshida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/I BAP, 3 % sucrose, 0.7 % Phytagar at 23 °C, 16 hr light. After two days of co-cultivation with Agrobacterium, the petiole explants are transferred to MSBAP-3 medium containing 3 mg/I BAP, cefotaxime, carbenicillin, or timentin (300 mg/I) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/I BAP). Shoots of about 2 cm in length are transferred to the rooting medium (MSO) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2S04, and 100 µmacetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

## Claims

1. Method for increasing plant yield relative to corresponding wild type plants comprising modulating expression in a plant of a nucleic acid encoding class I homeodomain leucine zipper (HDZip) hox5 polypeptide or homologue thereof, and optionally selecting for plants having increased yield, wherein said class I HDZip hox5 polypeptide or homologue comprises from N-terminal to C-terminal: (i) an acidic box; and (ii) a class I homeodomain; and (iii) a leucine zipper with more than 5 heptads.

2. Method according to claim 1, wherein said class I HDZip hox5 polypeptide or homologue thereof further comprises one or both of the following: (i) a Trp tail; and (ii) a RPFF amino acid motif, where R is Arg, P Pro and F Phe, and within this motif, allowing one or more conservative change(s) at any position, and/or one or two non-conservative change(s) at any position.

3. Method for increasing plant yield relative to corresponding wild type plants comprising introducing and expressing in a plant a class I HDZip hox5 nucleic acid or a variant thereof.

4. Method according to any one of the claims 1 to 3, wherein said variant is a portion of a class I HDZip hox5 nucleic acid, which portion encodes a polypeptide comprising from N-terminal to C-terminal: (i) an acidic box; and (ii) a class I homeodomain; and (iii) a leucine zipper with more than 5 heptads.

5. Method according to any of the claims 1 to 3, wherein said variant is a sequence capable of hybridising to a class I HDZip hox5 nucleic acid, which hybridising sequence encodes a polypeptide comprising from N-terminal to C-terminal: (i) an acidic box; and (ii) a class I homeodomain; and (iii) a leucine zipper with more than 5 heptads.

6. Method according to claims 4 to 5, wherein said class I HDZip hox5 nucleic acid or variant thereof is overexpressed in a plant.

7. Method according to any one of claims 4 to 6, wherein said class I HDZip hox5 nucleic acid or variant thereof is of plant origin, preferably from a monocotyledon plant, further preferably from the family Poaceae, more preferably from the genus Oryza, most preferably from Oryza sativa.

8. Method according to any one of claims 4 to 7, wherein said variant encodes an orthologue or paralogue of the class I HDZip hox5 protein of SEQ ID NO: 2.

9. Method according to any one of claims 4 to 8, wherein said class I HDZip hox5 nucleic acid or variant thereof is operably linked to a constitutive promoter, preferably said constitutive promoter is a GOS2 promoter, more preferably the constitutive promoter is a rice GOS2 promoter, further preferably the constitutive promoter is represented by a nucleic acid sequence substantially similar to SEQ ID NO: 33, most preferably the constitutive promoter is as represented by SEQ ID NO: 33.

10. Method according to any one of claims 1 to 9, wherein said increased yield is selected from one or more of: increased number of filled seeds, increased total seed yield, increased number of flowers per panicle, increased seed fill rate, increased harvest index (HI), increased thousand kernel weight (TKW), increased root length or increased root diameter, each relative to corresponding wild type plants.

11. Method according to any one of claims 1 to 10, wherein said increased yield occurs under abiotic stress, preferably said abiotic stress is an osmotic stress, selected from one or more of: water stress, salt stress, oxidative stress and ionic stress, preferably wherein said water stress is drought stress.

12. Plant, plant part or plant cell obtainable by a method according to any one of claims 1 to 11.

13. Use of a construct comprising:
(i) a class I HDZip hox5 nucleic acid or variant thereof
(ii) one or more control sequences capable of driving expression of the nucleic acid sequence of (i), and optionally
(iii) a transcription termination sequence
in a method according to any one of claims 3 to 12.

14. Construct according to claim 13, wherein said control sequence is a constitutive promoter, preferably said constitutive promoter is a GOS2 promoter, preferably the rice GOS2 promoter, further preferably the constitutive promoter is represented by a nucleic acid sequence substantially similar to SEQ ID NO: 33, most preferably the constitutive promoter is as represented by SEQ ID NO: 33.

15. Plant, plant part or plant cell transformed with a construct comprising a class I HDZip hox5 nucleic acid or variant thereof under the control of a GOS2 promoter, as defined in claim 14.

16. Method for the production of a transgenic plant having increased yield relative corresponding wild type plant, which method comprises:
(i) introducing and expressing in a plant or plant cell a class I HDZip hox5 nucleic acid or variant thereof;
(ii) cultivating the plant cell under conditions promoting plant growth and development.

17. Harvestable parts of a plant according to any one of claims 12 or 15, preferably said harvestable parts are seeds.

18. Use of a class I HDZip hox5 nucleic acid/gene or variant thereof, or use of a class I HDZip hox5 polypeptide or homologue thereof, in increasing plant yield relative to corresponding wild type plants, preferably said increased yield is selected from one or more of: increased number of filled seeds, increased total seed yield, increased number of flowers per panicle, increased seed fill rate, increased HI, increased TKW, increased root length or increased root diameter, each relative to corresponding wild type plants.

19. Use of a class I HDZip hox5 nucleic acid/gene or variant thereof, or use of a class I HDZip hox5 polypeptide or homologue thereof, as a molecular marker.
